# EUROPÄISCHE PATENTSCHRIFT

(11) **EP 3 721 225 B1**
(45) Veröffentlichungstag und Bekanntmachung des Hinweises auf die Patenterteilung: **01.10.2025**
(21) Anmeldenummer: 18815162.5
(22) Anmeldetag: 04.12.2018
(51) Int. Cl.: G01N 33/49, A61B 5/00, A61B 5/145, G01N 21/17, G01N 21/39

(54) **VORRICHTUNG UND VERFAHREN ZUM ANALYSIEREN EINES STOFFS**
DEVICE AND METHOD FOR ANALYZING A SUBSTANCE
DISPOSITIF ET PROCÉDÉ D'ANALYSE D'UNE SUBSTANCE

(30) Priorität: 04.12.2017 WO PCT/EP2017/081398
(43) Veröffentlichungstag der Anmeldung: 14.10.2020
(73) Patentinhaber: DiaMonTech AG, 10245 Berlin (DE)
(72) Erfinder: LUBINSKI, Thorsten, 10245 Berlin (DE); SCHRIEK, Uwe, 10627 Berlin (DE)
(74) Vertreter: Lucke, Andreas
(86) Internationale Anmeldenummer: PCT/EP2018/083509
(87) Internationale Veröffentlichungsnummer: WO 2019/110597

(56) Entgegenhaltungen:
- EP-A1- 1 048 265
- WO-A1-2017/097824
- US-A1- 2007 015 978
- US-A1- 2017 146 455
- JONAS KOTTMANN ET AL: "Mid-Infrared Fiber-Coupled Photoacoustic Sensor for Biomedical Applications", SENSORS, vol. 13, no. 1, 2 January 2013 (2013-01-02), CH, pages 535 - 549, XP055465861, ISSN: 1424-8220, DOI: 10.3390/s130100535

## Beschreibung

Das vorliegende Schutzrecht bezieht sich auf eine Vorrichtung und ein Verfahren zum Analysieren eines Stoffs unter besonderer, wenn auch nicht ausschließlicher, Berücksichtigung von Detektionsverfahren, die sich eines Piezo- Effektes bedienen. Die hier beschriebene Vorrichtung und das hier beschriebene Verfahren können zum Beispiel zur Analyse von tierischem oder menschlichem Gewebe, Körperflüssigkeiten und in einer Ausführungsform zur Messung von Glukose bzw. Blutzucker, genutzt werden.

Bekannte Verfahren zum Analysieren eines Stoffs, insbesondere zum Messen von Blutzucker, sind beispielsweise in den folgenden Druckschriften beschrieben:
- Guo et al.: "Noninvasive glucose detection in human skin using wavelength modulated differential laser photothermal radiometry", Biomedical Optics Express, Vol, 3, 2012, No. 11,
- Uemura et al.: "Non-invasive blood glucose measurement by Fourier transform infrared spectroscopic analysis through the mucous membrane of the lip: application of a chalcogenide optical fiber system", Front Med Biol Eng. 1999; 9(2): 137-153,
- Farahi et al.: "Pump probe photothermal spectroscopy using quantum cascade lasers", J. Phys. D. Appl. Phys. 45 (2012) und
- M. Fujinami et al.: "Highly sensitive detection of molecules at the liquid/liquid interface using total internal reflection-optical beam deflection based on photothermal spectroscopy", Rev. Sci. Instrum., Vol. 74, Number 1 (2003).
- (1) von Lilienfeld-Toal, H. Weidenmüller, M. Xhelaj, A. Mäntele, W. A Novel Approach to Non- Invasive Glucose Measurement by Mid-Infrared Spectroscopy: The Combination of Quantum Cascade Lasers (QCL) and Photoacoustic Detection Vibrational Spectroscopy, 38:209-215, 2005.
- (2) Pleitez, M. von Lilienfeld-Toal, H. Mäntele W. Infrared spectroscopic analysis of human interstitial fluid in vitro and in vivo using FT-IR spectroscopy and pulsed quantum cascade lasers (QCL): Establishing a new approach to non invasive glucose measurement Spectrochimica Acta. Part A, Molecular and biomolecular spectroscopy, 85:61-65, 2012
- (3) Pleitez, M. et al. In Vivo Noninvasive Monitoring of Glucose Concentration in Human Epidermis by Mid-Infrared Pulsed Photoacoustic Spectroscopy Analytical Chemistry, 85:1013-1020, 2013.
- (4) Pleitez, M. Lieblein, T. Bauer, A. Hertzberg, O. von Lilienfeld-Toal, H. Mäntele, W. Windowless ultrasound photoacoustic cell for in vivo mid-IR spectroscopy of human epidermis: Low interference by changes of air pressure, temperature, and humidity caused by skin contact opens the possibility for a non-invasive monitoring of glucose in the interstitial fluid Review of Scientific Instruments 84, 2013
- (5) M. A. Pleitez Rafael, O. Hertzberg, A. Bauer, M. Seeger, T. Lieblein, H. von Lilienfeld-Toal, and W. Mäntele. Photothermal deflectometry enhanced by total internal reflection enables non- invasive glucose monitoring in human epidermis. The Analyst, November 2014.

US 2007/0015978 A1 offenbart ein Verfahren und eine Vorrichtung zur nichtinvasiven Messung von Informationen betreffend einen lebenden Körper unter Verwendung einer Lichtquelle, die dazu konfiguriert ist, Licht zu generieren, welches eine spezifische Wellenlängenkomponente enthält. Die Vorrichtung umfasst eine Bestrahlungseinheit, die dazu konfiguriert ist, ein Subjekt mit dem Licht zu bestrahlen, und zumindest eine akustische Signaldetektionseinheit, die piezoelektrische Vorrichtungen umfasst, die aus einem piezoelektrischen Einheitskristall gebildet sind, der Bleititanat enthält, und die dazu konfiguriert sind, ein akustisches Signal zu detektieren, welches infolge der Absorption des genannten Lichts durch eine spezifische Substanz in oder auf dem Körper erzeugt wird.

JONAS KOTTMANN ET AL: "Mid-Infrared Fiber-Coupled Photoacoustic Sensor for Biomedical Applications", SENSORS, Bd. 13, Nr. 1, 2. Januar 2013 (2013-01-02), Seiten 535-549, beschreibt die Implementierung eines MIR-fasergekoppelten photoakustischen (PA) Sensors zur Untersuchung von kondensierten Proben im MIR-Fingerprint-Bereich. Das Licht eines Quantenkaskadenlasers mit externem Resonator (1010-1095 cm-1) wird über ein Silberhalogenid geliefert, das an einer PA-Zelle befestigt ist. Eine PA-Kammer ist konisch geformt, um den aus der Faser austretenden Strahl anzupassen und das Zellenvolumen zu minimieren. Daraus ergibt sich ein kompakter und handlicher Sensor für Untersuchungen biologischer Proben und die Überwachung von Inhaltsstoffen sowohl in vitro als auch in vivo. Die Leistungsfähigkeit des fasergekoppelten PA-Sensors wird durch die Messung von Glukose in wässrigen Lösungen demonstriert. Diese Messungen ergeben eine Nachweisgrenze von 57 mg/dL (SNR =1). Außerdem wurde der fasergekoppelte Sensor zur Aufzeichnung menschlicher Hautspektren an verschiedenen Körperstellen eingesetzt, um seine Flexibilität zu veranschaulichen.

WO 2017/097824 A1 offenbart eine Vorrichtung zum Analysieren eines Stoffs mit einer Anregungssendeeinrichtung zum Erzeugen mindestens eines elektromagnetischen Anregungsstrahls mit zumindest einer Anregungswellenlänge, einer Detektionseinrichtung zur Detektion eines Reaktionssignals und einer Einrichtung zum Analysieren des Stoffes anhand des detektierten Reaktionssignals.

US 2017/0146455A1 offenbart eine Einrichtung zur Analyse eines Stoffs. Ein optisches Medium wird auf einer Oberfläche des Stoffs angeordnet, wobei mindestens ein Bereich der Oberfläche des optischen Mediums mit der Oberfläche des Stoffs in Kontakt ist. Ein Anregungslichtstrahl wird durch den Kontaktbereich zwischen der Stoffoberfläche und der Oberfläche des Mediums eingestrahlt. Ein Meßlichtstrahl wird durch das optische Medium zu dem Kontaktbereich der Oberfläche des Mediums eingestrahlt, sodass der Meßlichtstrahl und der Anregungslichtstrahl an der Grenzfläche zwischen dem optischen Medium und dem Stoff überlappen. Der Meßlichtstrahl wird an dieser Grenzfläche reflektiert. Eine Ablenkung des reflektierten Meßlichtstrahls wird in Abhängigkeit von der Wellenlänge des Anregungsichtstrahls detektiert. Der Stoff wird basierend auf der detektierten Ablenkung des Meßlichtstrahls in Abhängigkeit von der Wellenlänge des Anregungsichtstrahls analysiert.

EP1048265A1 offenbart eine Vorrichtung zum Detektieren einer Substanz in einer Probe, insbesondere zur Detektion von Glukose in einer Körperflüssigkeit oder im Blut. Die Vorrichtung umfasst einen Halbleiterlaser zum Emittieren von Laserlicht im mittleren Infrarotbereich bei mindestens zwei diskreten Wellenlängen, die sich jeweils an einer zugehörigen Spitze oder einem zugehörigen Tal in dem Absorptionsspektrum der Substanz in der Probe befinden. Ein fotoakustischer Detektor detektiert akustische Signale, die von der Absorption des Laserlichts herrühren. Eine Anzeigeeinheit wertet die akustischen Signale für jede Wellenlänge separat aus und berechnet ein Detektionsergebnis basierend auf allen akustischen Signalen der unterschiedlichen Wellenlängen.

Es besteht die Aufgabe, eine Vorrichtung und ein Verfahren anzugeben, mit der sich ein Stoff, insbesondere ein tierisches oder menschliches Gewebe oder ein Bestandteil oder Inhaltsstoff des Gewebes, besonders einfach und kostengünstig analysieren lässt. Ein Aspekt der Erfindung liegt dabei auch bei der Erreichung einer geringen Baugröße der Vorrichtung.

Diese Aufgabe wird unter anderem durch eine Vorrichtung mit den Merkmalen gemäß Anspruch 1 gelöst. Ausgestaltungen der Vorrichtung sind in Unteransprüchen angegeben. Zudem bezieht sich die Erfindung auf ein Verfahren gemäß dem unabhängigen Verfahrens-Patentanspruch mit entsprechenden Ausgestaltungen gemäß dem/den von diesem abhängigen Unteranspruch/ansprüchen.

Verwiesen wird auf die deutsche Patentschrift DE 10 2014 108 424 B3, auf deren Inhalt hier konkret Bezug genommen wird und auf die diese Anmeldung inhaltlich aufbaut; der komplette Inhalt der deutschen Patentschrift DE 10 2014 108 424 B3 soll daher durch diese hier vorgenommene explizite Bezugnahme auch als Offenbarungsgehalt dieser Anmeldung angesehen werden ("incorporation by reference" für sämtliche Details der dortigen Offenbarung). Insbesondere bezieht sich diese Bezugnahme auf sämtliche in den erteilten Patentansprüchen genannten Merkmale. Darüber hinaus bezieht sich die Bezugnahme insbesondere auch auf Details des dort genannten Anregungs-Lichtstrahls, beispielsweise auf die dort genannten Zahlenwerte der Pulsfrequenzen und Wellenlängen(-bereiche), und ebenso auf die Details zur Messung von Glukosegehalt in der interstitiellen Flüssigkeit.

Die vorliegende Schutzrechtsanmeldung bezieht sich neben den unmittelbar bei Einreichung ausdrücklich genannten Anspruchsgegenständen und Ausführungsbeispielen auch auf weitere Aspekte, die am Ende der hiesigen Beschreibung aufgezählt sind. Diese Aspekte können einzeln oder in Gruppen jeweils mit Merkmalen der bei Einreichung genannten Ansprüche kombiniert werden. Diese Aspekte stellen jeweils auch für sich genommen, oder miteinander oder mit Anspruchsgegenständen dieser Anmeldung kombiniert, eigenständige Erfindungen dar. Die Anmelderin behält sich vor, diese Erfindungen zu einem späteren Zeitpunkt zum Gegenstand von Ansprüchen zu machen. Dies kann im Rahmen dieser Anmeldung oder auch im Rahmen von späteren Teilanmeldungen, Fortsetzungsanmeldungen ("continuation applications" in den USA), Teil-Fortsetzungsanmeldungen ("continuation-in-part applications" in den USA) oder Nachanmeldungen unter Inanspruchnahme der Priorität dieser Anmeldung geschehen.

Unter dem Begriff "Licht" oder "Laserlicht" werden im Zusammenhang der nachfolgenden Ausführungen elektromagnetische Wellen bzw. elektromagnetische Strahlung im sichtbaren Bereich, im nahen, mittleren und fernen Infrarotbereich sowie im UV-Bereich verstanden.

Ein möglicher Aspekt des hier vorgestellten Verfahrens ist die Fokussierung der Messung des Reaktionssignals auf selektierte Tiefenbereiche unterhalb der (Abstandsintervalle von der) Stoffoberfläche. Die Größe d hat dabei den größten Einfluss auf den mit dem Verfahren gemessenen Tiefenbereich. Sie ist definiert als
d = √(D/(π*f)), wobei D die thermische Diffusivität der Probe (hier bspw. Haut) ist und f die Modulationsfrequenz des Anregungstrahls. Literatur zur thermischen Diffusivität von Haut:
- U. Werner, K. Giese, B. Sennhenn, K. Plamann, and K. Kölmel, "Measurement of the thermal diffusivity of human epidermis by studying thermal wave propagation," Phys. Med. Biol. 37(1), 21-35 (1992).
- A. M. Stoll, Heat Transfer in Biotechnology, Vol 4 of Advances in Heat Transfer, J. P. Hartnett and T. Irvin, eds. (New York, Academic, 1967), p 117.

Zur Eliminierung von Reaktionssignalen aus den obersten Schichten des Stoffes zum Zweck der Qualitätsverbesserung der Messung können in einer Ausführungsform Änderungen der Messwerte im Vergleich zu vorangegangenen Messungen herangezogen werden, falls die Messwerte in den obersten Schichten sich im Vergleich zu anderen, tieferliegenden Schichten weniger oder langsamer verändern. Dies kann in einer Ausführungsform bei Messungen an der menschlichen Haut der Fall sein, wo die obersten Hautschichten praktisch keinem Austausch mit den unteren Schichten unterliegen und deshalb physiologische Parameter wenig veränderlich sind. Es kann auch die zeitliche Ableitung von Messwerten zu Reaktionssignalen zum Ausschluss der Signale aus den obersten Hautschichten herangezogen werden. So kann die Messung oder zumindest die Auswertung auf die interstitielle Flüssigkeit in der Haut begrenzt oder fokussiert werden.

Zu diesem Zweck kann eine Messung die Aufnahme von Reaktionssignalen für Spektren umfassen, die mehrfach mit verschiedenen Modulationsfrequenzen der Anregungslichtquelle erfasst werden, wobei die Ergebnisse für verschiedene Modulationsfrequenzen miteinander verknüpft werden, beispielsweise durch Differenzbildung oder Quotientenbildung der Messwerte von Reaktionssignalen für gleiche Wellenlängen und verschiedene Modulationsfrequenzen. Es sollte zur Durchführung einer solchen Messung auch eine Vorrichtung mit einer entsprechenden Steuereinrichtung für den Anregungsstrahl und einer Auswerteeinrichtung für die Spektren von Reaktionssignalen vorgesehen sein.

Im Folgenden wird zunächst auf die bei Einreichung aufgeführten Anspruchsgegenstände eingegangen.

Die Aufgabe wird mit den Merkmalen der Erfindung gemäß Patentanspruch 1 gelöst durch eine Einrichtung zur Analyse eines Stoffes mit:
- einem Messkörper, der eine Messfläche aufweist, die zur Messung wenigstens teilweise mit dem Stoff in Kontakt zu bringen ist,
- einer Anregungsstrahlquelle, insbesondere einer Lasereinrichtung, weiter insbesondere mit einem Quantenkaskadenlaser (QCL), einem durchstimmbaren QCL, und/oder mit einem Laserarray, vorzugsweise einem Array von QCLs, zur Erzeugung eines oder mehrerer Anregungsstrahlen mit verschiedenen Wellenlängen im infraroten Spektralbereich zwischen 3 µm und 20 µm, der die Messfläche durchsetzend auf den Stoff gerichtet ist, und
- einer Detektionsvorrichtung, die folgendes umfasst:
   - einen Detektionsbereich, der Teil des Messkörpers und insbesondere der Messfläche benachbart oder unmittelbar benachbart ist, und dessen Material elektrische Eigenschaften hat, die sich in Abhängigkeit von einer Änderung in dem Druck oder der Temperatur ändern, und
   - Elektroden, mit denen elektrische Signale erfasst werden können, die die genannten elektrischen Eigenschaften repräsentieren, wobei wenigstens zwei Elektroden (60, 6d) in einer Richtung senkrecht zu einer Flächennormalen (7) der Messfläche (2) voneinander beabstandet auf verschiedenen Seiten des Detektionsbereiches (4) angeordnet sind.

Dabei können die genannten Eigenschaften des Bereichs insbesondere Eigenschaften des Materials des Bereiches sein.

Bevorzugt ist dabei eine derartige Einrichtung zur Analyse eines Stoffes, bei der die elektrische Eigenschaft, die sich in Abhängigkeit von dem Druck oder der Temperatur ändert,
- zu piezoelektrischen Signalen an den Elektroden als Funktion der Druckänderung und/oder der Temperaturänderung führt, oder
- durch einen spezifischen elektrischen Widerstand gebildet wird, der sich in Abhängigkeit von der Temperatur ändert,
wobei die Einrichtung ferner eine elektrische Kontakteinrichtung umfasst, die die genannten Elektroden aufweist, welche mit dem Detektionsbereich des Messkörpers elektrisch leitend zur Erfassung des elektrischen Widerstandes und/oder der piezoelektrischen Signale verbunden sind.

Im Falle der Detektion piezoelektrischer Signale kann die elektrische Eigenschaft, die sich in Abhängigkeit von dem Druck oder der Temperatur ändert, als "Polarisation" bezeichnet werden. Andere elektrische Eigenschaften, die sich in Abhängigkeit von Druck und/oder Temperatur ändern und für die Zwecke der Erfindung ausgenutzt werden können, sind gleichfalls möglich, beispielsweise die Dielektrizitätskonstante.

Unter dem Anregungsstrahl soll in diesem Zusammenhang ein Lichtstrahl, ein Laserstrahl oder auch eine Mehrzahl von im wesentlichen parallelen Laserstrahlen verstanden werden, die zeitlich nacheinander oder gleichzeitig von der Anregungsstrahlquelle, insbesondere einer Lasereinrichtung oder einzelnen Laserelementen einer Anregungsstrahlquelle, insbesondere einer Lasereinrichtung zu dem zu analysierenden Stoff ausgesendet werden. Die Kohärenz des Anregungsstrahles wird dabei nicht benötigt, so dass auch nicht oder nur teilweise kohärente Strahlung verwendet werden kann. Insofern sind als Strahlquellen außer Lasereinrichtungen auch andere Lichtquellen, wie Leuchtdioden/Halbleiterdioden oder andere einsetzbar, die die Selektion von Wellenlängen oder Wellenlängenbereichen zulassen. Es können bei Verwendung einer breitbandigeren Lichtquelle für den Anregungsstrahl auch Wellenlängenfilter, beispielsweise abstimmbare Filter, verwendet werden, um selektiv für verschiedene Wellenlängen oder Wellenlängenbereiche Anregungsstrahlen zu erzeugen.

Für verschiedene Anwendungsfälle kann es sinnvoll sein, einen Anregungsstrahl im mittleren Infrarotbereich zu wählen. Als Lichtquelle kann beispielsweise auch ein Laser gewählt werden, der nach dem OPA (optical parametric amplification, parametrische Verstärkung) oder NOPA (non-collinear optical parametric amplification, nicht-kollineare optische parametrische Verstärkung) oder OPG (optical parametric generation, optische parametrische Erzeugung)- Verfahren arbeitet und mit einem solchen Verfahren die Erzeugung verschiedener Wellenlängen erlaubt.

Es ist auch die Verwendung eines sogenannten optisch parametrischen Oszillators (OPO) in einem optischen Resonator möglich, der einen optisch nichtlinearen Kristall, zum Beispiel Beta- Bariumborat aufweist. Der Kristall erzeugt aus der eingestrahlten Pumpwelle aufgrund der nichtlinearen Drei-Wellen-Wechselwirkung unter anderem Strahlung einer transformierten Wellenlänge. Auf diese Weise kann, wie auch mit den oben genannten OPA/NOPA-Verfahren oder -Anordnungen, beispielsweise aus einer Strahlung im nahen Infrarot-Wellenlängenbereich eine Strahlung im mittleren Infrarotbereich gewonnen werden, die für die in dieser Anmeldung beschriebenen spektroskopischen Verfahren verwendet werden kann.

Der Detektionsbereich des Messkörpers bezeichnet einen räumlichen Bereich oder Abschnitt des Messkörpers, der die genannten Eigenschaften aufweist, indem er einen druck- oder temperaturabhängigen spezifischen elektrischen Widerstand aufweist und /oder bei Druck- oder Temperaturänderungen elektrische, insbesondere piezoelektrische Spannungssignale erzeugt. Dies wird durch die Materialwahl und optional auch weitere Gestaltung, beispielsweise Bearbeitung, des Materials erreicht, aus dem der Detektionsbereich besteht.

Es kann der Fall sein, dass die dem Detektionsbereich benachbarten Bereiche des Messkörpers sich diesbezüglich, insbesondere durch das Material, aus dem sie bestehen, von dem Material des Detektionsbereichs unterscheiden. Es kann jedoch auch vorgesehen sein, dass die dem Detektionsbereich benachbarten Bereiche des Messkörpers ebenso wie der Detektionsbereich einen druck- oder temperaturabhängigen spezifischen elektrischen Widerstand aufweisen und /oder bei Druck- oder Temperaturänderungen elektrische, insbesondere piezoelektrische Spannungssignale erzeugen. Der Detektionsbereich kann durch Elektroden und/oder Trennbereiche von weiteren Bereichen des Messkörpers getrennt sein, wobei die Trennbereiche aus einem Material bestehen können, das wesentlich weicher, elastischer oder nachgiebiger ist als das Material des Detektionsbereichs, so dass das Material der Trennbereiche eine Druck- oder Temperaturerhöhung schlechter weiterleitet als das Material des Detektionsbereichs.

Der Messkörper kann insbesondere auch aus einem ersten Teil und einem als Sensor- Schicht ausgebildeten zweiten Teil des Messkörpers gebildet sein, wobei die Sensor- Schicht eine andere Beschaffenheit aufweisen kann, als der erste Teil des Messkörpers und insbesondere wenigstens teilweise aus einem piezoelektrischen Material bestehen kann. Die Sensor-Schicht kann im Bereich der Messfläche auf den ersten Teil des Messkörpers aufgebracht, insbesondere aufgeklebt sein. Die Sensor-Schicht kann auch aus einem Material bestehen, das eine stärkere Abhängigkeit des Brechungsindex vom Druck oder der Temperatur aufweist als der erste Teil des Messkörpers. Der erste Teil des Messkörpers kann für den Anregungsstrahl/die Anregungsstrahlen transparent sein und beispielsweise aus Silizium bestehen. Falls die Sensorschicht für den/die Anregungsstrahlen nicht transparent oder weniger Transparent ist als der erste Teil des Messkörpers, dann kann die Sensorschicht eine Aussparung zum Durchtritt des/der Anregungsstrahlen aufweisen. In der Aussparung kann beispielsweise auch eine Linse zur Fokussierung des /der Anregungsstrahlen auf einen Punkt in dem zu analysierenden Stoff vorgesehen sein.

Es kann zur Führung des/der Anregungsstrahlen auch ein Lichtwellenleiter innerhalb des Messkörpers vorgesehen sein, der in, an oder vor der Messfläche endet. Der Lichtwellenleiter kann in ein Substrat des Messkörpers integriert sein und in SOI- Technologie hergestellt sein. Der Lichtwellenleiter kann den Detektionsbereich durchsetzen oder auch an diesem vorbeigeführt sein. Der Lichtwellenleiter kann aus einem Material bestehen, das keine oder nur eine minimale Abhängigkeit des Brechungsindex von einem mechanischen Druck aufweist, um den Einfluss von Piezoelektrischer Druckerzeugung auf den Anregungsstrahl zu minimieren. Das Material des Lichtwellenleiters kann auch mechanisch von dem im Detektionsbereich vorgesehenen piezoelektrischen Material beabstandet oder von diesem durch eine nachgiebige, insbesondere elastische Materialschicht oder eine Gasschicht mechanisch entkoppelt sein.

Die Aufgabe wird alternativ mit den Merkmalen der Erfindung gemäß Patentanspruch 2 gelöst durch eine Einrichtung zur Analyse eines Stoffes mit:
- einem Messkörper, der eine Messfläche aufweist, die zur Messung wenigstens teilweise mit dem Stoff in Kontakt zu bringen ist,
- einer Anregungsstrahlquelle, insbesondere einer Lasereinrichtung, weiter insbesondere mit einem Quantenkaskadenlaser (QCL), einem durchstimmbaren QCL, und/oder mit einem Laserarray, vorzugsweise einem Array von QCLs, vorzugsweise im infraroten Spektralbereich, zur Erzeugung eines Anregungsstrahls mit verschiedenen wählbaren Wellenlängen der die Messfläche durchsetzend auf den Stoff gerichtet ist, und
- mit wenigstens einer Detektionsvorrichtung, die der Messfläche benachbart angeordnet ist und/oder an diese unmittelbar angrenzt, wobei die Detektionsvorrichtung eine Kontakteinrichtung mit wenigstens zwei Elektroden zur Erfassung von piezoelektrischen Signalen aufweist, die einander auf verschiedenen Seiten eines Detektionsbereiches gegenüberliegen. Im Detektionsbereich ist dabei ein Material angeordnet, das in Abhängigkeit von Temperatur- und /oder Druckänderungen, insbesondere aufgrund eines Piezoelektrischen Effekts, seinen elektrischen Widerstand ändert oder ein elektrisches Signal erzeugt.

Durch die Anregungsstrahlquelle, insbesondere Lasereinrichtung wird sowohl beim Gegenstand des Patentanspruchs 1 als auch beim Gegenstand des Patentanspruchs 2 ein Anregungsstrahl in den zu analysierenden Stoff ausgesandt, der beispielsweise bestimmte Wellenzahlen/Wellenlängen durchläuft oder eine Mehrzahl von Anregungsstrahlen mit bestimmten festen Wellenzahlen nacheinander oder gleichzeitig aussendet. Dabei kann die Wellenlängenselektion auch durch Filter erst im Lichtweg hinter der Anregungsstrahlquelle erfolgen.

Im Fall der Verwendung eines Arrays kann das Laserarray einfache Laser mit fester Wellenlänge aufweisen. Bei bestimmten Wellenlängen wird der Strahl in Abhängigkeit vom Material des zu analysierenden Stoffes absorbiert, so dass Energie frei wird, die wenigstens teilweise in Form einer Wärmewelle zur Oberfläche des Stoffes und weiter in den Messkörper und dort auch zum Detektionsbereich transportiert wird. Der Anregungsstrahl ist dabei vorteilhaft intensitätsmoduliert, wobei die Verwendung verschiedener, auch mehrerer Modulationsfrequenzen möglich ist. Es ist auch die Formung von Laserpulsen möglich, die im fouriertransformierten Bereich eine Mehrzahl von Modulationsfrequenzen enthalten. Die impulsartige Erwärmung des Materials im Detektionsbereich bewirkt eine Widerstandsänderung und/oder die Erzeugung eines elektrischen Spannungssignals und/oder auch andere Parameteränderungen, wie beispielsweise eine Änderung des Brechungsindex. Die beiden erstgenannten elektrischen Erscheinungen können mit Hilfe der Elektroden der Kontakteinrichtung und ihrer Zuleitungen an eine Messeinrichtung geleitet werden, die Spannungen und/oder elektrische Widerstände misst und die diese Änderungen auswertet und ihnen eine Temperaturerhöhung und/oder eine Absorptionsintensität des Anregungsstrahls in dem Stoff zuordnet. Die Absorptionsintensität kann somit in Abhängigkeit von den durchlaufenen Wellenzahlen/Frequenzen des Anregungsstrahls als Absorptionsspektrum gemessen werden.

Der Detektionsbereich ist vorteilhaft der Messfläche benachbart, das heißt, er ist entweder unmittelbar an diese angrenzend angeordnet oder in einem Abstand von der Messfläche. wobei dieser Abstand gering sein sollte (beispielsweise geringer als 10 Mikrometer oder geringer als 100 Mikrometer, jeweils gemessen am geringsten Abstand des Detektionsbereiches zur Messfläche). Die Detektionsvorrichtung kann beispielsweise auch eine Platte oder einen Körper umfassen, die/der als Teil des Messkörpers mit einem anderen Teil des Messkörpers zusammengefügt ist oder auch eine Beschichtung eines Teils des Messkörpers im Bereich der Messfläche.

Mittels der Detektionsvorrichtung kann, insbesondere in dem Fall, dass eine Druckänderung erfasst wird, beispielsweise bei Verwendung eines Piezo- sensitiven Materials/Piezo- Sensors, auch ein Reaktionssignal in Form einer Schallwelle nachgewiesen werden, die durch die Absorption des Anregungsstrahls in dem zu analysierenden Stoff erzeugt wird und mit bekannter Geschwindigkeit (in menschlichem Gewebe ca. 1500m/s) zur Messfläche und zum Detektionsbereich wandert. Mittels einer Auswerteeinrichtung, die mit einer Modulationseinrichtung für den Anregungsstrahl verbunden ist, kann wegen der guten Zeitauflösung der Messung der Reaktionssignale eine Phasenverschiebung zwischen der Modulation des Anregungsstrahls und dem Reaktionssignal gemessen und dadurch die Tiefe im Gewebe, in der die Absorption stattgefunden hat, ermittelt werden. Da die Signale häufig eine Überlagerung verschiedener Reaktionssignale aus verschiedenen Gewebeschichten sind, können verschiedene Messungen mit verschiedenen Modulationsfrequenzen durchgeführt und die Reaktionssignale zu verschiedenen Modulationsfrequenzen miteinander verknüpft werden, um insbesondere Signale aus oberen Gewebeschichten herauszurechnen und zu eliminieren, da diese durch Verschmutzung und abgestorbene Hautzellen besonders fehleranfällig sind.

Man beachte, dass in der vorliegenden Offenbarung derselbe Begriff "Reaktionssignal" in mehrfacher Hinsicht verwendet wird. Zum einen kann er die physikalische Antwort auf die Anregung durch den Anregungsstrahl bezeichnen, also beispielsweise eine Schallwelle, eine Erwärmung oder dergleichen. Andererseits kann er aber auch ein (typischerweise elektrisches) Signal bezeichnen, welches diese physikalische Antwort repräsentiert, also beispielsweise eine Spannung oder ein Stromfluss, der mithilfe der Elektroden gemessen wird. Zur Einfachheit und Kohärenz der Darstellung wird stets derselbe Begriff "Reaktionssignal" verwendet, wobei aus dem Zusammenhang ohne weitere Erläuterung ersichtlich ist, ob es sich dabei um die physikalische Antwort (beispielsweise eine Druckwelle), eine physikalische Folge dieser physikalischen Antwort (beispielsweise eine Kompression eines piezoelektrischen Materials) oder das zugehörige Messsignal (beispielsweise die von dem piezoelektrischen Material erzeugte Spannung) handelt.

Anstelle verschiedener Modulationsfrequenzen können auch Signale mit steilem Anstieg (im Idealfall sog. Dirac- Impulse) geformt werden, die eine Mischung vieler Modulationsfrequenzen darstellen und eine Analyse zu verschieden Modulationsfrequenzen durch eine Fourieranalyse ermöglichen.

Eine Implementierung der Einrichtung kann vorsehen, dass wenigstens zwei Elektroden, insbesondere entlang einer Flächennormalen der Messfläche, hintereinander in verschiedenen Entfernungen von der Messfläche angeordnet sind.

Die Messfläche kann dabei eben oder gekrümmt sein. Im Falle der Krümmung wird unter der Flächennormalen eine Normale auf der Messfläche an einem Punkt, insbesondere an einer Stelle verstanden, wo der Anregungsstrahl die Messfläche durchsetzt.

Wenigstens zwei Elektroden sollten so angeordnet sein, dass sie den Detektionsbereich ganz oder teilweise umgeben oder dass der Detektionsbereich ganz oder teilweise zwischen den zwei Elektroden liegt. Dabei können die Elektroden in verschiedenen geometrischen Anordnungen auf verschiedenen Seiten des Detektionsbereichs, insbesondere um den Detektionsbreich herum verteilt sein.

Eine weitere Implementierung der Einrichtung kann vorsehen, dass der Anregungsstrahl den Messkörper, insbesondere den Detektionsbereich durchsetzt, wobei insbesondere zur Führung des Anregungsstrahls in oder an dem Messkörper ein Lichtwellenleiter angeordnet und weiter insbesondere der Lichtwellenleiter in den Messkörper integriert ist.

Der Anregungsstrahl gelangt so durch den Messkörper, beispielsweise unmittelbar durch das Material des Messkörpers oder durch einen in diesem angeordneten Lichtwellenleiter hindurch und durch den Detektionsbereich hindurch oder am Detektionsbereich vorbei zu dem Punkt des zu analysierenden Stoffes, der die Anregungsstrahlung absorbiert und die Wärmestrahlung aussendet.

Als Lichtwellenleiter zur Führung des Anregungsstrahls kann ein in das Material des Messkörpers und/oder das Material des Detektionsbereichs integrierter Lichtwellenleiter verwendet werden. Derartige Lichtwellenleiter können beispielsweise im Epitaxialverfahren oder durch selektives Dotieren von Wavermaterial in oder auf einen Waver aufgebracht werden. Es kann jedoch in den Messkörper ein faseroptischer Lichtwellenleiter eingebracht oder auch wenigstens teilweise außen auf diesen aufgebracht werden.

Es kann auch vorgesehen sein, dass der Anregungsstrahl die Messfläche in einem Bereich durchsetzt, der dem Detektionsbereich unmittelbar benachbart ist und/oder an diesen angrenzt.

Eine weitere Implementierung der Einrichtung kann vorsehen, dass eine Modulationseinrichtung zur Modulation der Intensität des Anregungsstrahls vorgesehen ist. Dabei sind verschiedene Arten der Modulation möglich, durch einen mechanischen Chopper ebenso wie durch eine steuerbare Blenden- oder Ablenkspiegelvorrichtung oder einen Körper/eine Schicht, deren Transmission steuerbar ist. Zudem kann eine Modulation auch unmittelbar durch die Ansteuerung der Anregungslichtquelle/Laserlichtquelle erreicht werden. Eine Messung kann dabei die Aufnahme von Spektren bei einer oder mehreren Modulationsfrequenzen vorsehen, wobei Messungen bei verschiedenen Modulationsfrequenzen miteinander zur Gewinnung von Tiefeninformationen und/oder zur Eliminierung von Messdaten aus bestimmten Tiefenbereichen der Probe/des zu analysierenden Stoffes, verknüpft werden können. Insbesondere können somit Messdaten, die von der Oberfläche des Stoffes stammen, eliminiert werden. Dies sind Messdaten und Wärmewellen, die von Absorptionen des Anregungslichtstrahls an der Oberfläche des Stoffes herrühren und die von Verschmutzungen oder Anomalien des zu analysierenden Stoffes an seiner Oberfläche herrühren können. Ein Beispiel hierfür sind bei der Analyse der Haut eines Patienten die obersten Hautschichten, die teilweise aus abgestorbenen Zellen bestehen, welche eine nicht aussagekräftige Zusammensetzung aufweisen und/oder falsche Informationen liefern. Dies ist insbesondere dann der Fall, wenn in der Haut biologisch aktive Stoffe, aktiver Stoffwechsel oder Stoffwechselprodukte oder ähnliches nachgewiesen werden sollen.

Im Folgenden sind verschiedene geometrische Elektrodenkonstellationen aufgeführt, deren Vorteile diskutiert werden.

Hierzu kann beispielsweise vorgesehen sein, dass wenigstens zwei, insbesondere wenigstens drei oder vier, weiter insbesondere wenigstens 6, weiter insbesondere wenigstens 8 Elektroden hintereinander in verschiedenen Abständen von der Messfläche oder in Richtung senkrecht zu einer Flächennormalen der Messfläche voneinander beabstandet angeordnet sind.

Dabei ist/sind auf jeder Seite des Detektionsbereiches wenigstens eine oder mehrere der Elektroden angeordnet. Es stehen somit verschiedene Elektrodenpaare zur Verfügung, mit denen ein elektrischer Widerstand oder eine elektrische Spannung gemessen werden kann.

Es ist hierzu eine elektronische Einrichtung vorgesehen, die über die Zuleitungen mit mehreren oder allen Elektroden verbunden ist. Die elektronische Einrichtung weist eine Steuereinrichtung und insbesondere auch eine Auswerteeinrichtung auf, die die Erfassung und Auswertung von elektrischen Widerständen oder Spannungen zwischen verschiedenen wählbaren Elektroden nacheinander oder gleichzeitig bewirkt/bewirken.

Es können dazu verschiedene oder alle Paarungen von Elektroden versuchsweise ausgewählt werden. Die jeweils ausgewählten Elektroden eines Elektrodenpaares für eine versuchsweise Messung sollten dabei jeweils wenigstens einen Teil des Detektionsbereichs zwischen sich haben. Messwerte von einzelnen Elektrodenpaarungen können miteinander verglichen und bewertet werden. Bewertungsgrößen können beispielsweise die Signalstärke oder die Größe der Signaländerungen oder ein Signal/Rausch- Verhältnis oder eine andere Größe sein. Es soll hier neben der Integration der Elektroden in den Messkörper auch der Fall erfasst sein, dass mehrere Elektroden gestaffelt auf die Oberfläche des Messkörpers/Messfläche aufgeklebt oder dort eingelassen oder aufgedampft sind.

Es kann außerdem vorgesehen sein, dass wenigstens zwei, insbesondere wenigstens drei oder vier, weiter insbesondere wenigstens 6, weiter insbesondere wenigstens 8 Elektroden in Richtung einer Flächennormalen der Messfläche oder senkrecht dazu oder in einer Richtung zwischen 0 und 90 Grad zur Flächennormalen hintereinander in verschiedenen Abständen vom Detektionsbereich, insbesondere in verschiedenen Abständen von der Mitte des Detektionsbereichs, angeordnet sind. Der Detektionsbereich kann dabei zum Beispiel durch einen aus einem besonderen Material bestehenden räumlichen Bereich des Messkörpers definiert sein. Der Detektionsbereich kann auch durch die Gesamtanordnung als ein oberhalb des Eintrittsstelle des Anregungsstrahls in den zu analysierenden Stoff liegender Bereich des Messkörpers definiert werden.

Zudem kann der Detektionsbereich auch durch die Summe der Raumpunkte definiert sein, an denen ein elektrisches Signal durch die zur Verfügung stehenden Elektroden potenziell nachweisbar ist.

Eine weitere Implementierung kann vorsehen, dass wenigstens zwei, insbesondere wenigstens drei oder vier, weiter insbesondere wenigstens 6, weiter insbesondere wenigstens 8 Elektroden in einem kreisringförmigen Bereich oder einem kugelschalenförmigen Bereich um den Detektionsbereich herum und wenigstens teilweise einander auf verschiedenen Seiten des Detektionsbereiches gegenüberliegend angeordnet sind, wobei verschiedene Elektroden jeweils im wesentlichen denselben Abstand von der Mitte des Detektionsbereichs oder unterschiedliche Abstände von der Mitte des Detektionsbereichs aufweisen. Hierbei soll der Fall von auf der Oberfläche des Messkörpers verteilt angeordneten Elektroden mit umfasst sein.

Durch die genannte Elektrodenverteilung kann beispielsweise durch Messung an mehreren Elektrodenpaaren das oder die am besten geeignete Elektrodenpaar(e) zur Messung ausgewählt und benutzt werden.

Als Maß für die Qualität der Messung kann für die Auswahl der Elektrodenpaare beispielsweise entweder eine Signalstärke oder eine Signaldynamik oder ein Rauschen oder ein Signal/rausch-Verhältnis dienen.

Es kann zudem vorgesehen sein, dass eine oder mehrere oder alle der Elektroden der Kontakteinrichtung scheiben- oder plattenförmig, ringförmig, ringscheibenförmig, in Form eines viereckigen oder polygonalen Rahmens mit einer Öffnung, kalottenförmig oder strangförmig ausgebildet sind.

Eine Implementierung der Einrichtung kann vorsehen, dass eine oder mehrere oder alle der Elektroden der Kontakteinrichtung auf einer Oberfläche des Messkörpers oder der Detektionsvorrichtung angeordnet und insbesondere mittels eines Fügeverfahrens, weiter insbesondere durch Kleben oder Schweißen aufgebracht sind. Auch ein Aufbringen durch Aufdampfen oder aufstreichen ist denkbar. Dabei kann der Messkörper homogen aus einem einzigen Material bestehen oder im Detektionsbereich aus einem besonderen Material bestehen, das sich von dem Material der übrigen Bereiche des Messkörpers unterscheidet.

Die Elektroden bestehen für alle Ausführungsformen in der Regel aus einem Metall, jedenfalls aus einem elektrisch gut leitenden Stoff. Sie können auch aus einem elektrisch leitenden Kunststoff oder einem leitend gefüllten Stoff bestehen.

Eine weitere Implementierung der Einrichtung kann vorsehen, dass eine oder mehrere oder alle der Elektroden der Kontakteinrichtung im Inneren oder an der Außenseite des Messkörpers in einer oder mehreren Ausnehmungen, beispielsweise Bohrungen, Mulden oder Nuten des Messkörpers angeordnet sind, wobei sie insbesondere eingelegt, eingegossen, durch Spritzgießen oder durch ein additives Fertigungsverfahren (3D-Druck) eingebracht sind. Diese Varianten ermöglichen auf technisch einfache Art das Einbringen von Elektroden in einen Messkörper.

Es können auch Elektroden in dem Messkörper dadurch geschaffen werden, dass Bereiche des Materials des Messkörpers durch Bestrahlung oder Beschuss mit Teilchen elektrisch leitend gemacht werden. Dies ist zum Beispiel bei Kunststoffen möglich, bei denen durch hochenergetische Strahlung Kohlenstoffmoleküle teilweise zerstört und Kohlenstoffansammlungen gebildet werden, die leitfähig sind.

Außerdem kann vorgesehen sein, dass der Messkörper als Flachkörper, insbesondere als planparalleler Körper in Form einer Platte ausgebildet ist, wobei insbesondere die Dicke senkrecht zur Messfläche weniger als 50% der geringsten Ausdehnung des Messkörpers in einer in der Messfläche verlaufenden Richtung beträgt, insbesondere weniger als 25%, weiter insbesondere weniger als 10%.

Diese Ausführungsvariante des Messkörpers kann für verschiedene Detektionsverfahren der Wärmereaktionssignale verwendet werden und ist grundsätzlich nicht auf die Detektionsmethode mittels eines Piezoeffektes beschränkt. Sie kann auch bei der Messmethode mit einem an der Messfläche reflektierten Messstrahl und einem Detektor für dessen Ablenkung verwendet werden und lässt eine starke Reduzierung der Ausmaße des Messkörpers in Richtung senkrecht zur Messfläche zu. Unabhängig von der Messmethode, beispielsweise unter Verwendung des Piezoeffekts oder mittels der Messung der Ablenkung eines reflektierten Strahls, kann der als Flachkörper ausgebildete Messkörper im Bereich der Messfläche eine Schicht aufweisen, die im Falle der Piezo- Messmethode aus einem piezoelektrischen Material bestehen kann und im Fall der Detektion mittels Messung der Ablenkung eines reflektierten Messstrahls aus einem Material, bei dem sich der Brechungsindex in Abhängigkeit von der Temperatur stärker ändert als beim Material der übrigen Bereiche des Messkörpers.

Eine weitere Implementierung der Einrichtung kann vorsehen, dass der Messkörper eine Spiegeleinrichtung zur Reflexion des von der Anregungsstrahlquelle, insbesondere Lasereinrichtung eingestrahlten Anregungsstrahls auf die Messfläche aufweist oder eine solche Spiegeleinrichtung trägt.

Bei einem sehr flachen Messkörper kann der Anregungsstrahl von der Flachseite des Messkörpers her eingestrahlt werden. Der Anregungsstrahl breitet sich dann von der Anregungsstrahlquelle zunächst im Wesentlichen parallel zur Messfläche innerhalb des Messkörpers oder parallel zu den Grenzflächen des Messkörpers aus und wird dann zur Messfläche hin umgelenkt.

Anstelle einer Spiegelung des Anregungsstrahls kann dieser auch durch geeignete Materialgestaltung des Messkörpers zur Messfläche hin gebeugt werden. Der Flachkörper kann auch optische Fokussierelemente für den Anregungsstrahl aufweisen oder mit solchen Elementen, beispielsweise einer oder mehreren Linsen, verbunden sein.

Es kann auch vorgesehen sein, dass der Anregungsstrahl parallel zur Messfläche oder in einem Winkel von weniger als 30 Grad, insbesondere weniger als 20 Grad, weiter insbesondere weniger als 10 Grad oder weniger als 5 Grad zur Messfläche in den Messkörper eingestrahlt wird und dass der Anregungsstrahl in Richtung der Messfläche umgelenkt oder abgelenkt wird und diese durchsetzt.

In den Messkörper integriert oder mit diesem unmittelbar verbunden kann insbesondere bei Verwendung eines Flachkörpers auch eine Fokussiereinrichtung, beispielsweise in Form eines Beugungselementes/einer Linse vorgesehen sein, die den Anregungsstrahl auf die Messfläche und die Stoffoberfläche des zu analysierenden Stoffs fokussiert.

Es kann zudem vorgesehen sein, dass der Anregungsstrahl das Material des Messkörpers durchsetzt.

Es kann auch vorgesehen sein, dass der Messkörper wenigstens eine Ausnehmung, insbesondere eine Bohrung aufweist, die durch den Anregungsstrahl durchsetzt ist, wobei sich die Ausnehmung und/oder Bohrung insbesondere von der Messfläche ausgehend in den Messkörper erstreckt oder wobei die Ausnehmung oder Aussparung und/oder Bohrung den gesamten Messkörper von einer der Messfläche gegenüberliegenden Begrenzungsfläche des Messkörpers bis zu der Messfläche durchsetzt. Die Ausnehmung kann sich als Kanal oder Bohrung mit ihrer Längsachse auch wenigstens teilweise in Richtung des Anregungsstrahls parallel zur Messfläche erstrecken. Ist eine Sensor-Schicht vorgesehen, so kann die Ausnehmung, Aussparung und/oder Bohrung sich von der der Messfläche gegenüberliegenden Begrenzungsfläche der Sensor-Schicht ausgehend in den Messkörper hinein erstrecken, so dass die Sensor-Schicht selbst von der Ausnehmung nicht durchsetzt ist.

In diesem Fall kann der Messkörper einen hohlen Kanal/eine Bohrung/Ausnehmung für den Anregungsstrahl aufweisen, so dass der Anregungsstrahl das Material des Messkörpers nicht durchsetzt, obwohl der Anregungsstrahl durch den Messkörper und die Messfläche hindurch zu dem zu analysierenden Stoff gelangt.

Es kann auch vorgesehen sein, dass der Messkörper einen ersten Teil aufweist, der eine durchgehenden Kanal für den Anregungsstrahl aufweist und dass der Messkörper an seiner Unterseite auf dem ersten Teil eine Sensor-Schicht aufweist, die entweder durchgehend ohne Ausnehmung oder mit einer Fortsetzung der Ausnehmung des ersten Teils versehen ist. Ist die Sensor- Schicht dünner als 200 Mikrometer, insbesondere dünner als 100 Mikrometer, so kann der Anregungsstrahl diese - auch wenn es sich um einen Infrarotstrahl handelt - ohne zu große Absorption durchlaufen, und eine Ausnehmung, Bohrung oder ein Kanal in der Sensor- Schicht ist nicht notwendig. Die Sensor- Schicht des Messkörpers kann aus einem Material bestehen, das piezoelektrische Eigenschaften aufweist und einen Detektionsbereich gemäß der Erfindung ausbildet. Die Sensorschicht kann auch aus einem Material bestehen, bei dem eine Temperatur- und/oder Druckänderung eine Änderung des Brechungsindex bewirkt, so dass auch diese Änderung als Reaktionssignal, beispielsweise durch die Detektion des Reflexionswinkel eines Detektionsstrahls, der in der Sensor- Schicht reflektiert wird, nachgewiesen werden kann. Der erste Teil des Messkörpers kann dann beispielsweise aus einem Material wie Quarz oder Saphir bestehen, das im sichtbaren Bereich und für einen Detektionsstrahl durchlässig ist, jedoch im infraroten Spektralbereich weniger durchlässig oder undurchlässig ist.

Eine weitere Implementierung der Einrichtung kann vorsehen, dass in oder an dem Messkörper, insbesondere in der Detektionsvorrichtung, oder unmittelbar an diesen/dieses angrenzend und mit diesem im thermischen Kontakt stehend wenigstens eine Wärmesenke in Form eines Körpers angeordnet ist, dessen spezifische Wärmekapazität und/oder spezifische Wärmeleitfähigkeit größer ist als die spezifische Wärmekapazität und/oder spezifische Wärmeleitfähigkeit des Materials, aus dem der Messkörper besteht oder der als Peltier-Element ausgebildet ist.

Anstelle einer Wärmesenke in Form eines Körpers, dessen spezifische Wärmekapazität und/oder spezifische Wärmeleitfähigkeit größer ist als die spezifische Wärmekapazität und/oder spezifische Wärmeleitfähigkeit des Materials, aus dem der Messkörper besteht, kann auch ein aktives oder passives Kühlelement, insbesondere ein Peltier-Kühlelement zur Einstellung eines Temperaturgefälles vorgesehen sein. Das Temperaturgefälle oder die Absoluttemperatur kann mittels des Peltierelementes auch mit einer Regeleinrichtung geregelt werden.

Mit derartigen Wärmesenken, beispielweise in Form von Metall oder Kristallkörpern oder aktiv betriebenen Peltierelementen, lassen sich die geeigneten thermischen Eigenschaften des Messkörpers bezüglich der thermischen Diffusivität realisieren, die erforderlich sind, damit die Temperaturänderung sich einerseits mit der Modulationsfrequenz in dem Detektionsbereich genügend aufbaut und andererseits die Wärme auch ausreichend schnell abtransportiert wird. Dies hängt natürlich in erster Linie vom Material des Messkörpers/ der Detektionsvorrichtung ab, ist jedoch durch geeignete Hinzufügung von einer oder mehreren Wärmesenken beeinflussbar. Diese können beispielsweise wenigstens teilweise um den Detektionsbereich herum angeordnet oder auch an einer Seite des Detektionsbereiches vorgesehen sein.

Eine weitere Implementierung der Einrichtung kann vorsehen, dass in oder an dem Messkörper insbesondere in der Detektionsvorrichtung, oder unmittelbar an diesen/dieses angrenzend und mit diesem im thermischen Kontakt stehend wenigstens eine Wärmebarriere in Form eines Körpers angeordnet ist, dessen spezifische Wärmekapazität und/oder spezifische Wärmeleitfähigkeit geringer ist als die spezifische Wärmekapazität und/oder spezifische Wärmeleitfähigkeit des Materials, aus dem der Messkörper besteht.

Mit derartigen Wärmebarrieren, allein oder auch in Kombination mit Wärmesenken, lassen sich die geeigneten thermischen Eigenschaften des Messkörpers bezüglich der thermischen Diffusivität realisieren, die erforderlich sind, damit die Temperaturänderung sich einerseits mit der Modulationsfrequenz in dem Detektionsbereich genügend aufbaut und andererseits die Wärme auch entsprechend der Modulationsfrequenz ausreichend schnell abtransportiert wird. Dies ist durch geeignete Hinzufügung von einer oder mehreren Wärmebarrieren beeinflussbar. Diese können beispielsweise wenigstens teilweise um den Detektionsbereich herum angeordnet oder auch an einer Seite des Detektionsbereiches vorgesehen sein. Wärmebarrieren können beispielsweise durch thermisch isolierende Kunststoffelemente realisiert sein. Beispielsweise kann auf einer ersten Seite des Detektionsbereichs eine oder mehrere Wärmesenken und auf einer der ersten gegenüberliegenden zweiten Seite des Detektionsbereichs eine oder mehrere Wärmebarrieren vorgesehen sein, um einen Temperaturgradienten zu erzeugen und die Richtung des Wärmetransports zu beeinflussen.

Es kann vorgesehen sein, dass die Detektionsvorrichtung und/oder der Messkörper wenigstens teilweise aus einem piezoelektrischen Material, insbesondere einer piezoelektrischen Keramik, insbesondere einer PZT- Keramik, weiter insbesondere einer gesinterten Keramik, oder einem einkristallinen piezoelektrischen Material, insbesondere Quarz, Turmalin, Lithiumniobat, Galliumorthophosphat, Berlinit, Seignettesalz, Ferroelektrika wie Bariumtitanat (BTO) oder Blei-Zirkonat-Titanat, Galliumphosphat oder einem Blei-Magnesium-Niobat, oder Zinkoxid (ZnO) oder Aluminiumnitrid als Dünnschichtabscheidung oder polarisiertem Polyvinylidenfluorid besteht.

Das jeweilige Material sollte im Infrarotfrequenzbereich, vorteilhaft im mittleren Infrarotfrequenzbereich möglichst transparent sein.

Die genannten piezoelektrischen Materialien können als dünne Schicht auf einem Messkörper vorgesehen sein und die Messfläche ausbilden. Die Schichtdicke sollte dann geringer als 0,5 mm, insbesondere kleiner als 300 Mikrometer sein und/oder eine Ausnehmung oder Aussparung, beispielsweise eine Bohrung oder einen Kanal für den Anregungsstrahl aufweisen. Der übrige Teil des Messkörpers kann dann nicht piezoelektrisch und transparent für den Anregungsstrahl sein und/oder eine Ausnehmung für den Anregungsstrahl aufweisen. Dieser übrige Teil des Messkörpers kann für die Beschichtung mit einem piezoelektrischen Material als Wärmesenke wirken, das heißt, eine größere spezifische Wärmekapazität und/oder Wärmeleitfähigkeit aufweisen als die piezoelektrische Schicht.

Eine weitere Implementierung der Einrichtung kann vorsehen, dass ein piezoelektrisches Element oder ein piezoelektrischer Bereich des Messkörpers als Aktor mit einer Spannungsquelle verbindbar ist und in Abhängigkeit von einer steuerbaren Eingangsspannung für einen Anregungsstrahl eine Blockade darstellt.

Auf diese Weise kann das Material des Messkörpers durch Änderungen der Dielektrizitätskonstante auch als optischer Chopper für den Anregungsstrahl verwendet werden.

Ein Verfahren zum Betrieb einer Einrichtung gemäß der Erfindung kann vorsehen, dass ein modulierter Anregungsstrahl, insbesondere durch den Messkörper hindurch, auf den zu analysierenden Stoff gerichtet wird und dass gleichzeitig oder nacheinander Signale von verschiedenen Elektrodenpaaren der Kontakteinrichtung erfasst und ausgewertet werden, dass zunächst anhand von vorgegebenen Kriterien ermittelt wird, welche(s) der Elektrodenpaare zur Weiterverarbeitung geeignete Signale liefert/liefern und dass darauf die Signale von einem oder mehreren ausgewählten Elektrodenpaaren zur Messung verwendet und ausgewertet werden und dass insbesondere eine nachfolgende Messung durchgeführt wird, bei der die Signale des oder der ausgewählten Elektrodenpaare erfasst und ausgewertet werden.

Geeignete Signale lassen sich beispielsweise aufgrund der Stärke der Signale, des Signal/Rauschverhältnis oder der Steilheit selektieren, mit der die Signale der Modulation des Anregungsstrahles folgen. Mit der Auswahl der geeigneten Elektroden kann man gegebenenfalls auch eine Fehlausrichtung des zu analysierenden Stoffes in Bezug auf die Messeinrichtung feststellen und korrigieren, wenn z.B. die Wärmewelle nicht in die Mitte des Messbereiches gelangt. Dann werden möglicherweise anders verteilte Elektroden für die aktuelle Messung ausgewählt.

Der Anregungsstrahl wird bei der Messung bezüglich der Wellenzahl/Wellenlänge/Frequenz kontinuierlich oder in schritten Verändert oder es werden charakteristische Wellenlängenbereiche abgefahren. Bei Verwendung eines Arrays kann auch ein Anregungsstrahl in verschiedenen Wellenlängen oder Wellenlängenbereichen gleichzeitig oder nacheinander durch verschiedene Elemente eines Arrays ausgesandt werden.

Es kann in einer Implementierung des Verfahrens auch vorgesehen sein, dass nach einem ersten Messversuch in Abhängigkeit von den erfassten Signalen eine Fehlausrichtung der Einrichtung relativ zu dem zu analysierenden Stoff ermittelt und signalisiert wird und insbesondere der Nutzer zu einer Neuausrichtung aufgefordert wird.

Dieses Verfahren kann sogar auch parallel zu einer anderen Detektionsmethode, beispielsweise mit einem reflektierten Detektionsstrahl, angewendet werden, nur um Fehlausrichtungen eines Fingers, an dem eine Messung vorgenommen werden soll, zu ermitteln und zu signalisieren.

Fehlausrichtungen können beispielsweise dadurch erkannt werden, dass die Signalstärken verschiedener Paare von Elektroden bei einer ersten Messung in Form eines Profils/Vektors ermittelt werden und dieses Profil/dieser Vektor mit entsprechenden Größen früherer Messungen oder festgelegten Referenzgrößen verglichen wird. Dabei kann das Profil jeweils auf eine Signalstärke normiert werden. Überschreitet der Unterschied zu einem Referenzprofil bei bestimmten Elementen des Profils oder bezüglich der Asymmetrie bestimmte Schwellen so kann auf eine Fehlausrichtung geschlossen werden.

In einer weiteren Implementierung eines Verfahrens kann auch vorgesehen sein, dass
- mit einer Anregungssendeeinrichtung mindestens ein intensitätsmodulierter elektromagnetischer Anregungsstrahl mit zumindest einer Anregungswellenlänge erzeugt wird, die Anregungssendeeinrichtung den mindestens einen elektromagnetischen Anregungsstrahl in ein Stoffvolumen einstrahlt, das unterhalb der Oberfläche des Stoffs liegt,
- mit einer Detektionseinrichtung ein Reaktionssignal detektiert wird, und
- anhand des detektierten Reaktionssignals der Stoff analysiert wird, wobei insbesondere
- nacheinander unter Verwendung verschiedener Modulationsfrequenzen der Anregungssendeeinrichtung Reaktionssignale, insbesondere zeitliche Reaktionssignalverläufe für verschiedene Wellenlängen des Anregungsstrahls ermittelt und
- mehrere Reaktionssignalverläufe zu verschiedenen Modulationsfrequenzen miteinander verknüpft werden und wobei
- daraus insbesondere eine für einen Tiefenbereich unter der Stoffoberfläche spezifische Information gewonnen wird.

Die Figuren 1 bis 17 zeigen schematisch verschiedene Elemente der Vorrichtung und ihrer Elemente teilweise in unterschiedlichen Ausführungsformen.,
weiter zeigen
Figur 18: einen Querschnitt eines Messkörpers mit einer ersten integrierten Linse und mit einem auf die Messfläche aufgelegten Finger,
Figur 19 einen Querschnitt eines Messkörpers mit einer zweiten integrierten Linse,
Figur 20 einen Querschnitt eines Messkörpers mit einer dritten integrierten Linse,
Figur 21 einen Querschnitt eines Messkörpers mit einer ersten integrierten Linse und einem Anregungsstrahl,
Figur 22 einen Querschnitt eines Messkörpers mit einer zweiten integrierten Linse und einem Anregungsstrahl,
Figur 23 einen Querschnitt eines Messkörpers mit einer dritten integrierten Linse und einem Anregungsstrahl, sowie
Figuren 24, 25, 26 mehrere Anordnungen mit einem Messkörper und einer Anregungslichtquelle in Form einer Laser-Lichtquelle oder Anregungsstrahlquelle, insbesondere einer Lasereinrichtung, wobei der Anregungslichtstrahl mittels eines in ein Substrat des Messkörpers integrierten Lichtwellenleiters durch den Messkörper zur Messfläche geführt ist.

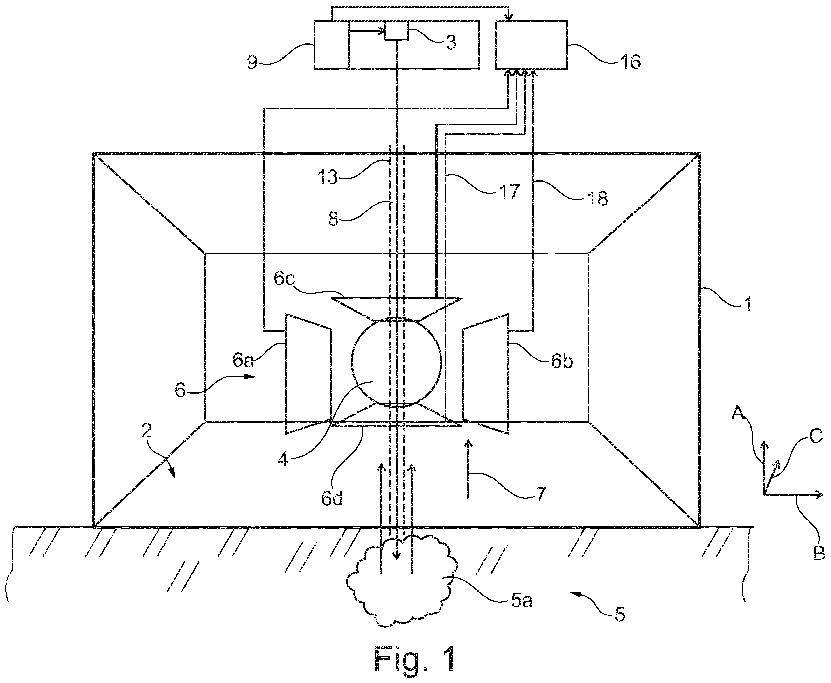

Die Figur 1 zeigt ein Ausführungsbeispiel für eine Einrichtung zum Analysieren eines Stoffes. Der Stoff 5 liegt vorzugsweise unmittelbar auf einem Messkörper 1 auf oder umgekehrt, jedenfalls sind der Stoff und eine Messfläche 2 des Messkörpers 1, der in der vorliegenden Offenbarung auch als "optisches Medium" bezeichnet wird, für einen Messvorgang in unmittelbarem körperlichen Kontakt. Der Messkörper 1 kann als für Licht transparenter oder zumindest im Infrarotbereich transparenter Festkörper, insbesondere Kristall oder Glaskörper oder Kunststoffkörper, insbesondere Polymerkörper, ausgebildet sein, der insbesondere im Infrarotbereich transparent ist, beispielsweise wenn die Einrichtung zum Messen des Glukose- bzw. Blutzuckergehalts in einer Flüssigkeit, wie in einer Ausführungsform beispielsweise in Blut, vorgesehen ist. Die Einrichtung kann dann zur Erzeugung einer Glukose- bzw. Blutzuckerspiegelangabe genutzt werden.

Die Einrichtung umfasst eine Anregungssendeeinrichtung 3 in Form eine Anregungsstrahlquelle, insbesondere eine Lasereinrichtung zum Aussenden eines oder mehrerer elektromagnetischer Anregungsstrahlen, vorzugsweise in Form von Anregungs-Lichtstrahlen mit einer oder mehreren Anregungswellenlängen, in ein Volumen 5a, das in dem Stoff 5 unterhalb eines ersten Bereiches der Oberfläche des Stoffes liegt. Die Anregungssendeeinrichtung 3 wird nachfolgend auch kurz als Lasereinrichtung bezeichnet. Bei der Lasereinrichtung kann es sich um einen bezüglich der Wellenlänge durchstimmbaren Laser, insbesondere durchstimmbaren Quantenkaskadenlaser, handeln; bevorzugt wird, wie weiter unten noch erläutert, eine Lichtquellenleiste oder ein Lichtquellenarray mit zumindest zwei Einzelemittern in Form von Lasern, insbesondere Halbleiterlasern mit festen Wellenlängen, oder Licht emittierenden Halbleiterdioden eingesetzt, mit denen jeweils eine vorgegebene individuelle Wellenlänge oder Licht in einem definierten engen Wellenlängenbereich emittiert wird, wobei auch die Möglichkeit besteht, Lichtquellen zu verwenden, die gleichzeitig oder nacheinander mit geeigneten Filtern kombiniert und in Reihe geschaltet werden, um bestimmte Wellenlängen oder Wellenlängenbereiche auszusondern. Werden mehrere Einzelemitter kombiniert, so können die einzelnen Anregungslichtstrahlen durch einen Multiplexer gemeinsam in einen Lichtweg eingekoppelt werden, beispielsweise in einen Lichtwellenleiter oder einen ausgenommenen Kanal oder einen sonstigen Lichtweg im optischen Medium. Auch ein Kollimator kann vorgesehen sein, um die von verschiedenen Emittern ausgesandten Lichtstrahlen möglichst parallel zueinander auszurichten und möglichst zu einem einzigen Strahl zu kombinieren, und zwar ebenso im Fall von mehreren gleichzeitig ausgestrahlten Lichtstrahlen wie auch bei mehreren nacheinander ausgesandten Lichtstrahlen.

Auf dem Weg des Anregungslichtes kann auch ein optisches Element zur Fokussierung des Anregungslichtes vorgesehen sein. Dieses kann beispielsweise zwischen der Lasereinrichtung und dem Messkörper oder am Messkörper selbst dort, wo der Anregungsstrahl in ihn eintritt, oder auch am Messkörper in dem Bereich vorgesehen sein, wo der Anregungsstrahl den Messkörper verlässt, zum Beispiel im Bereich der Messfläche, an der Messfläche, bündig mit der Messfläche oder zwischen der Messfläche und der Detektionsvorrichtung.

Das optische Element kann beispielsweise als konvexe Linse aus dem Material des Messkörpers herausgearbeitet sein oder auch aus einem Material bestehen, das von dem Material des Messkörpers verschieden ist.

Außerdem ist eine Einrichtung 9 zur Intensitätsmodulierung des oder der Anregungsstrahlen/Anregungslichtstrahlen vorgesehen, die vorzugsweise durch eine Modulationseinrichtung für die Anregungsstrahlquelle, insbesondere Lasereinrichtung insbesondere ihrer Ansteuerung, und/oder wenigstens einen im Strahlengang angeordneten gesteuerten Spiegel und/oder eine bzgl. ihrer Transparenz steuerbare und im Strahlengang angeordnete Schicht gebildet ist.

Eine nach einer Absorption des Anregungsstrahls in dem Bereich 5a des Stoffes ausgesandte Wärmewelle läuft in den Messkörper hinein und kann dort in einem Detektionsbereich 4 durch eine Detektionsvorrichtung nachgewiesen werden. Dies geschieht durch Nachweis einer lokalen Temperaturerhöhung oder Temperaturänderung, die zeitlich der Absorption sehr schnell folgt. Auch die Rückänderung der Temperaturänderung (Absinken der Temperatur) nach dem Ende einer Absorptionsphase (wenn im Rahmen der Modulation des Anregungsstrahls die Intensität des Anregungsstrahls absinkt) folgt dem Intensitätsverlauf der Absorptionsintensität sehr schnell mit einem gewissen Phasenversatz, der von der Tiefe abhängt, in der in dem Stoff der Anregungsstrahl absorbiert wird.

Die Amplitude des Reaktionssignals hängt hierbei von der Wellenlänge des Anregungsstrahls, den Absorptionseigenschaften der Probe, sowie den thermischen Eigenschaften, insbesondere der thermischen Diffusivität und thermischen Leitfähigkeit der Probe als auch des Messkörpers/optischen Mediums 1 ab. Darüber hinaus spielt auch die Kopplung des thermischen Signals von der Probe in den Messkörper hinein eine Rolle.

In dem gezeigten Ausführungsbeispiel ist die Detektionsvorrichtung 4,6 als Bereich 4 des Messkörpers 1 ausgebildet, der wenigstens teilweise oder abschnittsweise aus einem piezoelektrischen Material besteht, wobei die Detektionsvorrichtung 4,6 zudem Elektroden 6a, 6b, 6c und 6d aufweist, die auf jeweils einander gegenüberliegenden Seiten des Detektionsbereiches 4 angeordnet sind. Die Elektroden 6a bis 6d stellen einen elektrischen Kontakt mit dem Material des Detektionsbereichs 4 her und werden im Folgenden gemeinsam als "Kontakteinrichtung" 6 bezeichnet. Auf diese Weise lässt sich eine Temperatur oder Temperaturänderung je nach der Materialwahl des piezoelektrischen Materials durch eine zwischen den Elektroden erzeugte Piezo- Spannung oder durch einen elektrischen Widerstand oder eine Widerstandsänderung nachweisen.

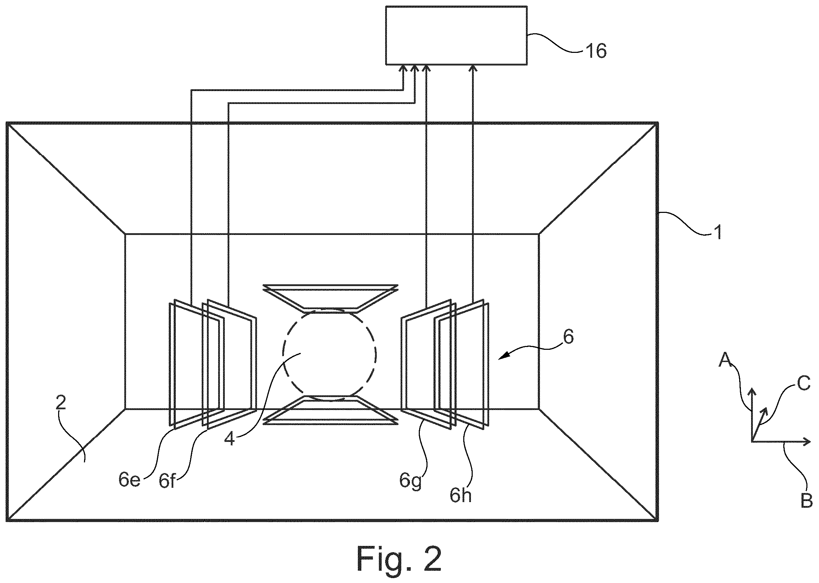

Dabei sind in dem Beispiel der Figur 1 die beiden rechteckigen, flächenförmigen oder plattenförmigen, ebenen Elektroden 6c und 6d entlang der Flächennormalen 7 der Messfläche 2 in verschiedenen Entfernungen von der Messfläche 2 parallel zueinander angeordnet. Die beiden ebenfalls ebenen, plattenförmigen Elektroden 6a und 6b sind in Richtung des Pfeils B senkrecht zur Richtung der Flächennormalen 7 und parallel zur Messfläche 2 voneinander beabstandet und parallel zueinander angeordnet. Das aus den Pfeilen A, B und C gebildete Koordinatensystem, bei dem der Pfeil A parallel zur Flächennormalen 7 der Messfläche 2 ist und die Pfeile B und C senkrecht dazu ausgerichtet sind, ist ebenso wie in Fig. 1 in den Figuren 2 bis 15 zur Orientierung eingezeichnet.

Eine Auswerteeinrichtung 16 zum Analysieren des Stoffes, die als elektronische Einrichtung, insbesondere digitale Verarbeitungseinrichtung, beispielsweise als Microcontroller oder Prozessor oder als Computer ausgebildet ist, steht über elektrische Leitungen 17, 18 im elektrischen Kontakt mit den Elektroden 6a, 6b, 6c und 6d der Kontakteinrichtung 6, wertet die detektierten Reaktionssignale aus und erzeugt in einer Ausführungsform eine Glukose- bzw. Blutzuckerspiegelangabe (BZA).

Dabei steht die Auswerteeinrichtung 16 auch mit der Modulationseinrichtung 9 in elektrischer Verbindung, so dass die Information über die Frequenz/Wellenlänge des Anregungsstrahls und insbesondere auch die Frequenz und/oder Phase der Modulation jeweils in der Auswerteeinrichtung 16 zur Verfügung steht und bei der Auswertung berücksichtigt werden kann. Auf diese Weise kann beispielsweise auch der Phasenversatz der Reaktionssignale zur Modulationsfunktion des Anregungsstrahls ausgewertet werden, um eine Information darüber zu erhalten, in welcher Tiefe in dem Stoff, das heißt auch, in welcher Entfernung von der Messfläche 2 oder dem Detektionsbereich 4 das Reaktionssignal erzeugt worden ist. Dadurch lassen sich Informationen über eine Tiefenprofilierung der Verteilung einer nachgewiesenen Substanz, beispielsweise Glucose, in dem Stoff 5 gewinnen.

Die Informationen über die Modulation des Anregungsstrahls können von der Modulationseinrichtung 9 zu der Auswerteeinrichtung 16 gesandt werden, es kann jedoch auch vorgesehen sein, dass die Auswerteeinrichtung 16 die Modulation unmittelbar steuert. Die Auswerteeinrichtung 16 kann zur Auswertung auch einen Lock-in-Verstärker aufweisen, der die Signale spezifisch bei der Modulationsfrequenz auswertet.

Die gezeigte Anordnung der Elektrodenpaare 6a/6b, 6c/6d ist nur beispielhaft. Es kann auch ein einzelnes Elektrodenpaar ausreichen, wobei wichtig ist, dass wenigstens ein Teil des Detektionsbereiches 4 zwischen den beiden Elektroden liegen muss. Zudem muss für eine optimale Funktion der zu analysierende Stoff, das heißt beispielsweise ein Finger eines Probanden, an der dafür vorgesehenen Stelle an der Messfläche 2 anliegen. Ein seitlicher Versatz des Fingers/Stoffes kann dazu führen, dass der Wärmeimpuls seine Wirkung nicht genau zwischen den Elektroden entfaltet und die Messwerte suboptimal oder falsch sind.

Die Elektroden 6a, 6b, 6c, 6d können in oder an den Messkörper 1 durch ein additives Verfahren (3d-Druck), durch Eingießen, Aufdampfen, Dotieren, gezielte Veränderung des Ursprungsmaterials des Messkörpers (beispielsweise Umwandlung von Kohlenwasserstoffen in elektrisch leitenden Kohlenstoff durch Korpuskularstrahlung oder Gammastrahlung oder Laserstrahlung), Ankleben oder Einführen in vorher eingebrachte Ausnehmungen oder Aussparungen ein- oder angefügt werden.

Es soll der Betrieb der Einrichtung gemäß Figur 1 und in diesem Zusammenhang ein Verfahren zum Analysieren eines Stoffs 5 beispielhaft näher für den Fall beschrieben werden, dass es sich bei dem zu analysierenden Stoff 5 um menschliches oder tierisches Gewebe handelt und im Rahmen der Analyse des Stoffs eine Glukose- bzw. Blutzuckerspiegelangabe ermittelt werden soll.

Nacheinander oder gleichzeitig werden mit der Laser-Lichtquelle 3 eine oder mehrere Anregungsstrahlen 8, bei denen es sich vorzugsweise um Infrarotstrahlen handelt, erzeugt. Die Wellenlänge des oder der Infrarotstrahlen liegt vorzugsweise in einem Bereich zwischen 3 µm und 20 µm, besonders bevorzugt in einem Bereich zwischen 8 µm und 11 µm.

Die Anregungsstrahlen 8 werden mit der Einrichtung 9 zur Intensitätsmodulierung intensitäts- bzw. amplitudenmoduliert. In einer Ausführungsform werden mit der Einrichtung 9 zur Intensitätsmodulierung kurze Lichtpulse erzeugt, vorzugsweise mit einer Pulsfrequenz zwischen 1 kHz und 1 MHz oder Pulspakete (Doppel- oder Mehrfachmodulation), vorzugsweise mit einer Frequenz der Einhüllenden zwischen 1 kHz und 10 kHz.

Die modulierten Anregungsstrahlen 8 werden in das optische Medium/den Messkörper 1, insbesondere unmittelbar in das Material des Messkörpers, eingekoppelt und gelangen nach Passieren der Messfläche 2 in das Volumen 5a innerhalb des Gewebes 5.

Ein Passieren des Messkörpers 1 oder das Eintreten in das Material, aus dem der Messkörper besteht, ist für das Funktionieren der Erfindung möglich, jedoch nicht notwendig, solange sichergestellt ist, dass der Anregungsstrahl 8 an der Unterseite der Messfläche 2 in den zu analysierenden Stoff 5 gelangt. Dies wird dadurch verdeutlicht, dass eine Ausnehmung/Aussparung 13 in der Figur 1 als potenzielle Ausnehmung dargestellt ist, die einen engen Kanal, beispielsweise eine Bohrung, bildet, die an der Unterseite des Messkörpers 1 in der Messfläche 2 mündet und durch die der Anregungsstrahl 8 zur Unterseite der Messfläche 2 und in den Stoff 5 gelangen kann. In diesem weiten Sinne ist das oben genannte Merkmal zu verstehen, dass der Ausgangsstrahl "die Messfläche 2 durchsetzend" auf den Stoff 5 gerichtet ist. Im Normalfall kann ein Aufbau ohne eine solche Ausnehmung 13 vorgesehen sein, sofern der Anregungsstrahl 8 durch das Material des Messkörpers 1 ohne weiteres hindurchgelangen kann. Eine solche Ausnehmung 13 kann auch den Messkörper nur teilweise durchdringen. Anstelle einer Ausnehmung 13 kann in demselben Bereich auch ein integrierter Lichtwellenleiter vorgesehen sein, in den der Anregungsstrahl eingekoppelt werden kann. Dieser kann in oder an der Messfläche oder einer Sensorschicht enden. Der Lichtwellenleiter kann mit üblichen Herstellungsverfahren in ein Substrat des Messkörpers integriert sein. Besteht beispielsweise der Messkörper ganz oder teilweise aus Silizium, so kann ein Lichtwellenleiter als SOI- Lichtwellenleiter (silizium on insulator) integriert sein. Ein solcher Lichtwellenleiter kann gerade oder auch gekrümmt verlaufen. Damit kann die Position der Anregungsstrahlquelle, insbesondere einer Lasereinrichtung, die den Anregungsstrahl erzeugt, relativ zum Messkörper frei gestaltet werden.

Die Wellenlänge der Anregungsstrahlen 8 ist - mit Blick auf die hier beispielhaft erläuterte Blutzuckermessung - vorzugsweise derart gewählt, dass die Anregungsstrahlen 8 von Glukose bzw. Blutzucker signifikant absorbiert werden. Zur Messung von Glukose bzw. Blutzucker sind folgende glucoserelevante Infrarotwellenlängen besonders gut geeignet (Vakuumwellenlängen)und können einzeln oder gruppenweise gleichzeitig oder nacheinander als feste Wellenlängen für eine Messung der Reaktionssignale eingestellt werden: 8,1 µm, 8,3 µm, 8,5 µm, 8,8 µm, 9,2 µm, 9,4 µm und 9,7 µm. Zudem können glucosetolerante Wellenlängen verwendet werden, die von Glucose nicht absorbiert werden, um andere vorhanden Stoffe zu ermitteln und deren Einfluss auf die Messung auszuschließen.

Für eine Messung kann ein Spektralbereich kontinuierlich durch Scannen der Anregungsstrahlquelle, insbesondere einer Lasereinrichtung 3 durchfahren werden, oder das Spektrum kann diskontinuierlich an Stützstellen durch bestimmte geeignete feste Wellenlängen abgedeckt werden.

Sollen andere Stoffe als Glucose nachgewiesen werden, so sind entsprechende Wellenlängen für die Anregungsstrahlen zu wählen, die als Absorptionswellenlängen für diese Stoffe charakteristisch sind.

Durch die Absorption der Anregungsstrahlen 8 im Gewebe 5 wird im Bereich des Volumens 5a eine lokale Temperaturerhöhung hervorgerufen, die einen Wärmetransport und damit einhergehend Druckwellen und Wärmeimpulse in Richtung auf die Oberfläche des Gewebes 5 und die mit dieser im Kontakt stehende Messfläche 2 auslöst. Durch die dadurch an der Messfläche 2 und benachbart zu diesem im Messkörper 1 auftretenden Temperatur- und Druckschwankungen werden die Dichte, die Brechzahl bzw. die Deformation, Mikrostruktur und das Reflexionsverhalten im Detektionsbereich 4 in der Nähe der Messfläche 2 moduliert und damit einhergehend wird als Reaktionssignal ein elektrischer Widerstand im Falle eines Piezomaterials beeinflusst oder eine Piezo-Spannung erzeugt oder geändert/moduliert.

Das Maß /die Amplitude der Intensitätsmodulation der Messwerte/des Reaktionssignals hängt von der Wellenlänge der Anregungsstrahlen (wegen der nötigen Absorption im Gewebe) und von der Pulsfrequenz/Modulationsfrequenz der Anregungsstrahlen (wegen des Wärmetransports und der Druckwellen vom Gewebeinneren in Richtung der Messfläche 2) und den thermischen Eigenschaften der Probe und des Messkörpers 1 ab.

Die Messung kann für mehrere verschiedene Modulationsfrequenzen durchgeführt werden und die Messergebnisse, beispielsweise in Form von Spektren können miteinander verknüpft werden. Die einzelnen Spektren stellen das Reaktionssignal, beispielsweise eine Piezospannung oder die Amplitude einer veränderlichen Piezospannung in Abhängigkeit von der Wellenlänge des Anregungsstrahls dar. Verschiedene Spektren können derartig miteinander verknüpft werden, dass Messwerte von der Oberfläche der Probe (des Stoffes 5) herausgerechnet/eliminiert werden können oder dass eine spezifische Information aus einem bestimmten Tiefenbereich gewonnen werden kann.

Jedes der Spektren zu einer bestimmten Modulationsfrequenz entsteht aus der Überlagerung von Antwortsignalen aus dem zu analysierenden Stoff 5 aus verschiedenen Tiefen, da der Anregungsstrahl 8 bei seinem Weg in die Probe jeweils teilweise in verschiedenen Tiefenschichten absorbiert wird.

Das Reaktionssignal stellt somit jeweils eine Mischung aus Signalen aus unterschiedlichen Tiefen dar.

Das Mischungsverhältnis der Signale aus verschiedenen Tiefen hängt von der Frequenz der Modulation des Anregungsstrahls 8 ab.

Durch Verknüpfung verschiedener Spektren zu unterschiedlichen Modulationsfrequenzen, beispielsweise eine Differenzbildung von Spektren zu höheren Modulationsfrequenzen von Spektren zu niedrigeren Modulationsfrequenzen oder eine Quotientenbildung von Spektren zu höheren Modulationsfrequenzen und Spektren zu niedrigeren Modulationsfrequenzen, jeweils auch mit unterschiedlicher Gewichtung der einzelnen Spektren, können Einflüsse von oberen Stoffschichten wenigstens reduziert oder eliminiert werden.

Die Einrichtung kann anhand von Vergleichen mit vorab durchgeführten Kalibrations- bzw. Vergleichsmessungen oder Referenzdatensätzen, die in einer Ausführungsform in Form von Vergleichstabellen oder Vergleichskurven in einem Speicher der Auswerteeinrichtung 16 abgespeichert sind, auf die jeweils aktuelle Konzentration von Glukose bzw. Blutzucker innerhalb des Gewebes bzw. innerhalb des Volumens 5a schließen und eine entsprechende Glukose- bzw. Blutzuckerspiegelangabe erzeugen. Die Vergleichstabellen oder Vergleichskurven können beispielsweise auf der Basis von Glukose- oder Blutzuckerwerten erstellt worden sein, die anhand von außerhalb des Patientenkörpers analysierten Blutproben ermittelt worden sind.

Die Anregungsstrahlquelle, die im gezeigten Ausführungsbeispiel durch eine Lasereinrichtung 3 zum Aussenden des oder der Anregungs-Lichtstrahlen 8 gebildet ist, kann als Array ausgebildet sein. Das Array weist wenigstens 5, vorteilhaft wenigstens 10, weiter vorteilhaft wenigstens 15 oder wenigstens 50 oder 100 einzeln ansteuerbare Emitter 100a für jeweils monochromatisches Licht von unterschiedlichen, festen Wellenlängen im Absorptionsspektrum eines zu analysierenden Stoffes auf. Die einzelnen Emitter können dabei Laseremitter sein, es können jedoch auch andersartige Emitter, beispielsweise geeignete Leuchtdioden oder andere Halbleiterbauelemente sein, die gezielt Strahlung in einem bestimmten Wellenlängenbereich abstrahlen.

Das Array erzeugt vorzugsweise Strahlen mit monochromatischem Licht mit einer oder mehreren, besonders bevorzugt allen der folgenden Wellenlängen (Vakuumwellenlängen): 8,1 µm, 8,3 µm, 8,5 µm, 8,8 µm, 9,2 µm, 9,4 µm und 9,7 µm sowie, falls gewünscht zusätzlich glucosetolerante Wellenlängen.

Es kann vorgesehen sein, dass die Anregungssendeeinrichtung/Anregungs-Lichtquelle 3 unmittelbar oder mittels einer Justagevorrichtung mit dem optischen Medium/Messkörper 1 mechanisch fest verbunden ist. Die Justagevorrichtung ermöglicht vorzugsweise eine Justage des Abstands der Anregungs-Lichtquelle 3 von dem Messkörper 1 bzw. eine Justage in Strahllängsrichtung und/oder eine Justage in der Ebene senkrecht dazu.

Es kann auch vorgesehen sein, dass die Anregungssendeeinrichtung 3 und der Messkörper 1 mit der Detektionsvorrichtung 4, 6 unmittelbar aneinander oder an einem gemeinsamen Träger (nicht gezeigt) befestigt sind. Der Träger kann durch ein Kunststoffteil, eine Leiterplatte oder ein Metallblech gebildet sein, das in einem Gehäuse montiert ist.

Der Träger kann auch durch das Gehäuse selbst oder ein Gehäuseteil gebildet sein.

Es kann auch vorgesehen sein, dass die Einrichtung zur Analyse eines Stoffes mit einem Gehäuse (nicht gezeigt), in dem sie angeordnet ist, am Körper, beispielsweise am Rumpf einer Person befestigbar ist, wobei die Anregungssendeeinrichtung 3 zum Aussenden eines oder mehrerer Anregungs-Lichtstrahlen 8 und die Detektionsvorrichtung 4, 6 zur Detektion des zeitabhängigen Reaktionssignals derart angeordnet und eingerichtet sind, dass die zur Messung geeignete Seite und die Messfläche 2 der Vorrichtung auf der dem Körper/Rumpf abgewandten Seite der Vorrichtung liegt, so dass der zu analysierende Stoff auf der dem Körper/Rumpf abgewandten Seite des Gehäuses vermessbar ist, indem beispielsweise der Patient einen Finger auf die Messfläche 2 legt. Hierzu ist beispielsweise vorgesehen, dass das Gehäuse mittels eines zum Gehäuse gehörenden Riemens am Körper einer Person, in einer Ausführungsform in Form eines Armbandes an einem Handgelenk befestigt ist. Auf der dem Handgelenk abgewandten Seite weist dann das Gehäuse ein für den Anregungs-Lichtstrahl 8 durchlässiges Fenster auf, oder der Messkörper 1 ist mit seiner nach außen weisenden Messfläche 2 unmittelbar in die nach außen vom Körper weg weisende Seite des Gehäuses eingepasst und bildet beispielsweise selbst mit der Messfläche 2 abschnittsweise die Oberfläche des Gehäuses.

Es kann bei dieser Konzeption dann eine Fingerbeere auf den Messkörper 1 aufgelegt und vermessen werden.

Der Messkörper 1 kann innerhalb des Gehäuses, ebenso wie der Träger, oder unmittelbar an dem Gehäuse befestigt sein. Der Messkörper 1 kann auch unmittelbar mit dem Träger verbunden sein, wobei eine Justageeinrichtung zur relativen Positionierung des Trägers zum optischen Medium/Messkörper 1 vorgesehen sein sollte.

Es ist auch denkbar, die Anregungs-Lichtquelle 3 direkt an dem Messkörper zu befestigen.

Durch das optische Fenster in dem Gehäuse und/oder durch den Messkörper 1 hindurch können auch andere Parameter der Stoffoberfläche bzw. der aufgelegten Fingerbeere vermessbar sein, wie in einer Ausführungsform ein Fingerabdruck. Hierzu kann in dem Gehäuse ein optischer Detektor in Form einer Kamera beispielsweise auch an dem Träger befestigt sein, die durch den Messkörper hindurch oder an diesem vorbei neben dem Messkörper ein Bild der Oberfläche des Stoffs 5 digital aufnimmt. Dieses Bild wird innerhalb einer Verarbeitungseinrichtung, die unmittelbar mit der Detektionsvorrichtung 4, 6 und auch mit der Anregungssendeeinrichtung 3 verbunden sein kann ebenso verarbeitet wie die Messinformationen von der Detektionsvorrichtung 4, 6. Die Verarbeitungseinrichtung kann auch Steuerungsaufgaben der Messung übernehmen. Sie kann auch wenigstens teilweise von den übrigen Teilen der Vorrichtung getrennt und entfernt sein und mittels einer Funkverbindung mit diesen kommunizieren.

Die Bilddaten von der Kamera können somit innerhalb des Gehäuses oder über eine Funkverbindung auch außerhalb des Gehäuses weiterverarbeitet und mit einer Personenidentitäts-Datenbank verglichen werden, um Kalibrationsdaten der identifizierten Person abzurufen und diese der Messung zugrunde zu legen.

Derartige Kalibrationsdaten können auch entfernt (remote) in einer Datenbank, in einer Ausführungsform einer Cloud, abrufbar gespeichert sein. Auch die Messdaten der Detektionsvorrichtung können sowohl innerhalb als auch außerhalb des Gehäuses weiterverarbeitet werden.

Werden Daten außerhalb des Gehäuses verarbeitet, so sollten die Ergebnisdaten vorzugsweise zu der Vorrichtung innerhalb des Gehäuses zurückgefunkt werden, um dort angezeigt werden zu können.

In jedem Fall kann eine Anzeige (nicht gezeigt) an dem Gehäuse vorgesehen sein, die vorteilhaft durch das optische Fenster, in einer Ausführungsform auch teilweise durch den Messkörper oder am Messkörper 1 ablesbar ist. Die Anzeige kann auch durch das optische Fenster eine Leuchtanzeige auf eine Anzeigefläche projizieren und zu diesem Zweck eine Projektionsvorrichtung aufweisen. Durch die Anzeige kann in einer Ausführungsform ein Mess- oder Analyseergebnis, insbesondere eine Glucosekonzentration angezeigt werden. Die Ausgabe kann in einer Ausführungsform über einen Zeichen- oder Farbcode erfolgen. Es kann durch die Anzeige oder eine zu dieser parallelen Signaleinrichtung in einer Ausführungsform ein Vorschlag für eine Insulindosis abhängig von weiteren Patientenparametern (z.B. Insulinkorrekturfaktor) oder eine automatische Signalübertragung an eine Dosiereinrichtung in Form einer Insulinpumpe erfolgen.

Alternativ kann auch eine Empfehlung zum Verzehr bestimmter Lebensmittel mit einer Mengenangabe erfolgen. Dies kann beispielsweise mit einem Zubereitungsvorschlag verbunden sein, der aus einer Datenbank abgerufen und insbesondere auch in elektronische Form übermittelt werden kann. Diese Zubereitungsanweisung kann auch an eine automatische Vorrichtung zur Nahrungszubereitung übermittelt werden.

Die Verbindung der Vorrichtung von und zu einer externen Datenverarbeitungseinrichtung kann durch alle gängigen Standards, wie Lichtleiter, Kabel, Funk (z. B. Bluetooth, WiFi), oder auch Ultraschall oder Infrarotsignale realisiert sein.

Die Figuren 2 bis 16 zeigen unter anderem besondere Ausgestaltungen der Kontakteinrichtung 6 mit in vielfältiger Weise verteilten Elektroden.

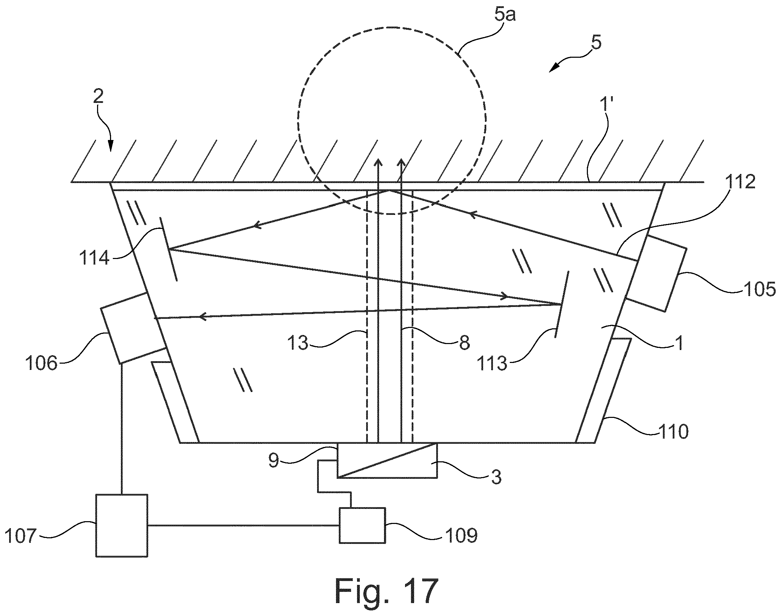

Die Figur 17 zeigt eine Ausgestaltung der Erfindung mit einer Detektionseinrichtung, die die Reflexion eines Messlichtstrahls erfasst.

In der Figur 2 ist wieder schematisch ein Messkörper 1 mit einer Messfläche 2 und einem Detektionsbereich 4 sowie einer Kontakteinrichtung 6 dargestellt. Aus der Figur 2 geht hervor, dass die Kontakteinrichtung 6 auch mehrere Elektroden auf jeder Seite des Detektionsbereichs 4 aufweisen kann. In dem dargestellten Beispiel sind auf einer ersten Seite des Detektionsbereichs 4 die Elektroden 6e, 6f und auf der gegenüberliegenden Seite des Detektionsbereichs 4 die Elektroden 6g, 6h dargestellt. Die einzelnen Elektroden sind in dem gezeigten Beispiel rechteckig plattenförmig ausgebildet. Für eine Messung kann durch die Auswerteeinrichtung 16 jeweils ein Piezosignal (in Form einer Piezospannung oder eines geänderten Widerstands) zwischen zwei Elektroden 6e, 6f, 6g, 6h ausgewertet und die Ergebnisse können verglichen werden. Es kann dann für die weitere Auswertung der Messung oder für eine nachfolgende Messung ein Elektrodenpaar ausgewählt werden. Dabei kann berücksichtigt werden, welches Elektrodenpaar die größten Signale oder die am wenigsten störanfälligen Signale liefert. Es kann für die Messung vorzugsweise ein Paar benachbarter Elektroden ausgewählt werden, also beispielsweise die Elektroden 6f und 6g oder auch die Elektroden 6e und 6f oder 6g und 6h. Es ist jedoch auch möglich, das Elektrodenpaar 6e, 6h für eine Messung zu verwenden und die zwischen diesen Elektroden liegenden Signale auszuwerten. Dabei ist zu berücksichtigen, dass die Elektroden, die für eine aktuelle Messung gerade nicht verwendet werden, andere Elektroden dielektrisch abschirmen können. Die nicht verwendeten Elektroden können beispielsweise auf einem floatenden Potential belassen oder in bestimmten Fällen mit Erdpotenzial verbunden werden.

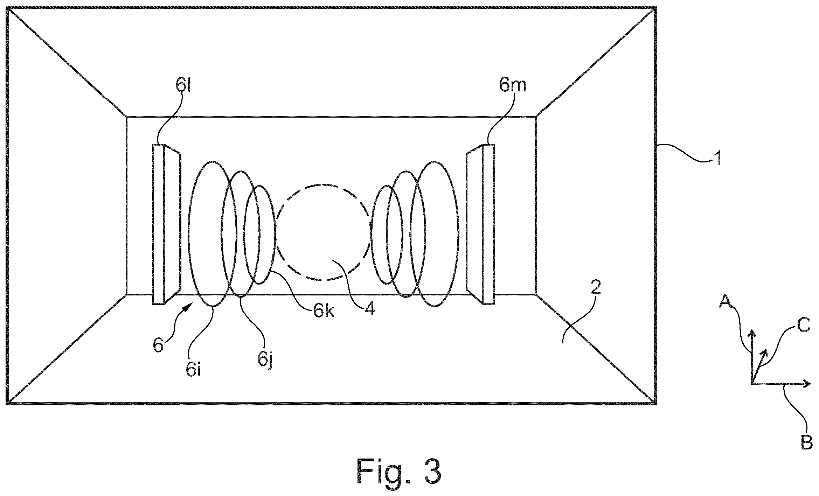

Aus der Figur 3 geht eine Anordnung einer Kontakteinrichtung 6 hervor, die auf jeder Seite des Detektionsbereichs 4 jeweils drei kreisringförmige Elektroden 6i, 6j, 6k aufweist. Auch diese Elektroden können paarweise zur Messung verwendet werden. Der Durchmesser der ringförmigen Elektroden nimmt in dem dargestellten Fall vom Detektionsbereich 4 ausgehend nach außen hin zu, so dass die Elektrode 6i einen größeren Durchmesser aufweist als die Elektrode 6j und diese einen größeren Durchmesser als die Elektrode 6k.

Zusätzlich kann außen hinter den Elektroden 6i, 6j, 6k beispielsweise ein Paar von planen, plattenförmigen Elektroden 6l, 6m vorgesehen sein.

Die ringförmige Ausgestaltung der Elektroden kann zusammen mit der nach außen zunehmenden Größe dazu führen, dass die äußeren Elektroden von dem innen liegenden Elektroden weniger abgeschirmt werden und verschiedene Elektrodenpaare unabhängig voneinander verwendet werden können.

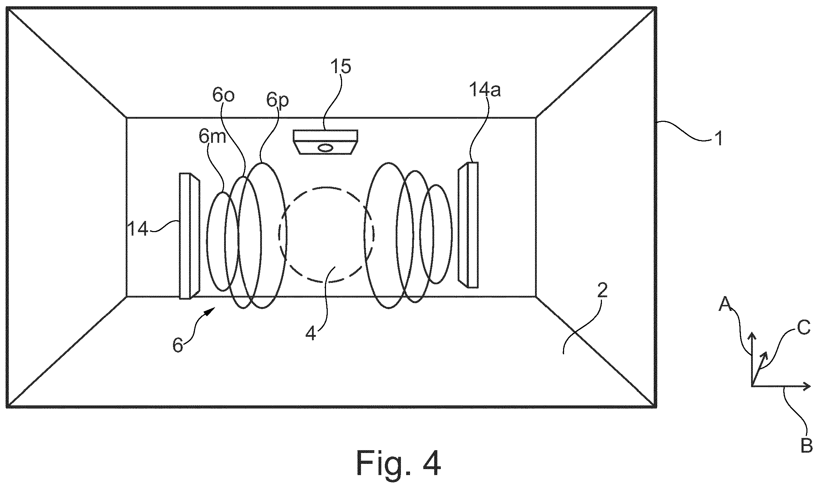

Die Figur 4 zeigt eine Ausgestaltung mit einer Kontakteinrichtung 6 mit jeweils drei auf jeder Seite des Detektionsbereichs in horizontaler Richtung - also der Richtung B - parallel zur Messfläche 2 hintereinanderliegenden ringförmigen Elektroden 6n, 6o, 6p, wobei der Durchmesser der ringförmigen Elektroden mit zunehmendem Abstand von dem Detektionsbereich 4 abnimmt.

Seitlich außerhalb der Elektroden ist auf jeder Seite des Detektionsbereiches eine Wärmesenke 14, 14a dargestellt, die in den Messkörper eingelassen oder außen auf ihn aufgebracht sein kann. Diese Wärmesenken können beispielsweise aus einem Metall oder einem anderen Material bestehen, dessen Wärmekapazität und/oder Wärmeleitfähigkeit größer ist als die des Materials/der Materialien, aus dem/denen der Messkörper 1 besteht.

Es kann auch ein einzelner, beispielsweise ringförmiger Körper als Wärmesenke vorgesehen sein, der den Detektionsbereich 4 oder den gesamten Messkörper 1 umgibt. Eine Wäremesenke kann dabei auch durch ein Peltierelement verwirklicht sein. Die Wärmesenke sorgt dafür, dass die Temperaturerhöhung, die durch die Ankunft einer Wärmewelle/eines Wärmeimpulses im Detektionsbereich erfolgt, durch Abkühlung möglichst schnell wieder ausgeglichen werden kann, so dass das Material des Messkörpers 1 im Detektionsbereich 4 möglichst schnell auf einen nachfolgenden Wärmeimpuls reagieren kann.

Die Wärmeimpulse folgen aufeinander mit der Modulationsfrequenz des Anregungsstrahls.

Oberhalb des Detektionsbereichs ist eine plattenförmige Wärmebarriere 15 vorgesehen, die eine Öffnung zum Durchtritt eines Anregungsstrahls 8 aufweist. Diese sorgt dafür, dass der Wärmeimpuls, wenn er im Detektionsbereich ankommt, bei einer zu großen Wärmeleitfähigkeit des Materials des Messkörpers 1 nicht zu schnell abgeführt wird, so dass sich kurzzeitig eine Temperaturerhöhung im Detektionsbereich 4 aufbauen kann, bevor die Wärme, beispielsweise über Wärmesenken, abgeführt wird.

Es können eine oder mehrere Wärmesenken und/oder eine oder mehrere Wärmebarrieren in einen Messkörper integriert oder außen auf diesem angebracht sein, um den Wärmetransport geeignet zu lenken. Dies kann insbesondere für flache Messkörper sinnvoll sein oder für Messkörper, die eine dünne Beschichtung aus einem Piezo- Material aufweisen und ansonsten aus einem Material bestehen, das keinen Piezo-Effekt aufweist.

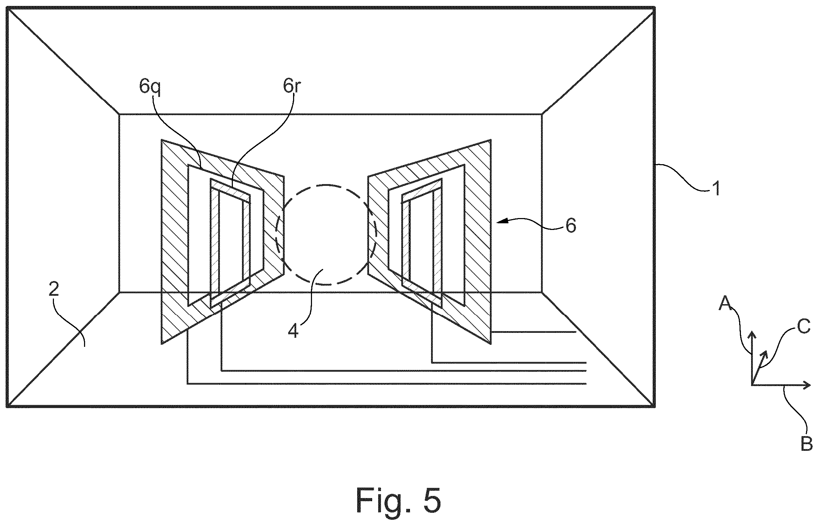

Die Figur 5 zeigt eine Kontakteinrichtung 6 mit jeweils zwei rahmenförmigen Elektroden 6q, 6r auf jeder Seite des Detektionsbereichs 4, wobei die rahmenförmigen Elektroden als rechteckige Rahmen ausgebildet sind und in gleicher Entfernung vom Detektionsbereich 4 oder auch in unterschiedlichen Entfernungen vom Detektionsbereich 4 angeordnet sein können. Die Elektroden 6q, 6r können somit auf jede Seite des Detektionsbereichs 4 beispielsweise koplanar und konzentrisch zueinander angeordnet oder bezüglich der Entfernung vom Detektionsbereich 4 gestaffelt sein.

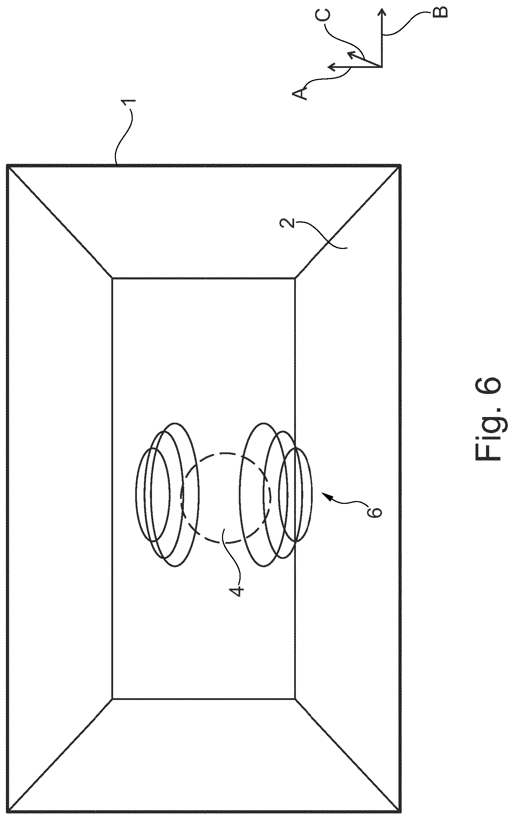

Die Figur 6 zeigt eine Kontakteinrichtung 6 mit jeweils drei ringförmigen Elektroden oberhalb und unterhalb des Detektionsbereichs 4, d. h. die Elektroden sind bezüglich der Flächennormalen in Richtung A auf beiden Seiten des Detektionsbereichs 4 hintereinander in verschiedenen Entfernungen von der Messfläche 2 angeordnet.

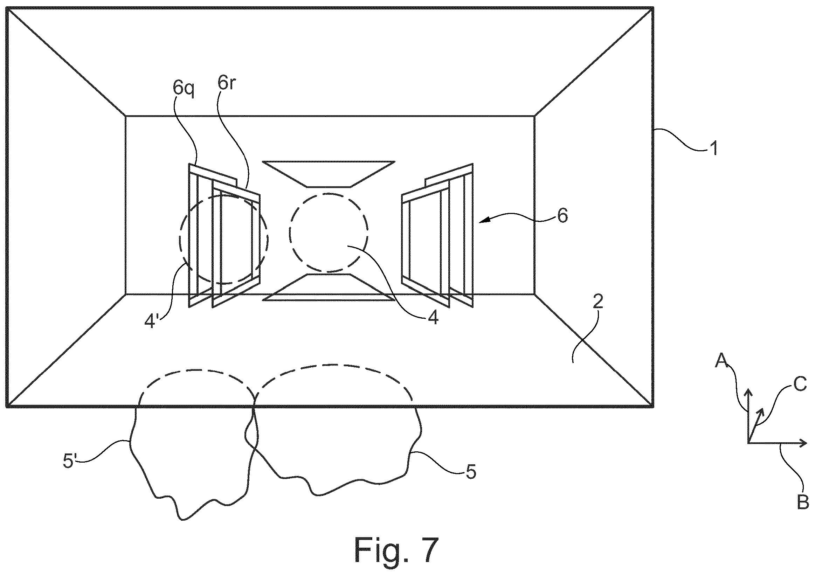

Die Figur 7 zeigt ein Beispiel für die Verwendung von Elektroden einer Kontakteinrichtung 6. Es sind auf jeder Seite des Detektionsbereichs 4 jeweils zwei rahmenförmige Elektroden 6q, 6r in unterschiedlichem Abstand vom Detektionsbereich 4 vorgesehen. Im Normalfall, wenn der zu analysierende Stoff in der Mitte unter der Messfläche 2 angeordnet ist, wie in der Figur 7 durch den Bereich 5 des Stoffes angedeutet ist, kann die Auswahl von zwei Elektroden beiderseits des Detektionsbereichs 4 für eine Messung optimal sein. Wird jedoch eine Probe auf die Messfläche 2 an der falschen Stelle aufgelegt, beispielsweise indem ein Finger eines Probanden seitlich gegenüber der optimalen Position verschoben ist, so wird der zu analysierende Stoff sich in Richtung der dargestellten Position 5' bewegen und ein optimaler Detektionsbereich wird im Bereich des Messkörpers 1 liegen, der mit 4' bezeichnet ist. Für diesen Fall kann es sinnvoll sein, zwei andere Elektroden, im dargestellten Fall die Elektroden 6q, 6r, für eine Messung auszuwählen und die zwischen diesen entstehenden elektrischen Signale auszuwerten. Es können in diesem Fall auch probeweise verschiedene Paare von Elektroden benutzt, die entsprechenden Signale ausgewertet und miteinander verglichen werden, um zu ermitteln, an welcher Stelle genau der zu analysierende Stoff 5 an der Messfläche 2 anliegt und welches Elektrodenpaar die besten Messergebnisse erzeugt.

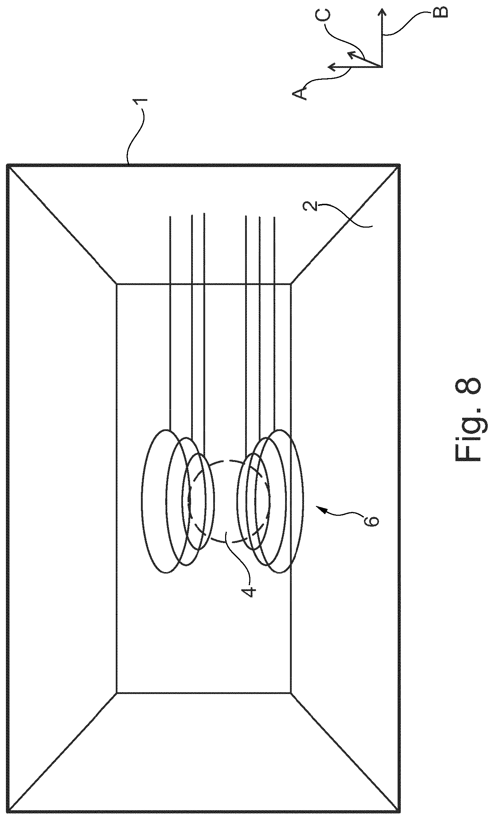

In der Figur 8 sind sechs ringförmige Elektroden in Richtung der Flächennormalen A hintereinanderliegend auf beiden Seiten des Detektionsbereichs 4 dargestellt. Auf jeder Seite des Detektionsbereichs liegen jeweils drei kreisringförmige Elektroden, die koaxial zueinander angeordnet sind, wobei der Durchmesser der einzelnen Elektroden mit zunehmendem Abstand vom Detektionsbereich 4 zunimmt. Auch hier können verschiedene Elektrodenpaare, die symmetrisch oder unsymmetrisch zu dem Detektionsbereich 4 einander gegenüberliegen, für eine Messung ausgewählt werden. Die Auswerteeinrichtung ist in dieser Darstellung, wie auch in einigen anderen der Figuren, der Übersichtlichkeit halber weggelassen; ebenso wie eine Darstellung des Anregungsstrahls und der Lasereinrichtung.

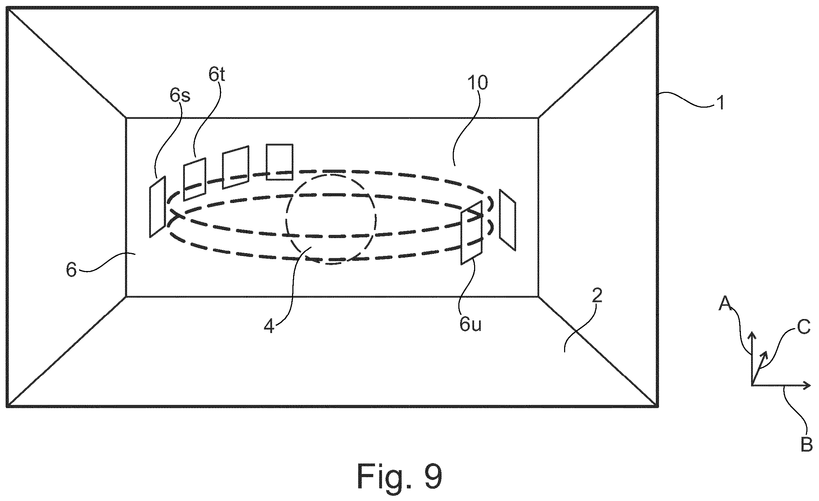

In der Figur 9 ist ein Messkörper 1 mit einem Detektionsbereich 4 dargestellt, wobei die Kontakteinrichtung 6 eine Mehrzahl von Elektroden 6s, 6t, 6u aufweist, die in einem kreisringförmigen Bereich 10 um den Detektionsbereich 4 herum verteilt sind. Bei dieser Verteilung der Elektroden 6s, 6t, 6u ist es sinnvoll, Signale jeweils zwischen zwei diametral einander gegenüberliegenden Elektroden 6s, 6u für eine Messung auszuwählen, wobei verschiedene Elektrodenpaare für eine Selektion versuchsweise betrieben werden können.

In diesem Zusammenhang soll bemerkt werden, dass der Detektionsbereich 4 in den Figuren als ein Bereich des Messkörpers gekennzeichnet ist, der der geforderten Materialwahl entspricht, so dass er einen Piezoeffekt aufweist und der gleichzeitig in einem Bereich des Messkörpers 1 liegt, in dem ein Reaktionssignal von dem zu analysierenden Stoff 5 in Form eines Wärmeimpulses ankommt. Ein möglicher Detektionsbereich 4 hängt auch von den für die Messung ausgewählten Elektroden 6 ab und liegt üblicherweise zwischen den für die Messung ausgewählten Elektroden 6, sobald der Messkörper 1 in diesem Bereich 4 aus dem geforderten Material besteht bzw. einen Piezoeffekt aufweist. Der Detektionsbereich 4 ist somit nicht notwendig in dem Messkörper vorgegeben, sondern ergibt sich als der Bereich, in dem die Reaktionssignale aus dem zu analysierenden Stoff mittels des verwendeten physikalischen Effekts durch die ausgewählten Kontaktelektroden 6 bei geeigneter Positionierung des Stoffs 5 unter der Messfläche 2 nachweisbar sind.

Die in der Figur 9 gezeigten Elektroden 6s, 6t, 6u können rechteckig oder rund oder oval und dabei eben oder teilzylinderförmig, d. h. in einer Achse gekrümmt, ausgebildet sein.

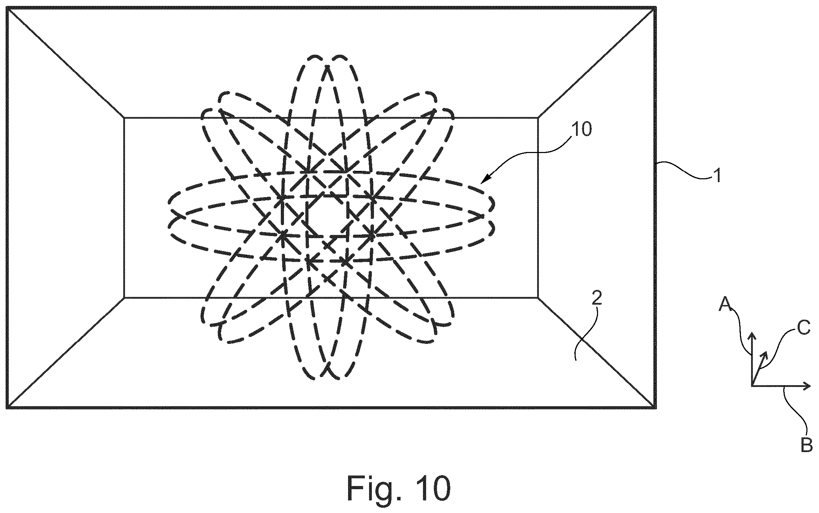

Die Figur 10 zeigt in einem Beispiel, dass der kreisringförmige Bereich 10, in dem die Elektroden um einen Detektionsbereich herum angeordnet sind, nicht in Form eines zu der Messfläche parallelen Kreisrings angeordnet sein muss, sondern als Kreisring im Raum des Messkörpers 1 in verschiedenen Winkellagen vorgesehen sein kann.

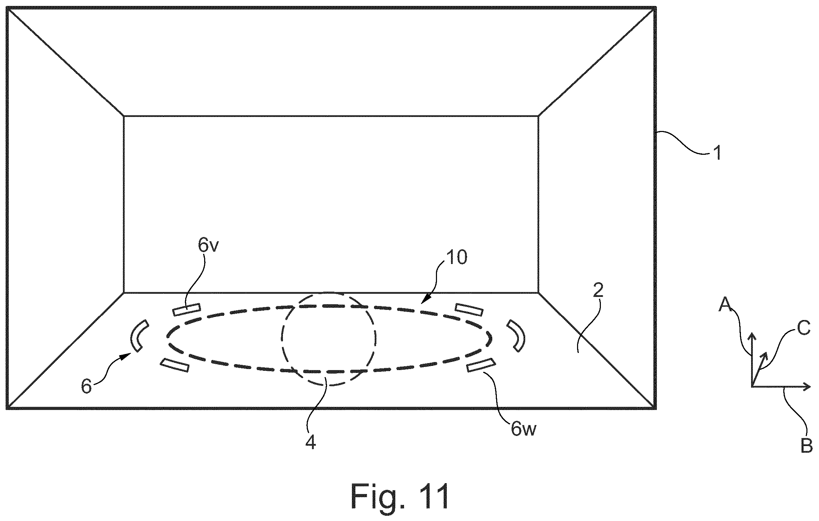

In der Figur 11 ist eine Anordnung gezeigt, bei der die Kontakteinrichtung 6 unmittelbar im Bereich der Messfläche 2 in dem Messkörper 1 oder an dem Messkörper 1 vorgesehen ist. Einzelne Elektroden 6v, 6w sind unmittelbar an der Messfläche 2 in einem kreisringförmigen Bereich 10 um den Detektionsbereich 4 herum verteilt angeordnet.

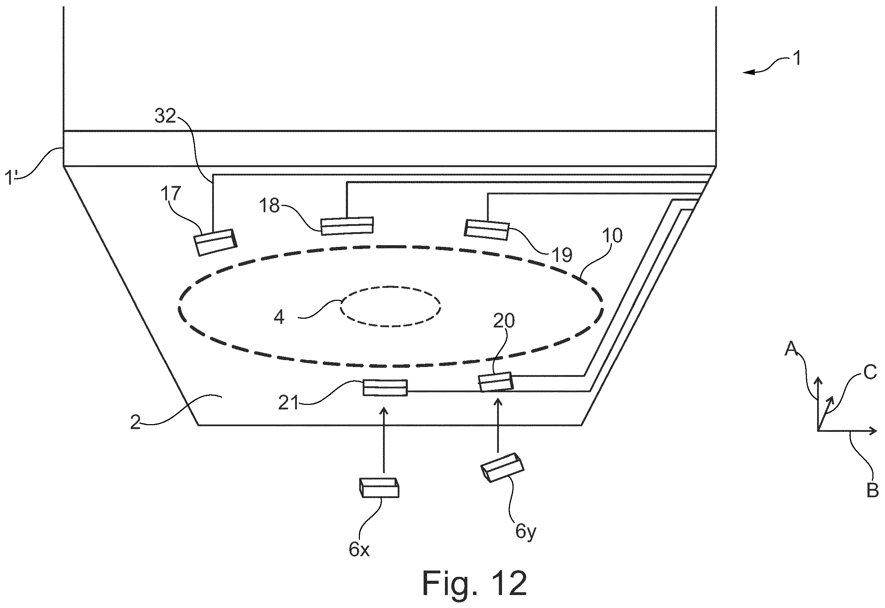

Die Figur 12 zeigt dabei eine spezielle Ausführungsform der Struktur, die in der Figur 11 angedeutet ist. In der Figur 12 ist der Messkörper 1 von der Unterseite, d. h. von außen auf die Messfläche 2 blickend, dargestellt, wobei um den kreisringförmigen Bereich 10 herum Ausnehmungen zur Aufnahme von Elektroden in einer unteren Schicht 1' des Messkörpers 1 vorgesehen sind. Die Ausnehmungen sind mit 17, 18, 19, 20, 21 bezeichnet. In die Ausnehmungen sind Elektrodenkörper 6x, 6y einlegbar und können dort kraftschlüssig oder stoffschlüssig gehalten werden. Die Elektroden 6x, 6y können beispielsweise in den Ausnehmungen 21, 20 eingeklinkt werden. Dabei weisen die Ausnehmungen 17, 18, 19, 20, 21 beispielsweise Kontaktierungen auf, die mit Leiterbahnen 32 auf der Messfläche 2 verbunden sind. Die Leiterbahnen 32 sind elektrisch mit einer nicht dargestellten Auswerteeinrichtung verbunden. Beim Einsetzen der Elektroden 6x, 6y in die Ausnehmungen werden diese mit den Leiterbahnen 32 und somit mit einer Auswerteeinrichtung elektrisch verbunden.

Die Elektroden können für eine Messung ebenso wie bereits oben beschrieben ausgewählt werden.

Dabei kann beispielsweise vorgesehen sein, dass die Schicht 1' des Messkörpers 1 aus einem piezoelektrischen Material besteht, während der Rest des Messkörpers 1 aus einem anderen, entweder ebenfalls piezoelektrisch sensitiven oder nicht piezoelektrischen Material besteht.

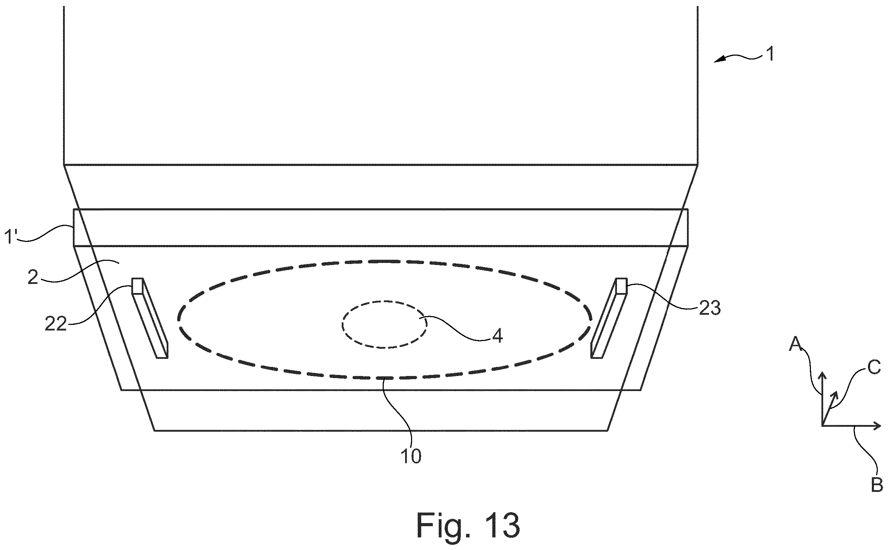

In der Figur 13 ist eine Ausführungsvariante dargestellt, in der die Schicht 1' des Messkörpers 1 Elektroden 22, 23 trägt, die außen auf die Schicht 1' (Sensor- Schicht) aufgebracht, beispielsweise aufgeklebt oder aufgedampft, sind. Die Schichtdicke der Sensor- Schicht 1', welche aus piezoelektrischem Material besteht, kann zwischen 0 und 1 mm, insbesondere zwischen 0 und 500 Mikrometer, insbesondere kleiner als 100 Mikrometer sein. Die Schicht kann mit dem ersten Teil/übrigen Bereich des Messkörpers 1 verklebt sein oder in einer anderen Fügetechnik mit diesem zusammengefügt sein. Der Anregungsstrahl kann diese Schicht 1' passieren. Für den Anregungsstrahl kann im übrigen Bereich des Messkörpers 1 (im sogenannten ersten Teil des Messkörpers 1) eine kanalförmige Ausnehmung vorgesehen sein. Das Material dieses übrigen Bereichs des Messkörpers 1 kann dann aus einem für Infrarotstrahlung undurchlässigen Material, beispielsweise Quarz oder Saphir, bestehen. Diese Elektroden lassen die Messung eines Piezoeffekts im Detektionsbereich 4 innerhalb der Schicht 1' zu. Es können weitere Elektroden der gleichen Art unter der Messfläche 2 oder in Ausnehmungen in der Messfläche 2 in einem kreisringförmigen Bereich 10 um den Detektionsbereich 4 herum vorgesehen sein.

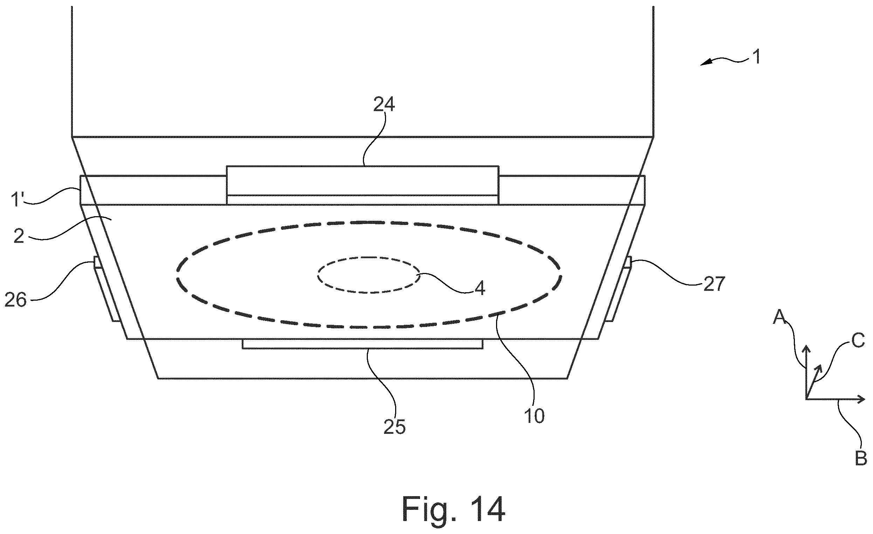

Die Figur 14 zeigt eine Ausführungsform mit einer piezoelektrischen Schicht 1' eines Messkörpers 1, bei der seitlich auf die Schicht 1' zwei Elektroden 24, 25 aufgesetzt sind. Auch diese Elektroden 24, 25 können beispielsweise aufgeklebt oder aufgedampft oder in anderer Form auf die Oberfläche der Schicht 1' aufgebracht oder in Ausnehmungen in der Schicht 1 eingebracht sein. Auch die übrigen Seitenflächen der Schicht 1' können Elektroden 26, 27 aufweisen, wobei alle Elektroden durch Lichtleitungen oder Leiterbahnen mit einer Auswerteeinrichtung verbunden sind.

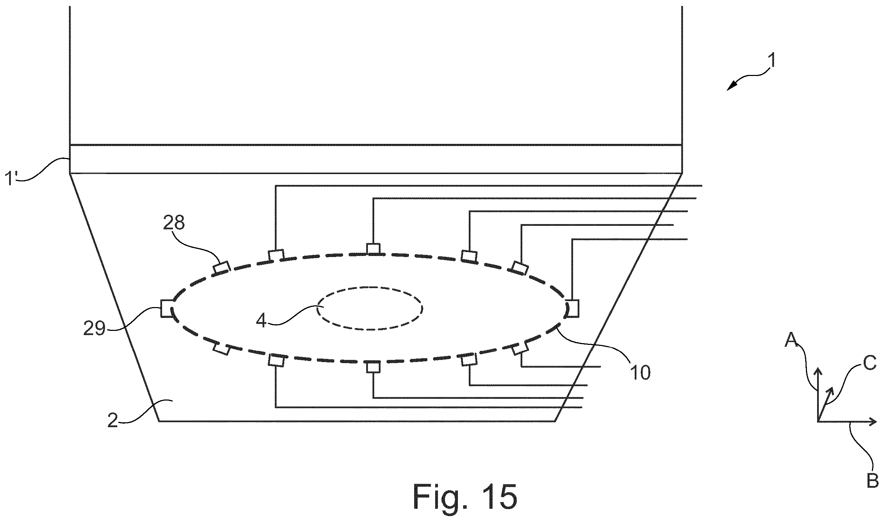

Die Figur 15 zeigt eine Mehrzahl von Elektroden 28, 29, die an der Unterseite einer piezoelektrischen Schicht 1' eines Messkörpers, d. h. an der Messfläche 2, in einem kreisringförmigen Bereich 10 um einen Detektionsbereich 4 angeordnet sind. Auch hier können wahlweise verschiedene Paare von Elektroden für eine Messung verwendet werden, wobei die beiden Elektroden eines Paares einander auf verschieden Seiten des Detektionsbereichs 4 gegenüberliegen sollen. Auch diese Elektroden können beispielsweise aufgeklebt oder aufgedampft oder in anderer Form auf die Oberfläche der Schicht 1'aufgebracht oder in Ausnehmungen/Aussparungen in der Schicht eingebracht sein. Versuchsweise können verschiedene Paare von Elektroden für eine Messung zusammengestellt und betrieben werden, um das optimale Elektrodenpaar je nach der Positionierung des Stoffs 5 unter der Messfläche 2, also je nach Lage des optimalen Detektionsbereichs 4 zu selektieren.

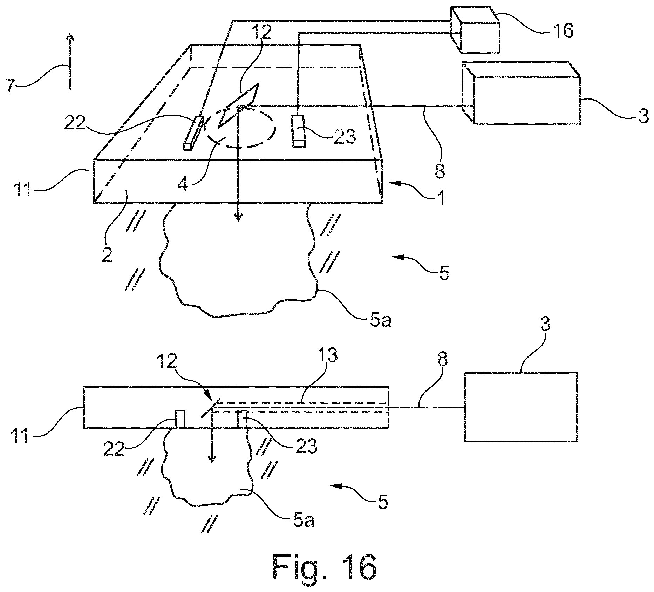

Die Figur 16 zeigt einen Messkörper 11, der als Flachkörper ausgebildet ist und beispielsweise ähnlich aufgebaut und mit Elektroden versehen sein kann, wie die in den Figuren 14, 15 dargestellten Schichten 1' des Messkörpers 1. Jedoch kann in diesem Fall der gesamte Messkörper 1 durch den Flachkörper 11 gebildet sein. An der Unterseite des Flachkörpers 11 ist die Messfläche 2 vorgesehen und die Ausdehnung des Flachkörpers 11 in Richtung der Flächennormalen 7 der Messfläche 2 ist klein gegenüber der Ausdehnung in den Richtungen parallel zur Messfläche 2. Um für den Aufbau der Einrichtung in Richtung der Flächennormalen 7 einen minimalen Platz zu beanspruchen, kann vorgesehen sein, die Lasereinrichtung 3 seitlich neben dem Flachkörper über einer gedachten Verlängerung der Messfläche an einer seiner Flachseiten anzuordnen und den Anregungsstrahl 8 parallel zur Messfläche 2 oder im Wesentlichen parallel zu dieser in den Messkörper/Flachkörper 11 einzustrahlen. Für den Anregungsstrahl 8 kann dann auch eine kanalförmige Ausnehmung in den Flachkörper eingebracht sein, ähnlich wie sie in Fig. 1 unter Bezugszeichen 13 gezeigt ist. Es ist dann beispielsweise eine Spiegeleinrichtung 12 innerhalb des Flachkörpers vorgesehen, die den Messstrahl 8 zur Messfläche 2 hin und weiter durch die Messfläche 2 in den Stoff 5 reflektiert. Auch der Flachkörper kann insgesamt aus einem piezoelektrischen Material bestehen oder im Bereich der Messfläche eine piezoelektrische Schicht aufweisen.

Alternativ kann auch vorgesehen sein, dass die Lasereinrichtung 3 etwas oberhalb des Flachkörpers 11 angeordnet ist, so dass der Anregungsstrahl 8 parallel zu dem Flachkörper zu einer oben auf den Flachkörper 11 angeordneten Spiegeleinrichtung 12 gestrahlt und dort in den Flachkörper 11 senkrecht zur Messfläche 2 eingespiegelt wird. In beiden Fällen ist die Ausdehnung der Einrichtung in Richtung der Flächennormalen 7 gegenüber der Ausführungsform, die in der Figur 1 dargestellt ist, drastisch reduziert. Der Messkörper 11 und die Lasereinrichtung 3 können damit zusammen in einem flachen Gehäuse untergebracht werden, ggf. gemeinsam mit der Auswerteeinrichtung 16.

Innerhalb, unterhalb des Flachkörpers 11 oder seitlich von diesem können die Elektroden 22, 23 vorgesehen sein, die für die Messungen am Detektionsbereich 4 verwendet werden. Es ist bei dieser Ausgestaltung jede der weiter oben bereits erläuterten Anordnungen von zwei oder mehr Elektroden ebenso verwendbar.

Auch ein solcher als Flachkörper 11 ausgebildeter Messkörper kann aus einem ersten Teil und einer mit diesem zusammengefügten/ auf diesen aufgeklebten Sensor- Schicht, beispielsweise piezoelektrischen Schicht gebildet sein, wobei dann die piezoelektrische Schicht die Messfläche bildet und mit Elektroden versehen ist. Es kann auch in diesem Fall eine Ausnehmung für den Anregungsstrahl in dem ersten Teil des Messkörpers 11 vorgesehen sein und dieser kann dann optional aus einem für Infrarotstrahlung undurchlässigen oder wenig durchlässigen Material bestehen, wie beispielsweise Quarz oder Saphir.

Im unteren Bereich der Figur 16 ist die oben perspektivisch dargestellte Anordnung in einer Seitenansicht gezeigt, um den Reflexionsweg des Anregungsstrahls 8 deutlich zu machen.

In dem Fall, dass ein oben beschriebener Flachkörper verwendet wird mit einer Lasereinrichtung, die seitlich neben diesem angeordnet und derart ausgerichtet ist, dass sie einen intensitätsmodulierten und bezüglich der Wellenlänge variierten Anregungsstrahl im wesentlichen parallel zur Messfläche in oder über den Flachkörper aussendet, wobei der Anregungsstrahl zur Messfläche umgelenkt wird, kann zur Detektion ein separat durch eine Strahlquelle erzeugter, in den Messkörper eingestrahlter und im Bereich der Messfläche reflektierter Messstrahl verwendet werden, dessen Ablenkung (Ablenkwinkel) im Messkörper im Bereich der Messfläche durch die Reaktionssignale aus dem zu analysierenden Stoff beeinflusst wird. Der Ablenkwinkel kann gemessen und daraus die Intensität der Reaktionssignale bestimmt werden, die der Absorptionsintensität des Anregungsstrahls in dem Stoff 5 und der Dichte/Konzentration einer absorbierenden/zu detektierenden Substanz in dem Stoff entspricht.

Auch für eine solche Anwendung kann der Flachkörper homogen aus einem Material aufgebaut sein, dessen Brechungsindex von der Temperatur abhängt oder er kann im Bereich der Messfläche eine Schicht aufweisen, die aus einem Material besteht, dessen Brechungsindex von der Temperatur abhängt.

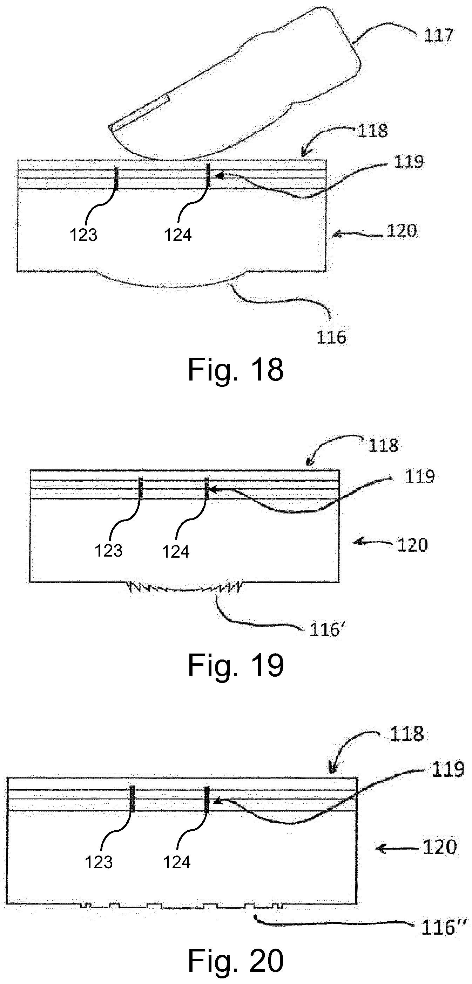

Die Figur 18 zeigt, ebenso wie die Figuren 19 bis 23, in einer Querschnittsdarstellung ein Substrat 120, in das eine erste Elektrodenanordnung 119 mit zwei schematisch eingezeichneten, zueinander parallelen Plattenelektroden 123, 124 als Teil einer piezoelektrischen Detektionseinrichtung eingebettet ist. Schraffuren von geschnittenen Partien sind der Übersichtlichkeit halber weggelassen. Die Messfläche 118 liegt jeweils in dem in den Figuren oberen Teil des Substrats 120, das ganz oder teilweise aus Silizium bestehen kann. Zur Anschauung ist in der Figur 18 ebenso wie in der Figur 20 ein menschlicher Finger 117 als beispielhaftes Messobjekt dargestellt, dessen Substanz analysiert werden soll. Der Finger wird jeweils zur Analyse auf die Messfläche 118 aufgelegt.

In den Figuren 18 bis 23 sind im Bereich des Messkörpers jeweils Substrate 120 gezeigt, deren Material für einen Anregungsstrahl 121 im Infrarotbereich, im mittleren Infrarotbereich oder allgemein in dem Wellenlängenbereich des Anregungsstrahls 121 durchlässig ist. Dies gilt beispielsweise für ein Substrat 120 aus Silizium für den mittleren Infrarotbereich. Der Messkörper kann außer dem Substrat 120 jeweils noch andere Körper und Schichten umfassen, wie beispielsweise die piezoelektrischen Bereiche/den Detektionsbereich und eine oder mehrere Deckschichten zum mechanischen Schutz der Messfläche 118 und/oder zur Impedanzanpassung, die in den Figuren 18-23 an die Messfläche 118 angrenzend schematisch dargestellt sind. Ein Anregungsstrahl 121 kann, sofern das Substrat 120 für seine Wellenlängenbereiche transparent ist, durch das Substratmaterial hindurch auf die Messfläche 118 und durch diese hindurch in den zu vermessenden Stoff (beispielsweise den Finger 117) gerichtet werden. Es ist in einem solchen Fall nicht notwendig, für den Anregungsstrahl 121 eine Öffnung im Substrat 120 vorzusehen. Der Anregungsstrahl 121 kann an der Elektrodeneinrichtung 119 vorbei oder durch diese hindurch gelenkt werden.

An der der Messfläche 118 gegenüberliegenden Seite des Messkörpers oder des Substrats 120 ist in das Substrat 120 jeweils eine Linse 116, 116', 116" integriert, insbesondere durch das Material des Substrats 120 geformt und beispielsweise durch abtragende Verfahren, insbesondere durch Ätzen oder Sputtern aus dem Material des Substrats herausgearbeitet.

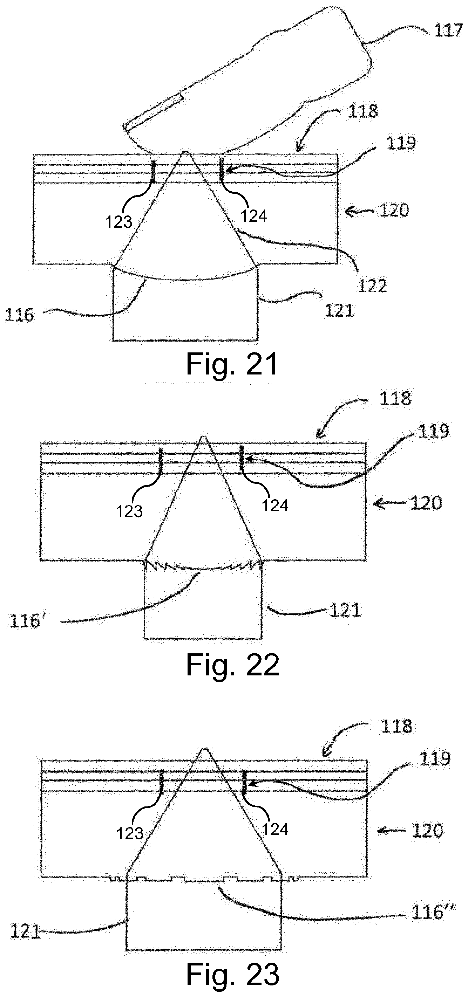

Es sind in den Figuren 18 bis 23 drei Beispiele für mögliche Linsenformen dargestellt, wobei die erste Linse in den Figuren 18 und 21 dargestellt ist, die zweite Linse in den Figuren 19 und 22 und die dritte Linse in den Figuren 20 und 23.

Die erste Linse 116 entspricht einer normal brechenden, refraktiven konvexen Sammellinse, die zweite Linse 116' entspricht einer Sammellinse (refraktiv) im Fresnellschliff (Kinoformlinse) und die dritte Linse 116" entspricht einer diffraktiven Linse, die eine Bündelung des Anregungsstrahls 10 durch Beugung an einer konzentrischen Gitterstruktur bewirkt. Die optischen Achsen der Linsen 116-116" können jeweils senkrecht auf der Messfläche 118 stehen, so dass eine Anregungslichtquelle unmittelbar gerade durch das Substrat 120 hindurchstrahlen kann. Jedoch können die optischen Achsen auch gegenüber der senkrechten zur Messfläche 118 schräggestellt sein, um eine möglicherweise platzsparende Positionierung der Anregungslichtquelle schräg zum Substrat zu erlauben.

In den Figuren 21, 22 und 23 sind jeweils die Linsenformen 116, 116', 116" am Substrat 120 mit den Anregungsstrahlen 121 und den auf den zu analysierenden Stoff fokussierten Strahlenbündeln 122 dargestellt.

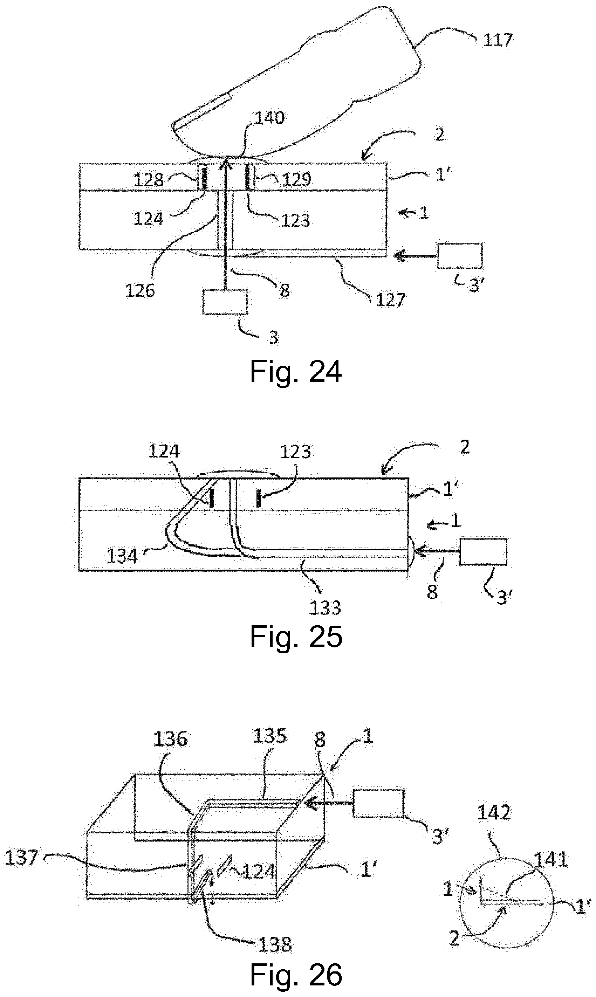

In der Figur 24 ist ein Messkörper 1 mit einer Sensorschicht 1' im Querschnitt dargestellt, bei dem ein Anregungsstrahl 8 von der Laseranordnung 3 aus in einen Lichtwellenleiter 126 geleitet wird, der den Messkörper 1 bis zur Schicht 1' durchsetzt. Der Lichtwellenleiter 126 kann auch durch die Schicht 1' hindurch bis zur Messfläche 2 reichen, jedoch kann auch vorgesehen sein, dass entweder die Schicht 1' eine Aussparung für den Anregungsstrahl 8 aufweist oder der Anregungsstrahl 8 durch das Material der Schicht 1' hindurchfällt. Im Bereich der Messfläche 2, beispielsweise unmittelbar an die Messfläche 2 angrenzend und/oder innerhalb der Schicht 1', kann eine Linse 140 zur Fokussierung des Anregungsstrahles 8 auf einen Punkt in dem zu untersuchenden Stoff vorgesehen sein. Der Lichtwellenleiter 126 verläuft gerade von der Laservorrichtung 3 bis zur Messfläche 2 und durchsetzt die Detektionseinrichtung und/oder den Bereich zwischen den Elektroden 123, 124. Um eine Störwirkung des Piezoelektrischen Effektes und der resultierenden Kraftwirkung des Materials des Detektionsbereichs auf den Lichtwellenleiter zu vermeiden, kann dieser aus einem Material bestehen, das keine oder eine minimale Abhängigkeit des Brechungsindex von äußeren Kräften oder Druck aufweist. Ein Lichtwellenleiter kann auch, beispielsweise, wenn die Lasereinrichtung seitlich neben dem Messkörper angeordnet ist, teilweise oder vollständig an und entlang der Oberfläche des Messkörpers verlaufen (vgl. Figur 26). In der Figur 24 verläuft der Lichtwellenleiter 127 von der Lasereinrichtung 3' zunächst auf einem ersten Teil seiner Länge an oder auf der Oberfläche des Messkörpers, um dann weiter auf einem zweiten Teil seiner Länge wie der Lichtwellenleiter 126 durch den Messkörper hindurch zu verlaufen. Der Anregungsstrahl 8 kann im Bereich der Richtungsänderung des Lichtwellenleiters beispielsweise an einem Spiegel reflektiert werden oder der Lichtwellenleiter kann dort gekrümmt sein. Ein solcher Lichtwellenleiter 126, 127 kann in das Material des Messkörpers fertigungstechnisch integriert (beispielsweise durch SOI- Silizium on Insulator Technologie,) oder als faseroptischer Lichtwellenleiter mit diesem beispielsweise durch Verkleben verbunden sein, oder der Lichtwellenleiter kann auf einem Teil seiner Länge integriert und auf einem anderen Teil seiner Länge als faseroptischer Lichtwellenleiter ausgeführt sein.

In der Figur 24 ist auch dargestellt, dass die Elektroden 123, 124 üblicherweise in Ausnehmungen oder Schlitzen des Messkörpers vorgesehen sind. Diese Ausnehmungen 128, 129, die in dem vorliegenden Fall mit einem Polymer durch Ausgießen verschlossen sind, dienen der Abtrennung eines Detektionsbereiches und damit eines den Detektionsbereich definierenden piezoelektrischen Körpers, der sich infolge einer Wärme- und/oder Druckwelle ausdehnen und zusammenziehen kann und dabei die piezoelektrischen, messbaren Effekte zeigt. Diese können dann durch die Elektroden erfasst werden.

Entsprechende Ausnehmungen 128, 129 können an allen im vorliegenden Text gezeigten Elektroden in den verschiedenen Messkörpern vorgesehen und gegebenenfalls mit einem nicht piezoelektrischen Material, beispielsweise mit einem Polymer, vergossen sein.

Die Materialaussparungen 128, 129 können beispielsweise bei Herstellung des Messkörpers 1 vorgesehen oder später durch Ätzen oder Sputtern oder durch Sägen oder Laserschneiden eingebracht sein.

Es kann jedoch, wie aus der Figur 25 in zwei verschiedenen Varianten der Lichtwellenleitergestaltung ersichtlich ist, auch ein gekrümmter Lichtwellenleiter 133, 134 vorgesehen sein, der den Anregungsstrahl von einer Position an dem Messkörper 1, an der die Lasereinrichtung 3' vorgesehen ist, zur Messfläche 2 führt. Dadurch, dass die Führung des Lichtwellenleiters 133, 134 relativ frei gestaltet werden kann, kann zwischen dem von dem Anregungsstrahl 8 durchsetzten Bereich und dem Detektionsbereich ein Mindest-Abstand gehalten werden. Der Anregungsstrahl 8 kann auch schräg zur Messfläche 2, beispielsweise in einem Winkel zwischen 0 Grad und 60 Grad, insbesondere zwischen 0 und 45 Grad zur Flächennormalen der Messfläche 2 auf die Messfläche 2 treffen und durch diese hindurchtreten.

Wegen der geringen Eindringtiefe in den zu analysierenden Stoff liegt der Bereich des Stoffes, in dem der Anregungsstrahl 8 mit diesem wechselwirkt, trotz einer schrägen Einstrahlrichtung unmittelbar unterhalb der Detektionseinrichtung und der Elektroden 123, 124. Die gekrümmten Lichtwellenleiter 133, 134 können beispielsweise wenigstens abschnittsweise als faseroptische Lichtwellenleiter in einer Bohrung oder ähnlichen Aussparung des Messkörpers 1 verlegt und dort eingeklebt oder eingegossen sein.

Es kann auch, wie aus der Figur 26 ersichtlich, zur Führung des Anregungsstrahls 8 ein Lichtwellenleiter 135, 136, 137, 138 vorgesehen sein, der beispielsweise in mehreren Richtungen und/oder in zwei oder drei aufeinander senkrecht stehenden Richtungen an einer oder zwei oder drei verschiedenen, aneinander angrenzenden Oberflächen des Messkörpers 1 entlang geführt ist. Ein solcher Lichtwellenleiter 135, 136, 137, 138 kann beispielsweise, wie auch die in den Figuren 24 und 25 gezeigten Lichtwellenleiter, in den jeweiligen Messkörper 1 integriert sein. An den Oberflächen eines Messkörpers ist dies in SOI- Technologie oder je nach dem Material des Messkörpers in einer verwandten Festkörper-Herstellungstechnologie besonders einfach möglich. In einem Silizium-Substrat kann dazu ein Lichtwellenleiter eingebracht sein, der durch Siliziumoxidschichten oder andere Schichten abgedeckt und von dem Substrat getrennt ist. Dazu kann zunächst in das Substrat eine passende Ausnehmung geätzt oder gesputtert werden, um danach das Material der Abdeckung und des Lichtwellenleiters geeignet abzuscheiden. Die Abdeckung des Lichtwellenleiters kann in diesem Fall beispielsweise mit der Oberfläche des Messkörpers bündig abschließen so dass der Lichtwellenleiter 135, 136, 137, 138 nicht über den Messkörper 1 hinausragt. Durch den Verlauf des Lichtwellenleiters 135, 136, 137, 138 entlang den Oberflächen des Messkörpers 1 wird eine Wechselwirkung des Anregungsstrahls 8 mit der Detektionseinrichtung und den Einwirkungen des piezoelektrischen Materials vermieden. Der letzte Lichtwellenleiter 138 endet dann in dem Bereich, in dem der Anregungsstrahl 8 in den zu analysierenden Stoff eintreten soll. Dort kann am Ende des Lichtwellenleiters 138 ein Element vorgesehen sein, das den Anregungsstrahl 8 in den Stoff hinein lenkt, beispielsweise ein Spiegel.

In dem unten rechts in der Figur in einem Kreis 142 dargestellten Ausschnitt der Figur 26 ist dargestellt, dass der Lichtwelleneiter 138 auch in einer schräg auf die Messfläche 2 zulaufenden Nut (gestrichelt dargestellt) des Messkörpers 1 angeordnet werden kann, so dass die Längsachse des Lichtwellenleiters parallel zu dem Boden 141 der Nut durch die Messfläche 2 hindurch auf den zu analysierenden Stoff ausgerichtet ist.

Die vorliegende Schutzrechtsanmeldung bezieht sich (wie einleitend bereits erwähnt) neben den oben beschriebenen Anspruchsgegenständen und Ausführungsbeispielen auch auf die folgenden Aspekte. Diese Aspekte oder einzelne ihrer Merkmale können einzeln oder in Gruppen jeweils mit Merkmalen der Ansprüche kombiniert werden. Die Aspekte stellen darüber hinaus auch jeweils für sich genommen oder miteinander oder mit Anspruchsgegenständen kombiniert eigenständige Erfindungen dar. Die Anmelderin behält sich vor, diese Erfindungen zu einem späteren Zeitpunkt zum Gegenstand von Ansprüchen zu machen. Dies kann im Rahmen dieser Anmeldung oder auch im Rahmen von späteren Teilanmeldungen oder Nachanmeldungen unter Inanspruchnahme der Priorität dieser Anmeldung geschehen.

### ASPEKTE:

1) Verfahren zum Analysieren eines Stoffes in einem Körper, umfassend:
   - Aussenden eines Anregungs-Lichtstrahls (Anregungsstrahls) mit einer oder mehreren spezifischen Anregungswellenlängen durch einen ersten Bereich der Oberfläche des Körpers,
   - Intensitätsmodulation des Anregungs-Lichtstrahls mit einer oder mehreren Frequenzen, insbesondere nacheinander, durch einen mechanischen, elektrischen oder optischen Chopper, insbesondere durch eine elektronische Ansteuerung der Anregungslichtquelle, eine Verstelleinrichtung für einen Resonator eines als Anregungslichtquelle dienenden Anregungslasers oder eine bewegliche Spiegeleinrichtung, eine steuerbare Beugungseinrichtung, eine Verschluss- oder Spiegeleinrichtung, die an einem Motor, wie einem Stepmotor, oder mit einem MEMS gekoppelt ist oder eine bezüglich der Transmission oder Reflexion steuerbare Schicht im Strahlengang,
   - mittels eines außerhalb des Körpers angeordneten Detektors die zeitaufgelöste Detektion eines Reaktionssignals, das auf die Wirkung der wellenlängenabhängigen Absorption des Anregungs-Lichtstrahls im Körper zurückgeht.

Der Detektor kann beispielsweise durch einen optischen Medium/Messkörper mit einem Detektionsbereich gebildet sein, der insbesondere der Messfläche (= mit dem zu analysierenden Stoff in Kontakt stehenden Grenzfläche des Messkörpers) benachbart oder unmittelbar benachbart, ist und der einen druck- oder temperaturabhängigen spezifischen elektrischen Widerstand aufweist und /oder bei Druck- oder Temperaturänderungen elektrische, insbesondere piezoelektrische Spannungssignale erzeugt, und mit einer elektrischen Kontakteinrichtung, die Elektroden aufweist, welche mit dem Detektionsbereich des optischen Mediums/Messkörpers elektrisch leitend zur Erfassung des elektrischen Widerstandes und/oder der elektrischen Signale verbunden sind, wobei mit der Kontakteinrichtung und dem Detektionsbereich eine Detektionseinrichtung gebildet ist.

Der Detektor/die Detektionseinrichtung kann beispielsweise ein piezoelektrisches Material aufweisen oder einen temperaturabhängigen Widerstand mit positivem oder negativem Temperaturkoeffizienten (Thermistor) oder ein Thermoelement.

Die Modulation kann in einer Ausführungsform durch Interferenz oder Beeinflussung der Phase oder Polarisation der Strahlung der Anregungssendeeinrichtung erfolgen, insbesondere, wenn diese eine Laserlichteinrichtung umfasst. Die Modulation kann auch durch Steuerung eines aktiv betriebenen Piezoelementes sein, das ein Teil/Element des Messkörpers ist und dessen Transmission oder Reflexionseigenschaft//Reflektivität durch eine Spannungssteuerung an dem Piezoelement steuerbar ist. Die Reaktionssignale können beispielsweise Intensitäten oder Ablenkungswinkel eines reflektierten Messstrahls oder Spannungssignale eines mit einem Piezoeffekt arbeitenden Detektors sein.

2) Verfahren nach Aspekt 1, dadurch gekennzeichnet, dass der Anregungslichtstrahl/Anregungsstrahl durch mehrere Emitter oder Multiemitter, insbesondere in Form eines Laser-Arrays, erzeugt wird, die Licht mit unterschiedlichen Wellenlängen gleichzeitig oder nacheinander oder in Pulsmustern, auch abwechselnd aussenden.

3) Verfahren nach Aspekt 1 oder 2, dadurch gekennzeichnet, dass an dem ersten Bereich der Oberfläche des Körpers ein akustisches Reaktionssignal durch einen akustischen Sensor erfasst wird.

4) Verfahren nach einem der Aspekte 1 bis 3, dadurch gekennzeichnet, dass an dem ersten Bereich der Oberfläche des Körpers ein Reaktionssignal durch einen Infrarotstrahlungssensor, insbesondere ein Thermoelement, ein Bolometer oder ein Halbleiterdetektor, zum Beispiel ein Quantum-Cascade-Detektor, oder einen Piezo-Detektor erfasst wird. Der Piezo-Detektor kann dabei beispielsweise in oder an einem Messkörper/optischen Medium gebildet sein.

5) Verfahren nach einem der Aspekte 1 bis 4, umfassend die Schritte:
- Herstellen des Kontakts eines optischen Mediums/Messkörpers mit einer Stoffoberfläche des Körpers, so dass zumindest ein Bereich der Oberfläche des optischen Mediums/Messkörpers (beispielsweise eine Messfläche) in Kontakt mit dem ersten Bereich der Oberfläche des Körpers steht;
- Aussenden eines Anregungs-Lichtstrahls mit einer Anregungswellenlänge in ein in dem Stoff unterhalb des ersten Bereiches der Oberfläche liegendes Volumen insbesondere durch den Bereich der Oberfläche des optischen Mediums hindurch, der in Kontakt mit dem ersten Bereich der Stoffoberfläche steht,
- Messen der Temperatur oder Temperaturänderung und/oder einer Druckänderung in dem ersten Bereich der Oberfläche des optischen Mediums durch ein optisches pyrometrisches oder photothermisches Verfahren,
- Analysieren des Stoffes anhand der detektierten Temperaturerhöhung in Abhängigkeit von der Wellenlänge des Anregungs-Lichtstrahls. Dabei kann dieser Vorgang bei einer Messung für verschiedene Modulationsfrequenzen durchgeführt werden und die Ergebnisse für verschiedene Modulationsfrequenzen können miteinander kombiniert werden.

6) Verfahren nach Aspekt 5, gekennzeichnet durch
das Aussenden eines Mess-Lichtstrahls durch das optische Medium/den Messkörper auf den Bereich der Oberfläche, des optischen Mediums, der in direktem Kontakt mit der Stoffoberfläche steht, derart, dass der Mess-Lichtstrahl und der Anregungslichtstrahl auf der Grenzfläche des optischen Mediums/Messkörpers und der Stoffoberfläche, an der der Mess-Lichtstrahl reflektiert wird, einander unmittelbar benachbart sind oder überlappen;
Direktes oder indirektes Detektieren einer Ablenkung des reflektierten Mess-Lichtstrahls in Abhängigkeit von der Wellenlänge des Anregungs-Lichtstrahls und
Analysieren des Stoffes anhand der detektierten Ablenkung des Mess-Lichtstrahls in Abhängigkeit von der Wellenlänge des Anregungs-Lichtstrahls. Dabei kann dieser Vorgang bei einer Messung für verschiedene Modulationsfrequenzen durchgeführt werden und die Ergebnisse für verschiedene Modulationsfrequenzen können miteinander kombiniert werden.

Dieses Verfahren kann beispielsweise auch mit einem flachen Messkörper und seitlicher Einstrahlung des Anregungsstrahls (im wesentlichen parallel zur Messfläche) sowie mit Einspiegelung des Anregungsstrahls zur Messfläche und zu dem zu analysierenden Stoff angewendet werden.

7) Verfahren nach einem der Aspekte 5 oder 6, dadurch gekennzeichnet, dass der Mess-Lichtstrahl durch dieselbe Lichtquelle erzeugt wird, die den Anregungs-Lichtstrahl erzeugt.

8) Verfahren nach einem der Aspekte 5, 6 oder 7, dadurch gekennzeichnet, dass der Mess-Lichtstrahl nach der Ablenkung und vor der Detektion innerhalb des optischen Mediums, außerhalb des optischen Mediums oder teilweise innerhalb und teilweise außerhalb des optischen Mediums ein- oder mehrmals reflektiert wird.

9) Verfahren nach Aspekt 1 oder einem der anderen vorangehenden bzw. folgenden, dadurch gekennzeichnet, dass der Anregungs-Lichtstrahl ein intensitätsmodulierter, insbesondere gepulster Anregungs-Lichtstrahl insbesondere im Infrarot-Spektralbereich ist, wobei insbesondere die Modulationsrate zwischen 1 Hz und 10 kHz, vorzugsweise zwischen 10Hz und 3000Hz liegt.

10) Verfahren nach Aspekt 1 oder einem der anderen vorangehenden bzw. folgenden, dadurch gekennzeichnet, dass das Licht des/der Anregungs- Lichtstrahlen durch eine integrierte Anordnung mit mehreren Einzellasern, insbesondere einem Laserarray, gleichzeitig oder nacheinander oder teilweise gleichzeitig und teilweise nacheinander erzeugt wird.

11) Verfahren nach Aspekt 1 oder einem der anderen vorangehenden bzw. folgenden, dadurch gekennzeichnet, dass aus den bei verschiedenen Modulationsfrequenzen des Anregungs-Lichtstrahls gewonnenen Reaktionssignalen eine Intensitätsverteilung der Reaktionssignale in Abhängigkeit von der Tiefe unter der Oberfläche ermittelt wird, in der die Reaktionssignale entstehen.

12) Verfahren nach Aspekt 1 oder einem der anderen vorangehenden bzw. folgenden, dadurch gekennzeichnet, dass aus der Phasenlage der Reaktionssignale im Verhältnis zu einem modulierten Anregungs- Lichtstrahl bei einer oder verschiedenen Modulationsfrequenzen des Anregungs-Lichtstrahls eine Intensitätsverteilung der Reaktionssignale in Abhängigkeit von der Tiefe unter der Oberfläche ermittelt wird, in der die Reaktionssignale entstehen.

13) Verfahren nach Aspekt 11 oder 12, dadurch gekennzeichnet, dass zur Ermittlung der Intensitätsverteilung der Reaktionssignale in Abhängigkeit von der Tiefe unter der Oberfläche die Messergebnisse zu verschiedenen Modulationsfrequenzen gewichtet und miteinander verknüpft werden.

14) Verfahren nach Aspekt 11, 12 oder 13, dadurch gekennzeichnet, dass aus der gewonnenen Intensitätsverteilung über die Tiefe unter der Oberfläche des Körpers eine Stoffdichte eines den Anregungs- Lichtstrahl in spezifischen Wellenlängenbereichen absorbierenden Stoffes in einer bestimmten Tiefe oder einem Tiefenbereich ermittelt wird.

15) Verfahren nach Aspekt 1 oder einem der anderen vorangehenden bzw. folgenden, dadurch gekennzeichnet, dass unmittelbar vor oder nach oder während der Detektion des Reaktionssignals/der Reaktionssignale wenigstens eine biometrische Messung an dem Körper in dem ersten Bereich der Oberfläche, in dem die Stoffanalyse vorgenommen wird oder unmittelbar diesem benachbart, insbesondere eine Vermessung eines Fingerabdrucks durchgeführt und der Körper, insbesondere eine Person identifiziert wird und dass insbesondere der Detektion der Reaktionssignale durch die Identifikation der Person zugehörige Referenzwerte (Kalibrierungswerte) zugeordnet werden.

Die Biometrische Messung kann auch die Vermessung eines Spektrums von Reaktionssignalen beim Durchfahren eines Spektrums des Anregungs-Lichtstrahls umfassen. Durch Auswertung des Spektrums kann ein Profil von in dem Körper vorhandenen Stoffen und ihr Mengen- oder Dichteverhältnis ermittelt werden, das die Identifizierung einer Person ermöglichen kann.

16) Vorrichtung zum Analysieren eines Stoffes,
mit einer Einrichtung zum Aussenden eines oder mehrerer Anregungs-Lichtstrahlen mit jeweils einer Anregungswellenlänge in ein in dem Stoff unterhalb eines ersten Bereiches seiner Oberfläche liegendes Volumen, mit einer Einrichtung zum Modulieren eines Anregungslichtstrahls, die durch eine Modulationseinrichtung der Strahlungsquelle, insbesondere ihrer Ansteuerung, eine Interferenzeinrichtung, eine Phasen- oder Polarisationsmoduliereinrichtung und/oder wenigstens einen, im Strahlengang angeordneten gesteuerten Spiegel, und/oder eine bzgl. ihrer Transparenz steuerbare, im Strahlengang angeordnete Schicht gebildet ist, sowie mit einer Detektionseinrichtung zur Detektion eines zeitabhängigen Reaktionssignals in Abhängigkeit von der Wellenlänge des Anregungslichts und der Intensitäts- Modulation des Anregungslichts und mit einer Einrichtung zum Analysieren des Stoffes anhand der detektierten Reaktionssignale.

17) Vorrichtung nach Aspekt 16 mit einer Einrichtung zur Ermittlung von Reaktionssignalen gesondert nach verschiedenen Intensitäts-Modulationsfrequenzen und/oder mit einer Einrichtung zur Ermittlung von Reaktionssignalen in Abhängigkeit von der Phasenlage des jeweiligen Reaktionssignals relativ zur Phase der Modulation des Anregungs- Lichtstrahls, insbesondere in Abhängigkeit von der Modulationsfrequenz des Anregungs- Lichtstrahls.

18) Vorrichtung zum Analysieren eines Stoffes nach Aspekt 16 oder 17, mit einem optischen Medium/einem Messkörper zum Herstellen des Kontakts der Oberfläche des optischen Mediums (beispielsweise einer sogenannten Messfläche) mit einem ersten Bereich der Stoffoberfläche, sowie mit
einer Einrichtung zum Aussenden eines Anregungs-Lichtstrahls mit einer oder mehreren Anregungswellenlängen in ein in dem Stoff unterhalb des ersten Bereiches der Oberfläche liegendes Volumen insbesondere durch den Bereich der Oberfläche des optischen Mediums (der Messfläche) hindurch, der in Kontakt mit der Stoffoberfläche steht, sowie mit einer Einrichtung zum
Messen von Reaktionssignalen in Form von Temperatur- und/oder Druckänderungen in dem Bereich innerhalb des Messkörpers in der unmittelbaren Nachbarschaft der Messfläche (einem sogenannten Detektionsbereich), die mit dem ersten Bereich der Stoffoberfläche im Kontakt steht durch ein optisches Verfahren, das sich eines Mess- Lichtstrahls bedient oder durch das oben beschriebene Verfahren unter Verwendung eines Piezo- Effektes, und mit einer Einrichtung zum Analysieren des Stoffes anhand der detektierten Reaktionssignale in Form von Temperaturänderungen/Druckänderungen in Abhängigkeit von der Wellenlänge des Anregungs-Lichtstrahls und der Intensitätsmodulation des Anregungs-Lichtstrahls, insbesondere der Modulationsfrequenz des Anregungslichtstrahls.

Bei diesem Aspekt und den folgenden, sich auf diesen beziehenden Aspekten kann auch vorgesehen sein, dass der Messkörper einen ersten Teil aufweist, der eine Ausnehmung/Aussparung in Form eines durchgehenden Kanals für den Anregungsstrahl aufweist und dass der Messkörper an seiner Unterseite auf dem ersten Teil eine Sensor-Schicht aufweist, die entweder durchgehend ohne Ausnehmung/ Aussparung für den Anregungsstrahl oder mit einer Fortsetzung der Ausnehmung des ersten Teils versehen ist. Ist die Sensor-Schicht dünn genug, beispielsweise dünner als 200 Mikrometer, insbesondere dünner als 100 Mikrometer, so kann der Anregungsstrahl diese je nach dem gewählten Material der Schicht auch, wenn es sich um einen Infrarotstrahl handelt, ohne zu große Absorption durchlaufen und eine Ausnehmung/ Aussparung in der Sensor- Schicht ist nicht notwendig. Die Sensor- Schicht des Messkörpers kann mit dem ersten Teil/übrigen Teil des Messkörpers verklebt oder mittels einer anderen Fügetechnik zusammengefügt sein und aus einem Material bestehen, das piezoelektrische Eigenschaften aufweist und einen Detektionsbereich gemäß der Erfindung ausbildet. Die Sensorschicht kann auch aus einem Material bestehen, bei dem eine Temperatur- und/oder Druckänderung eine Änderung des Brechungsindex bewirkt, so dass auch diese Änderung als Reaktionssignal, beispielseise durch die Detektion des Reflexionswinkels eines Detektionsstrahls, der in oder an der Sensor- Schicht reflektiert wird, nachgewiesen werden kann. Der erste Teil/übrige Teil des Messkörpers kann dann beispielsweise aus einem Material bestehen, das im sichtbaren Bereich und für einen Detektionsstrahl durchlässig, jedoch im Infraroten Spektralbereich weniger durchlässig oder undurchlässig ist, wie Quarz oder Saphir oder einem Kunststoff, beispielsweise einem Polymer.

19) Vorrichtung nach Aspekt 18, dadurch gekennzeichnet, dass die Anregungs-Lichtquelle unmittelbar mit dem optischen Medium/Messkörper mechanisch fest verbunden ist.

20) Vorrichtung nach Aspekt 18, dadurch gekennzeichnet, dass eine Einrichtung zum Aussenden eines Mess-Lichtstrahls in den Bereich des optischen Mediums/Messkörper vorgesehen ist, der mit dem ersten Bereich der Stoffoberfläche im Kontakt steht und dass diese Einrichtung/oder die Detektionseinrichtung zur Detektion des Mess- Lichtstrahls unmittelbar mit dem optischen Medium/Messkörper mechanisch fest verbunden, in diesen integriert oder mittels eines Lichtwellenleiters an dieses angekoppelt ist.

21) Vorrichtung nach Aspekt 18, 19 oder 20, dadurch gekennzeichnet, dass das optische Medium/der Messkörper unmittelbar eine Abbildungsoptik trägt und/oder dass in das optische Medium/den Messkörper eine Abbildungs-Optik integriert ist.

22) Vorrichtung nach Aspekt 18 oder einem der anderen vorangehenden bzw. folgenden, dadurch gekennzeichnet, dass die Oberfläche des optischen Mediums/Messkörpers mehrere gegeneinander geneigte Teilflächen aufweist, an denen der Mess-Lichtstrahl mehrfach reflektiert wird.

23) Vorrichtung nach Aspekt 18 oder einem der anderen vorangehenden bzw. folgenden, dadurch gekennzeichnet, dass in oder an dem optischen Medium/Messkörper ein oder mehrere Spiegelflächen zur Reflektion des Mess- Lichtstrahls vorgesehen sind.

24) Durch die Mehrfachreflexion des Messstrahls wird der Weg des Strahls verlängert, so dass sich Winkelabweichungen besser detektieren lassen (vgl. auch Fig. 17).

25) Vorrichtung nach Aspekt 16 oder 17, dadurch gekennzeichnet, dass die Detektionseinrichtung zur Detektion eines zeitabhängigen Reaktionssignals einen akustischen Detektor zur Erfassung akustischer Wellen an der Stoffoberfläche, insbesondere mit einem Resonator, weiter insbesondere mit einem Helmholtzresonator.

Es kann unabhängig davon als Detektor eine Quartzgabel mit vorzugsweise der gleichen Resonanzfrequenz wie ein vorgesehener Resonator dienen. Der Resonator kann offen oder geschlossen sein. Die Quartzgabel ist vorzugsweise im oder am Hals des Resonantors (offbeam) oder inner-/außerhalb des Resonators (in-beam) angeordnet.

26) Vorrichtung nach Aspekt 16, 17 oder 18, dadurch gekennzeichnet, dass die Detektionseinrichtung zur Detektion eines zeitabhängigen Reaktionssignals einen Wärmestrahlungs- Detektor zur Erfassung der Wärmestrahlung an der Stoffoberfläche, insbesondere einen Infrarotdetektor, weiter insbesondere ein Thermoelement, ein Bolometer, oder einen Halbleiterdetektor oder ein Piezoelement aufweist.

27) Vorrichtung nach einem der Aspekte 16 bis 25, dadurch gekennzeichnet, dass die Anregungs-Lichtquelle und die Detektionseinrichtung unmittelbar aneinander oder an einem gemeinsamen Träger befestigt sind, der insbesondere durch ein Gehäuse oder Gehäuseteil der Vorrichtung gebildet ist.

28) Vorrichtung nach einem der Aspekte 16 bis 26, dadurch gekennzeichnet, dass die Vorrichtung ein tragbares Gehäuse aufweist, das am Körper einer Person befestigbar ist, wobei die Einrichtung zum Aussenden eines oder mehrerer Anregungs-Lichtstrahlen und die Detektionseinrichtung zur Detektion eines zeitabhängigen Reaktionssignals derart angeordnet und eingerichtet ist, dass der zu analysierende Stoff im Betrieb, wenn die Vorrichtung am Körper getragen wird, auf der dem Körper abgewandten Seite des Gehäuses vermessen wird, insbesondere, dass die Messfläche des Messkörpers sich auf der dem Körper abgewandten Seite befindet.

29) Vorrichtung nach einem der Aspekte 16 bis 26, dadurch gekennzeichnet, dass die Vorrichtung ein tragbares Gehäuse aufweist, das am Körper einer Person befestigbar ist und dass das Gehäuse der Vorrichtung ein für den Anregungs- Lichtstrahl durchlässiges Fenster auf seiner bei der vorgesehenen Trageposition vom Körper abgewandten Seite aufweist.

Das Fenster kann unmittelbar vor dem Messkörper angeordnet sein oder durch die Messfläche des Messkörpers gebildet sein.

29a) Vorrichtung zum Analysieren eines Stoffes mit einer Anregungssendeeinrichtung zum Erzeugen mindestens eines elektromagnetischen Anregungsstrahls, insbesondere Anregungs-Lichtstrahls, mit zumindest einer Anregungswellenlänge, einer Detektionseinrichtung zur Detektion eines Reaktionssignals und einer Einrichtung zum Analysieren des Stoffes anhand des detektierten Reaktionssignals.

30) Vorrichtung nach einem der vorhergehenden Aspekte 16 bis 29a, dadurch gekennzeichnet, dass die Detektionseinrichtung zur Messung der Deformation eines Kristalls oder eines anderen im sichtbaren Bereich des Spektrums durchsichtigen Materials eingerichtet ist.

Die Deformation kann analog zur photothermischen ,Bouncing Method' durch die Wahl von steileren (größeren) Einfallswinkeln des Messstrahls zur Probeoberfläche effektiver gemessen werden und der Einfluss der Mirage-Effekt bedingten Ablenkung des Messstrahls minimiert werden.

### Literatur:

M. Bertolotti, G.L. Liakhou, R. Li Voti, S. Paolino, and C. Sibilia. Analysis of the photothermal deflection technique in the surface refection theme: Theory and Experiment. Journal of Applied Physics 83, 966 (1998)

Ein Cantilever kann entweder direkt auf der Probe platziert werden oder auf einem hinreichend dünnen optischen Medium auf welchem die Probe auf der einen Seite und der Cantilever auf der gegenüberliegenden Seite platziert wird. Durch die thermische Expansion der Probe bzw. des optischen Elements wird der Cantilever in Folge der durch die Absorption des modulierten Pumpstrahls/Anregungsstrahls bedingte thermische Expansion in Schwingung versetzt. Der Messstrahl wird an einer Messfläche des Cantilevers reflektiert und bedingt durch die Schwingung abhängig von der eingestrahlten Wellenlänge und den thermischen Eigenschaften der Probe, sowie der Modulationsfrequenz, abgelenkt. Diese Ablenkung wird detektiert.

31) Vorrichtung nach einem der vorhergehenden Aspekte 16 bis 30, dadurch gekennzeichnet, dass die Anregungssendeeinrichtung einen Abfragelaser oder eine LED, beispielsweise eine NIR(near-infrared)-LED, enthält.

32) Vorrichtung nach einem der vorhergehenden Aspekte 16 bis 31, dadurch gekennzeichnet, dass die Anregungssendeeinrichtung einen Probe-Laser aufweist, der einen kleineren Durchmesser als ein zusätzlicher Pump-Laser (= Laser zur Erzeugung des Anregungsstrahls) hat.

33) Vorrichtung nach einem der vorhergehenden Aspekte 16 bis 32, dadurch gekennzeichnet, dass zur Erzielung eines günstigeren Signal-/Rausch-Verhältnis eine spezielle Beschichtung, insbesondere des Emitters, z. B. IRE, vorgesehen ist, so dass Wärme besser abgeführt wird (z. B. "Wärmeleitpaste").

Das optische Element kann an der Kontaktfläche derartig beschichtet sein, dass eine verbesserte Ableitung des thermischen Signals in das optische Medium erfolgen kann. Zudem kann die Beschichtung des weiteren auch als Kratzschutz dienen, und auch durch geschickte Materialwahl eine reflektierende Oberfläche für den Messstrahl darstellen. Hierbei muss die Transparenz für das Anregungslicht zwingend gegeben bleiben.

34) Vorrichtung nach einem der vorhergehenden Aspekte 16 bis 33, dadurch gekennzeichnet, dass die Vorrichtung eine Einrichtung für
i. Pulstrains / Doppelmodulation
ii. Schwingspiegel
iii. MEMS Interferometer
aufweist.

35) Vorrichtung nach einem der vorhergehenden Aspekte 16 bis 34, dadurch gekennzeichnet, dass die Vorrichtung für eine Person am Körper in einer Ausführungsform durch eine mit dem Gehäuse verbundene Halteeinrichtung wie einen Riemen, ein Band oder eine Kette oder eine Spange, dauerhaft tragbar ausgeführt ist und/oder die Detektionseinrichtung eine Detektionsfläche aufweist, die auch als Anzeigefläche für Informationen wie Messwerte, Uhrzeiten und/oder Textinformationen nutzbar ist.

Die Detektionsfläche kann mit der Messfläche identisch sein oder ihre Verlängerung/Erweiterung bilden.

36) Vorrichtung nach dem vorhergehenden Aspekt 35, dadurch gekennzeichnet, dass die Vorrichtung eine Abziehfolie im Bereich der Detektionsfläche/Messfläche, vorzugsweise neben der Detektionsfläche/Messfläche, zur Vorbehandlung der Stoffoberfläche und Sicherstellung einer sauberen Oberfläche und/ oder in einer Ausführungsform im Fall der Glucosemessung konkret zur Hautreinigung aufweist.

37) Vorrichtung nach einem der vorhergehenden Aspekte 16 bis 36, dadurch gekennzeichnet, dass die Detektionseinrichtung eingerichtet ist zum Lesen und Erkennen von Fingerabdrücken zum Abruf bestimmter Werte/ Kalibrierungen einer Person und/oder dass sie eine Vorrichtung zur Erkennung der Lage eines Fingers, vorzugsweise zum Erkennen und Bestimmen einer ungewollten Bewegung während der Messung, aufweist.

38) Vorrichtung nach einem der vorhergehenden Aspekte 16 bis 37, dadurch gekennzeichnet, dass die Detektionseinrichtung eine Ergebnisanzeige aufweist, die vorzugsweise mit Farbkodierung, als analoge Anzeige, in einer Ausführungsform inkl. Fehleranzeige (beispielhaft: "100mg/dl plus/minus 5mg/dl"), akustisch und/ oder mit Ergebnisanzeige von Messwerten in größeren Schritten als die Messgenauigkeit der Vorrichtung es erlaubt(Beispielsweise durch eine mehrfarbige Ampelanzeige) ausgeführt ist. Hierdurch werden z.B. kleine Schwankungen, die einen Benutzer verunsichern könnten, nicht mitgeteilt.

39) Vorrichtung nach einem der vorhergehenden Aspekte 16 bis 38, dadurch gekennzeichnet, dass die Vorrichtung
zum Austausch gemessener Daten sowie zum Abruf von Kalibrier- oder Identifikationsdaten oder anderen Daten von anderen Geräten oder Cloud-Systemen Datenschnittstellen, beispielsweise leitungsgebundene oder nicht leitungsgebundene Schnittstellen (Infrarot-, Licht- oder Funkschnittstellen) aufweist,
wobei die Vorrichtung vorzugsweise so eingerichtet ist, dass die Datenübertragung verschlüsselt, insbesondere durch Fingerabdruck oder andere biometrische Daten des Bedieners verschlüsselt, erfolgen kann.

40) Vorrichtung nach einem der vorhergehenden Aspekte 16 bis 39, dadurch gekennzeichnet, dass die Vorrichtung so eingerichtet ist, dass ein Vorschlag für eine der Person zu gebende Insulindosis oder zu verzehrende Substanzen/Lebensmittel mitsamt der zu verzehrenden Menge durch die Vorrichtung ermittelbar sind (z.B. Insulinkorrekturfaktor) und/oder dass das Körpergewicht, Körperfett mitmessbar und/oder manuell eingebbar oder von anderen Geräten an die Vorrichtung übermittelbar ist.

41) Vorrichtung nach einem der vorhergehenden Aspekte 16 bis 40, dadurch gekennzeichnet, dass die Vorrichtung zur Erhöhung der Messgenauigkeit eingerichtet ist zur Ermittlung weiterer Parameter, in einer Ausführungsform mittels Sensoren zum Ermitteln der Hauttemperatur, Diffusivität/ Leitfähigkeit/ Feuchtigkeit der Haut, zur Messung der Polarisation des Lichtes (Ausschluss von Wasser/Schweiß auf der Fingeroberfläche).

Wasser und Schweiß auf der Hautoberfläche eines Menschen, die die Glucosemessung beeinflussen können, können durch eine Test- Anregung mit einer Anregungsstrahlung durch die Anregungssendeinrichtung mit den wasserspezifischen Banden bei 1640 cm-1 (6.1 µm) und 690 cm-1 (15 µm) detektiert werden. Sollte die Absorption einen bestimmten Wert überschreiten ist der Messort/die Stoffoberfläche/Hautoberfläche zu nass für eine zuverlässige Messung. Alternativ kann die Leitfähigkeit des Stoffes in der Nähe oder direkt an der Messstelle gemessen werden, um die Feuchtigkeit zu bestimmen. Es kann dann eine Fehlermeldung sowie eine Anweisung zum Trocknen ausgegeben werden.

42) Vorrichtung nach einem der vorhergehenden Aspekte 16 bis 41, dadurch gekennzeichnet, dass die Vorrichtung über eine Abdeckung im Strahlengang des Pump- und/oder Messstrahl-Lasers verfügt. Hierdurch kann für die obligatorische Augensicherheit von Lebewesen gesorgt werden.

43) Vorrichtung nach einem der vorhergehenden Aspekte 16 bis 42, dadurch gekennzeichnet, dass die Vorrichtung über eine wechselbare Detektionsfläche/Messfläche verfügt.

44) Vorrichtung nach einem der vorhergehenden Aspekte 16 bis 43 dadurch gekennzeichnet, dass die Vorrichtung als optisches Medium/Messkörper über einen stellenweise geriffelten oder aufgerauhten Kristall als Messkörper verfügt, der der Probe (z.B. dem Finger) einen bessere Justierung ermöglicht. Die Messstelle, auf die die zu analysierende Stoffoberfläche aufgelegt wird, ist vorzugsweise ohne Riffelung und glatt ausgebildet.

45) Vorrichtung nach einem der vorhergehenden Aspekte 16 bis 44 dadurch gekennzeichnet, dass für den Messstrahl eine zylindrische TEMpl TEMoo Mode oder anstelle der zylindrischen TEMpl TEMoo Mode andere Moden TEMo1 (Dougnut) TEMo2 oder TEMo3 verwendet werden. Besonders die letzteren haben den Vorteil das ihre Intensität auf das Sensitivitäts-Profil der Quadrantendiode abgestimmt werden können, die den Detektor für den abgelenkten Messstrahl darstellt. Desweiteren können rechteckige Moden TEMmn benutzt werden wie TEM30 oder TEMo3 oder höher. Damit können Abtast/Messstrahlen genutzt werden die in horizontaler oder vertikaler Richtung Störungsunempfindlicher sind.

46) Vorrichtung nach einem der vorhergehenden Aspekte 16 bis 45, dadurch gekennzeichnet, dass die Vorrichtung nicht nur an einem Punkt, sondern in einem Raster misst. Dies kann entweder durch eine Verschiebung des Pump- oder des Probe-Lasers oder der Detektionseinheit gegenüber der Hautoberfläche eines Probanden geschehen. Anstatt einer Verschiebung ist auch ein oder mehrere Arrays von Pump- oder Probe-Lasern denkbar, die über das Array räumlich verteilt sind.

Zudem sind auch noch folgende Aspekte der Erfindung anzuführen:
47) Vorrichtung (10) zum Analysieren eines Stoffes, insbesondere auch nach einem der Ansprüche 16 bis 46, mit
   einer Anregungssendeeinrichtung/Lasereinrichtung zum Erzeugen mindestens eines elektromagnetischen Anregungsstrahls, insbesondere Anregungs-Lichtstrahls, mit zumindest einer Anregungswellenlänge,
   einer Detektionseinrichtung zur Detektion eines Reaktionssignals und
   einer Einrichtung) zum Analysieren des Stoffes anhand des detektierten Reaktionssignals.
   Das zeitabhängige Reaktionssignal kann in der Temperatur- oder Druckerhöhung im Messkörper sowie in jeder diese nachweisenden Messgröße bestehen, beispielsweise in der Ablenkung eines Messstrahls oder einem elektrischen Signal eines im oder am Messkörper befindlichen Piezoelementes.
48) Vorrichtung nach Aspekt 47, (Bezugszeichen beziehen sich auf die Figur 17)
   dadurch gekennzeichnet, dass
   die Anregungssendeeinrichtung eine Strahlungsquelle 3, in einer Ausführungsform eine monochromatische, insbesondere eine polarisierte Strahlungsquelle, weiter insbesondere eine Laserlichtquelle ist,
   die Vorrichtung ein optisches Medium/einen Messkörper 1, 1' aufweist, das/der mit dem Stoff 5, insbesondere einem ersten Bereich 5a der Oberfläche des Stoffs, in direktem Kontakt steht (Der Messkörper kann insgesamt homogen sein und aus einem Material bestehen, dessen Brechungsindex sich mit der Temperatur ändert oder er kann zumindest im Bereich der Messfläche eine Schicht 1' aus einem solchen Material aufweisen oder eine Schicht, bei der sich der Brechungsindex in Abhängigkeit von der Temperatur stärker ändert als in den übrigen Bereichen des Messkörpers),
   wobei vorzugsweise die Anregungssendeeinrichtung so angeordnet ist, dass der ausgesendete Anregungsstrahl 8 das optische Medium/den Messkörper 1, 1' (nicht notwendig das Material des Messkörpers) passiert und die Kontur des Messkörpers an der Oberfläche des optischen Mediums/an der Messfläche wieder verlässt, und
   die Vorrichtung eine Einrichtung 105 zum Aussenden eines Messstrahls insbesondere Mess-Lichtstrahls 112, umfasst, die so angeordnet ist, dass der ausgesendete Messstrahl in das optische Medium eindringt und wobei vorzugsweise im Betrieb der Messstrahl und der Anregungsstrahl sich auf einer Grenzfläche/Messfläche 2 des optischen Mediums und der Oberfläche des Stoffs, an der der Messstrahl (112) reflektiert wird, überlappen,(dieser Bereich kann identisch oder teilweise identisch mit der Messfläche sein) und
   die Detektionseinrichtung eine Einrichtung 106 zum Empfangen des das Reaktionssignal bildenden reflektierten Messstrahls 112 und/oder zum direkten oder indirekten Detektieren einer Ablenkung des reflektierten Messstrahls ist.

   - Dabei kann zudem vorgesehen sein, dass die Anregungssendeeinrichtung 3 mehr als zwei Sendeelemente in Form von Lasern, insbesondere in Form eines ein-, zwei- oder mehrdimensionalen Sendeelementarrays, umfasst und sich die festen Wellenlängen der elektromagnetischen Anregungsstrahlen der zwei oder mehr Sendeelemente unterscheiden, und
   - eine Modulationseinrichtung für eine Intensitätsmodulation des Anregungsstrahls vorgesehen ist, sowie dass
   - eine Detektionseinrichtung zur Detektion eines Reaktionssignals vorgesehen ist sowie
   - eine Einrichtung 107, 109 zum Analysieren des Stoffes anhand des detektierten Reaktionssignals,
   - wobei der Messkörper aus einem für den Messstrahl transparenten Material, insbesondere Glas, Kristall oder einem transparenten Kunststoff besteht, wobei die Detektionseinrichtung eine Einrichtung 106 zum Empfangen des das Reaktionssignal bildenden ein- oder mehrmals (gemäß Fig. 17 außer in der Schicht 1' noch an zwei weiteren Reflexionselementen 114, 115 zur Verlängerung des Strahlengangs) innerhalb des Messkörpers reflektierten Messstrahls und/oder zum direkten oder indirekten Detektieren einer Ablenkung des reflektierten Messstrahls ist und die Einrichtung zum Aussenden eines Messstrahls und die Detektionseinrichtung derart zueinander ausgerichtet sind, dass die Detektionseinrichtung als das zeitabhängige Reaktionssignal den Messstrahl detektiert, nachdem dieser zumindest einmal im Bereich der Messfläche des optischen Mediums /Messkörpers reflektiert worden ist. Dabei kann die Detektionseinrichtung 106 ein ortsempfindliches fotoelektrisches Nachweiselement, beispielsweise eine Quadrantendiode aufweisen, das im Strahlengang des Messlichtstrahls hinter der an der Messfläche liegenden Reflexionsstelle angeordnet ist und die Position des Messstrahls erfasst.

Die Detektionseinrichtung ist somit zur Detektion eines zeitabhängigen Reaktionssignals in Abhängigkeit von der Wellenlänge des Anregungslichts und/oder der Intensitäts-Modulation des Anregungslichts geeignet. Zu diesem Zweck ist die Auswerteeinrichtung 109 auch mit einer Modulationseinrichtung 9 für den Anregungsstrahl verbunden. Ferner ist die Vorrichtung dazu geeignet, anhand des detektierten Reaktionssignals den Stoff zu analysieren, wobei nacheinander unter Verwendung verschiedener Modulationsfrequenzen der Anregungssendeeinrichtung jeweils für verschiedene Wellenlängen des Anregungsstrahls Reaktionssignale, insbesondere zeitliche Reaktionssignalverläufe, ermittelt werden und mehrere Reaktionssignalverläufe zu verschiedenen Modulationsfrequenzen durch die Auswerteeinrichtung 109 miteinander verknüpft werden und dass daraus eine für einen Tiefenbereich unter der Stoffoberfläche spezifische Information gewonnen wird.

Auch für die Ausführungsformen gemäß Aspekt 47 oder 48 ist es denkbar, den Messkörper als Flachkörper auszubilden, mit einer Dicke/Abmessung senkrecht zur Messfläche, die kleiner als 50%, insbesondere kleiner als 20%, weiter insbesondere kleiner als 10% der geringsten der Abmessungen des Messkörpers parallel zur Messfläche sein kann. Die Anregungssendeeinrichtung/die Lasereinrichtung zur Erzeugung des Anregungsstrahls kann dann seitlich neben dem Messkörper derart angeordnet und ausgerichtet sein, dass sie den Anregungsstrahl im wesentlichen parallel zur Messfläche (oder mit einer Winkelabweichung von weniger als 20 Grad von dieser Richtung)in den Messkörper einstrahlt.(Dies kann erfordern, dass der Anregungsstrahl von der Anregungssendeeinrichtung aus in einen Lichtwellenleiter und von dort in den Messkörper eingekoppelt wird. Es kann aber auch zwischen der Anregungssendeeinrichtung/Lasereinrichtung und dem Messkörper eine Spiegeleinrichtung vorgesehen sein, so dass der Anregungsstrahl zunächst von der Anregungssendeeinrichtung ausgehend durch einen ersten Spiegel in Richtung zu einer seitlichen, gedachten Verlängerung der Messfläche reflektiert und danach in eine Richtung parallel zur Messfläche umgelenkt wird). Der Anregungsstrahl kann dann zur Messfläche umgelenkt werden und von dort in den zu analysierenden Stoff eindringen.

49) Vorrichtung nach einem der Aspekte 47 oder 48,
dadurch gekennzeichnet, dass
die Vorrichtung ein optisches Medium/einen Messkörper aufweist, das mit dem Stoff, insbesondere einem ersten Bereich der Oberfläche des Stoffs, in direktem Kontakt steht und dass die Detektionseinrichtung zur Detektion eines Reaktionssignals eine Parameteränderung des optischen Mediums/Messkörpers, insbesondere in einem dem ersten Bereich benachbarten Bereich, infolge des Reaktionssignals, insbesondere eine Verformung und/oder Dichteänderung oder eine Änderung des Brechungsindex des optischen Mediums, erfasst.

Bei den Vorrichtungen der oben genannten Art, insbesondere bei den Vorrichtungen gemäß den Aspekten 47, 48 oder 49 kann insbesondere auch vorgesehen sein, dass der Messkörper im Bereich der Messfläche mit einem Material beschichtet ist, das seinen Brechungsindex in Abhängigkeit von der Temperatur oder dem Druck stärker ändert als der übrige Bereich des Messkörpers, wobei die Beschichtung vorteilhaft dünner als 1 mm , weiter vorteilhaft dünner als 0,5 mm, insbesondere dünner als 0,2 mm oder dünner als 0,1mm ist. Die Beschichtung kann auch als aufgeklebte oder einem übrigen /ersten Teil des Messkörpers angefügte Sensor- Schicht ausgebildet sein.

In den übrigen Teil des Messkörpers, der sich an die Beschichtung oder Sensor-Schicht anschließt, kann eine Ausnehmung 13 (vgl. Fig. 17) derart eingebracht sein, dass der Anregungsstrahl in diesem Bereich des Messkörpers das Material zumindest eines ersten Teiles des Messkörpers nicht berührt oder durchläuft. Der übrige Bereich des Messkörpers muss dabei für den Messstrahl, das heißt, im sichtbaren Bereich des Spektrums durchlässig sein, so dass dieser Messstrahl zur Beschichtung/Sensorschicht gelangen und an dieser oder in dieser reflektiert werden kann.

Das Material des Messkörpers, das sich an die Beschichtung/Sensor-Schicht 1' (vgl. auch Figur 17) anschließt, kann dabei eine spezifische Wärmekapazität oder Wärmeleitfähigkeit aufweisen, die größer ist als diejenige des Materials der Beschichtung 1', so dass der übrige Teil des Messkörpers, der sich an die Beschichtung anschließt, als Wärmesenke für die Beschichtung dienen kann. Alternativ oder zusätzlich kann an dem Messkörper auch eine zusätzliche Wärmesenke oder ein Peltierelement 110 vorgesehen sein, mittels dessen sich mit einer Regelungseinrichtung die Temperatur des Messkörpers regeln lässt.

Der Reflexionswinkel des Messstrahls stellt in diesem Fall das zu erfassende Reaktionssignal dar.

50) Vorrichtung nach einem der Aspekte 47, 48 oder 49,
dadurch gekennzeichnet, dass
die Detektionseinrichtung ein mit dem optischen Medium verbundenes oder in dieses integrierte Piezoelement als Detektor zur Erfassung der Verformung und/oder Temperatur- oder Dichteänderung aufweist.

51) Vorrichtung nach Aspekt 47 oder einem der folgenden,
dadurch gekennzeichnet, dass die Detektionseinrichtung Temperatursensoren als Detektor zur Erfassung des Reaktionssignals aufweist.

52) Vorrichtung nach Aspekt 47 oder einem der folgenden,
dadurch gekennzeichnet, dass
die Vorrichtung eine Einrichtung zur Intensitätsmodulierung des Anregungslichtstrahls aufweist und
die Detektionseinrichtung zur Detektion eines zeitabhängigen Reaktionssignals in Abhängigkeit von der Wellenlänge des Anregungslichts und/oder der Intensitäts-Modulation des Anregungslichts geeignet ist.

53) Vorrichtung nach Aspekt 47 oder einem der folgenden,
dadurch gekennzeichnet, dass
die Anregungssendeeinrichtung/Laserlichtquelle zur Erzeugung des Anregungsstrahls den mindestens einen elektromagnetischen Anregungsstrahl in ein Stoffvolumen einstrahlt, das unterhalb eines ersten Bereiches der Oberfläche des Stoffs liegt.

54) Vorrichtung nach Aspekt 47 oder einem der folgenden,
dadurch gekennzeichnet, dass
die Anregungssendeeinrichtung/ Laserlichtquelle zur Erzeugung des Anregungsstrahls zwei oder mehr Sendeelemente, insbesondere in Form eines ein-, zwei- oder mehrdimensionalen Sendeelementarrays, umfasst.

Die einzelnen Sendeelemente können dabei beispielsweise QC Laser oder Halbleiterlaser mit fester Wellenlänge sein.

55) Vorrichtung nach Aspekt 47 oder einem der folgenden,
dadurch gekennzeichnet, dass
die zwei oder mehr Sendeelemente jeweils einen eigenen elektromagnetischen Anregungsstrahl erzeugen und diesen in das Volumen unterhalb des ersten Bereiches einstrahlen.

56) Vorrichtung nach Aspekt 47 oder einem der folgenden
dadurch gekennzeichnet, dass
sich die Wellenlängen der elektromagnetischen Anregungsstrahlen der zwei oder mehr Sendeelemente unterscheiden.

57) Vorrichtung nach Aspekt 47 oder einem der folgenden,
dadurch gekennzeichnet, dass
die Anregungssendeeinrichtung/ Laserlichtquelle zur Erzeugung des Anregungsstrahls zwei oder mehr Laser, insbesondere in Form eines ein- oder zwei-dimensionalen Laserarrays, und/oder zwei oder mehr Leuchtdioden, insbesondere in Form eines ein-, zwei- oder mehrdimensionalen Diodenarrays, umfasst.

58) Vorrichtung nach Aspekt 47 oder einem der folgenden,
dadurch gekennzeichnet, dass
die Anregungssendeeinrichtung unmittelbar - oder mittelbar mittels einer Justiervorrichtung - mit einem optischen Medium/Messkörper, das/der mit dem Stoff, insbesondere dem ersten Bereich der Oberfläche des Stoffs,in dem die Messung zur Stoffanalyse vorgenommen wird, mechanisch fest verbunden ist.

59) Vorrichtung nach Aspekt 47 oder einem der folgenden,
dadurch gekennzeichnet, dass
die Einrichtung zur Intensitätsmodulierung eine elektrische Modulationseinrichtung, die mit der Anregungssendeeinrichtung/ Laserlichtquelle zur Erzeugung des Anregungsstrahls elektrisch in Verbindung steht und diese elektrisch ansteuert, umfasst oder durch eine solche gebildet ist.

60) Vorrichtung nach Aspekt 47 oder einem der folgenden,
dadurch gekennzeichnet, dass
die Einrichtung zur Intensitätsmodulierung wenigstens einen im Strahlengang angeordneten gesteuerten Spiegel umfasst.

61) Vorrichtung nach Aspekt 47 oder einem der folgenden,
dadurch gekennzeichnet, dass
die Einrichtung zur Intensitätsmodulierung wenigstens eine bezüglich ihrer Transparenz steuerbare, im Strahlengang angeordnete Schicht umfasst oder durch eine solche gebildet ist.

62) Vorrichtung nach Aspekt 47 oder einem der folgenden,
dadurch gekennzeichnet, dass
eine Einrichtung (105) zum Aussenden eines Messstrahls, insbesondere Mess-Lichtstrahls, in denjenigen Bereich eines optischen Mediums/Messkörpers vorgesehen ist, der mit dem ersten Bereich der Oberfläche des Stoffs, an dem die Stoffanalyse durchgeführt wird, in Kontakt steht Die entsprechende Oberfläche des Messkörpers wird auch Messfläche genannt.

63) Vorrichtung nach Aspekt 47 oder einem der folgenden,
dadurch gekennzeichnet, dass
die Einrichtung zum Aussenden eines Messstrahls und die Detektionseinrichtung derart zueinander ausgerichtet sind, dass die Detektionseinrichtung als das zeitabhängige Reaktionssignal den Messstrahl detektiert, nachdem dieser zumindest einmal an derjenigen Grenzfläche des optischen Mediums (=der Messfläche) reflektiert worden ist, die mit dem Stoff, insbesondere dem ersten Bereich der Oberfläche des Stoffs, in Kontakt steht.

Die Messfläche kann die Außenfläche einer Sensor- Schicht sein, die einen Teil des Messkörpers bildet und mit dem übrigen Teil des Messkörpers, insbesondere durch Kleben, verbunden ist.

64) Vorrichtung nach Aspekt 47 oder einem der folgenden,
dadurch gekennzeichnet, dass
die Einrichtung zum Aussenden eines Messstrahls und/oder die Detektionseinrichtung und/oder Anregungssendeeinrichtung unmittelbar mit dem optischen Medium/Messkörper mechanisch fest verbunden und/oder mittels eines Lichtwellenleiters an dieses/diesen angekoppelt ist.

65) Vorrichtung nach Aspekt 47 oder einem der folgenden,
dadurch gekennzeichnet, dass
das optische Medium/der Messkörper unmittelbar eine Abbildungsoptik trägt und/oder in das optische Medium eine Abbildungsoptik integriert ist. Die Abbildungsoptik kann beispielsweise eine oder mehrere in den Messkörper eingeformte Linsen oder Reflexionsflächen enthalten. Die Oberfläche des Messkörpers kann beispielsweise zu diesem Zweck in Form einer Linse geformt sein.

66) Vorrichtung nach Aspekt 47 oder einem der folgenden,
dadurch gekennzeichnet, dass
die Oberfläche des optischen Mediums mehrere gegeneinander geneigte Teilflächen aufweist, an denen ein Messstrahl, insbesondere der Mess-Lichtstrahl, mehrfach reflektiert wird.

67) Vorrichtung nach Aspekt 47 oder einem der folgenden,
dadurch gekennzeichnet, dass
in oder an dem optischen Medium/Messkörper ein oder mehrere Spiegelflächen zur Reflektion des Anregungsstrahls oder eines Messstrahls, insbesondere Mess-Lichtstrahls, vorgesehen sind.

68) Vorrichtung nach Aspekt 47 oder einem der folgenden,
dadurch gekennzeichnet, dass
die Anregungssendeeinrichtung (und/oder die Einrichtung zum Aussenden des Messstrahls und/oder die Detektionseinrichtung unmittelbar aneinander oder an einem gemeinsamen Träger befestigt sind. Dieser Träger kann als ganzer gegenüber dem Messkörper kontrolliert beweglich und diesem gegenüber mittels einer Justiervorrichtung justierbar sein.

69) Vorrichtung nach Aspekt 47 oder einem der folgenden,
dadurch gekennzeichnet, dass
der Träger durch eine Leiterplatte, eine Metallplatte oder Kunststoffplatte oder ein Gehäuse oder Gehäuseteil der Vorrichtung gebildet ist.

70) Vorrichtung nach Aspekt 47 oder einem der folgenden,
dadurch gekennzeichnet, dass
die Anregungssendeeinrichtung einen integrierten Halbleiterbaustein umfasst, der einen oder mehrere Laserelemente sowie mindestens ein mikrooptisches Bauelement und vorzugsweise zusätzlich ein Modulationselement aufweist.

71) Vorrichtung nach Aspekt 47 oder einem der folgenden,
dadurch gekennzeichnet, dass
das Modulationselement wenigstens ein gegenüber dem übrigen Halbleiterbaustein bewegliches und bezüglich einer Position steuerbares Element, insbesondere einen Spiegel aufweist.

72) Vorrichtung nach Aspekt 47 oder einem der folgenden,
dadurch gekennzeichnet, dass
das Modulationselement eine in ihrer Strahlungsdurchlässigkeit steuerbare Schicht aufweist.

73) Vorrichtung nach Aspekt 47 oder einem der folgenden,
dadurch gekennzeichnet, dass
das Modulationselement eine elektronische Ansteuerschaltung für die Modulation des einen oder mehrerer Laserelemente aufweist.

74) Vorrichtung nach einem der vorangehenden Aspekte, dadurch gekennzeichnet, dass der Messkörper oder das optische Medium als Flachkörper, insbesondere als planparalleler Körper in Form einer Platte ausgebildet ist, wobei insbesondere die Dicke senkrecht zur Messfläche (das heißt der Grenzfläche des Optischen Mediums, an die der zu analysierende Stoff angelegt wird) weniger als 50% der geringsten Ausdehnung des Messkörpers in einer parallel zur Messfläche verlaufenden Richtung beträgt, insbesondere weniger als 25%, weiter insbesondere weniger als 10% oder weniger als 5% oder weniger als 1 %.

Dabei kann an einer an die Messfläche angrenzenden oder dieser gegenüberliegenden Begrenzungsfläche oder an der Messfläche selbst eine Abbildungsoptik befestigt oder in diese Fläche eine Abbildungsoptik integriert sein. Die Abbildungsoptik kann dabei wenigstens eine Linse enthalten.

75) Vorrichtung nach einem der vorangehenden Aspekte dadurch gekennzeichnet, dass der Messkörper/ das optische Medium eine Spiegeleinrichtung zur Reflexion des von der Lasereinrichtung eingestrahlten Anregungsstrahls zur die Messfläche (oder zur Grenzfläche des Optischen Mediums, an die der zu analysierende Stoff angelegt wird,) aufweist oder trägt.

76) Einrichtung zur Analyse eines Stoffes nach Anspruch 1 oder einem der folgenden, dadurch gekennzeichnet, dass der Anregungsstrahl parallel zur Messfläche (oder der Grenzfläche des Optischen Mediums, an die der zu analysierende Stoff angelegt wird) oder in einem Winkel von weniger als 30 Grad, insbesondere weniger als 20 Grad, weiter insbesondere weniger als 10 Grad oder weniger als 5 Grad zur Messfläche (oder der Grenzfläche des Optischen Mediums, an die der zu analysierende Stoff angelegt wird) in den Messkörper eingestrahlt wird und dass der Anregungsstrahl in Richtung der Messfläche (oder der Grenzfläche des Optischen Mediums, an die der zu analysierende Stoff angelegt wird) umgelenkt oder abgelenkt wird und diese durchsetzt.

Der Messkörper kann eine kanalartige Ausnehmung für den Anregungsstrahl aufweisen, die mit ihrer Längsrichtung parallel zur Messfläche verläuft, so dass die Strecke, die der Anregungsstrahl bis zu seinem Austritt durch die Messfläche in dem Material des Messkörpers zurücklegt, reduziert, insbesondere auf Null reduziert wird. Wird eine Sensor- Schicht in den Messkörper integriert, so kann die Ausnehmung/Aussparung in dem Messkörper bis zu dieser reichen.

77) Verfahren zum Analysieren eines Stoffes, wobei bei dem Verfahren
mit einer Anregungssendeeinrichtung mindestens ein elektromagnetischer Anregungsstrahl mit einer oder mehreren Anregungswellenlängen durch den wenigstens teilweise gleichzeitigen oder aufeinander folgenden Betrieb mehrerer Laseremitter einer Laserlichtquelle erzeugt und in den Stoff hineingestrahlt wird,
mit einer Detektionseinrichtung ein Reaktionssignal detektiert wird und anhand des detektierten Reaktionssignals der Stoff analysiert wird.

78) Verfahren nach Aspekt 77, dadurch gekennzeichnet, dass nacheinander unter Verwendung verschiedener Modulationsfrequenzen der Anregungssendeeinrichtung Reaktionssignale, insbesondere zeitliche Reaktionssignalverläufe, ermittelt werden und dass mehrere Reaktionssignalverläufe zu verschiedenen Modulationsfrequenzen miteinander verknüpft werden und dass daraus insbesondere eine für einen Tiefenbereich unter der Stoffoberfläche spezifische Information gewonnen wird.

79) Verfahren nach Aspekt 78,
dadurch gekennzeichnet, dass
Reaktionssignalverläufe zu verschiedenen Modulationsfrequenzen jeweils für verschiedene Wellenlängen des Anregungsstrahls ermittelt werden und daraus insbesondere jeweils eine für einen Tiefenbereich unter der Stoffoberfläche spezifische Information gewonnen wird.

80) Verfahren nach Aspekt 79,
dadurch gekennzeichnet, dass
bei Verwendung von mehreren Modulationsfrequenzen des Anregungsstrahls zur gleichen Zeit das detektierte Reaktionssignal mittels eines Analyseverfahrens, vorzugsweise einer Fourier-Transformation, seinen Frequenzen entsprechend getrennt wird und
jeweils nur ein Teilsignal herausgefiltert, gemessen und analysiert wird, das einer zu verarbeitenden Frequenz entspricht.

Auf diese Weise kann eine Mehrzahl von Signalen zu unterschiedlichen Modulationsfrequenzen nacheinander analysiert werden und die Ergebnisse zu verschiedenen Modulationsfrequenzen können miteinander verknüpft werden, um eine Tiefeninformation über die Signale zu erhalten oder Signale, die von der Stoffoberfläche kommen, zu eliminieren.

81) Verfahren nach einem der voranstehenden Aspekte 77 bis 80,
dadurch gekennzeichnet, dass
ein optisches Medium/ein Messkörper mit dem Stoff, insbesondere einem ersten Bereich der Oberfläche des Stoffs, in direkten Kontakt gebracht wird,
mit der Anregungssendeeinrichtung der ausgesendete Anregungsstrahl erzeugt und insbesondere derart ausgestrahlt wird, dass dieser in das optische Medium eindringt und dieses an einem vorbestimmten Punkt an der Oberfläche des optischen Mediums, insbesondere an einer Messfläche, wieder verlässt,
mit einer Einrichtung zum Aussenden eines Messstrahls ein Messstrahl, insbesondere Mess-Lichtstrahl, derart erzeugt wird, dass dieser in das optische Medium /den Messkörper eindringt und dass insbesondere der Messstrahl und der Anregungsstrahl sich im Betrieb auf einer Grenzfläche des optischen Mediums und der Oberfläche des Stoffs, an der der Messstrahl reflektiert wird, insbesondere an der Messläche, überlappen, und
mit der Detektionseinrichtung ein das Reaktionssignal bildender reflektierter Messstrahl gemessen
und/oder direkt oder indirekt die Ablenkung des reflektierten Messstrahls detektiert wird.

Der reflektierte Messstrahl kann beispielsweise dadurch gemessen werden, dass seine Intensität mit einem ortsauflösenden lichtempfindlichen Halbleiterbauelement, insbesondere einer Quadrantendiode, erfasst wird.

82) Verfahren nach einem der voranstehenden Aspekte 77 bis 81,
dadurch gekennzeichnet, dass
in Abhängigkeit von einer in dem Stoff ermittelten Stoffkonzentration eine Dosiereinrichtung zur Abgabe einer Substanz in den Stoff, insbesondere in einen Patientenkörper angesteuert wird und/oder ein akustisches und/oder optisches Signal ausgegeben wird und/oder ein Signal mittels einer Funkverbindung an eine Verarbeitungseinrichtung abgegeben wird und/oder dass der gemessenen Stoffkonzentration ein oder mehrere Nahrungsmittel oder Nahrungsmittelkombinationen mittels einer Datenbank zugeordnet und als Ernährungsinformation, insbesondere Ernährungsempfehlung ausgegeben werden.

Zusätzlich oder kombiniert mit einer solchen Empfehlung kann auch eine Mengenangabe für die Nahrungsmittel oder Nahrungsmittelkombinationen ausgegeben werden. Unter Nahrungsmittelkombinationen sollen auch zubereitete Nahrungsportionen verstanden werden.

Alle Merkmale und Maßnahmen des Anregungsstrahls, seiner optischen Führung und Modulation, die in den Aspekten im Zusammenhang mit einer beliebigen Messmethode angeführt werden, insbesondere im Zusammenhang mit einem Mess- Lichtstrahl und der Detektion seiner Ablenkung, können, ebenso wie die Merkmale zum mechanischen Aufbau und zur Justierbarkeit, den Merkmalen zum Gehäuse und zur Kommunikation mit externen Einrichtungen, Datenbanken und angeschlossenen Geräten ebenfalls bei der Detektionsmethode eingesetzt werden, wie sie in den Patentansprüchen der vorliegenden Anmeldung beansprucht ist, also unter Verwendung eines Piezo- Effektes zur Detektion der aus dem Stoff als Reaktionssignal ausgesandten Wärmewelle in einen Messkörper.

Weitere Detektionsmethoden zum Nachweis eines Reaktionssignals nach Aussendung eines Anregungsstrahls können umfassen:
- Photoakustische Detektion - photoakustische Detektion mittels einer Stimmgabel oder eines anderen Schwingungselementes oder: eine leicht abgewandelte Form der Photoakustik mit offener QePAS-Zelle (Quartz enhanced PhotoAcustic Spectroskopy)). Durch diese Methoden können Druckschwankungen/schwingungen an der Stoffoberfläche nachgewiesen und ebenso ausgewertet werden, wie oben für die gemessene Strahlablenkung beschrieben.

Grundsätzlich können zur Tiefenprofilierung ermittelte Werte einer Phasenverschiebung des Reaktionssignals gegenüber einer periodischen Modulation des Anregungsstrahls genutzt werden. (Erwärmungs-/Abkühlphasen der Stoffoberfläche sollten dazu genauer bzgl. ihres Verlaufes ausgewertet werden).

Mit der beschriebenen Vorrichtung kann ein Vorrat von Klebestreifen zum Abtragen toter Hautschichten verbunden sein, um eine möglichst störungsfreie Messung an einem menschlichen Körper zu erlauben, sowie Pflaster mit Wärmeleitpaste, die auf das optische Medium regelmäßig aufgebracht werden können. Das optische Medium kann bei geeigneter Befestigung und Justierung der übrigen Teile austauschbar sein.

Die Vorrichtung kann zur Messung nicht nur an einem Finger einer Person, sondern auch an einer Lippe oder einem Ohrläppchen vorgesehen und eingerichtet sein.

Die Messung kann durch Kombination mehrerer der geschilderten und erläuterten Messsysteme mit ähnlicher Fehleranfälligkeit bezüglich der Genauigkeit und Zuverlässigkeit verbessert werden.

DAQ und LockIn-Verstärker in der Auswertung können in einem Gerät zusammengefasst werden und die Auswertung kann insgesamt digitalisiert werden.

Die Messung kann mit der Vorrichtung auch an einer relativ zu dieser bewegten Stoffoberfläche durchgeführt werden, so dass im Zuge einer Rastermessung: Anregungslichtquelle und- und/oder Messlichtquelle während der Messung in einem Raster über die Haut fahren, sich ggf. Hautunregelmäßigkeiten kompensieren oder wegmitteln lassen.

Die Sensitivität der Detektionseinrichtung/Deflektionseinheit kann durch Einstellung /Variation der Wellenlänge des Probe-Beams/der Messlichtquelle optimiert werden. Hierzu kann die Messlichtquelle bzgl. der Wellenlänge veränderbar sein oder mehrere Laserlichtquellen verschiedener Wellenlänge zur Auswahl oder Kombination enthalten.

Für die Ablenkung des Pump-/Probe-Lasers kann ein optimaler transversaler Mode (TEM) gewählt werden.

Anregungssendeeinrichtung, Messlichtquelle und Detektor können als ein gemeinsamer Array aufgebaut werden und die Strahlen können im optischen Medium geeignet umgelenkt werden, um Aussendung und Empfang aller Strahlen auf eine Stelle zu konzentrieren.

Eine Linse auf oder im Kristall des optischen Mediums kann dazu beitragen, den Messlichtstrahl Reaktionssignalabhängig stärker abzulenken.

Zudem ist die Verwendung einer gapfree Photodiode zur Detektion denkbar, dann könnte eine Linse den Messlichtstrahl nach Austritt bündeln und so eine genauere Messung ermöglichen.

Eine zusätzliche Ausgestaltung der Erfindung gemäß den Patentansprüchen wird in dem folgenden Konzept dargestellt. Dieses Konzept stellt darüber hinaus auch für sich genommen, kombiniert mit den obigen Aspekten oder mit Anspruchsgegenständen zumindest eine eigenständige Erfindung dar. Die Anmelderin behält sich vor, diese Erfindung oder Erfindungen zu einem späteren Zeitpunkt zum Gegenstand von Ansprüchen zu machen. Dies kann im Rahmen dieser Anmeldung oder auch im Rahmen von späteren Teilanmeldungen oder Nachanmeldungen unter Inanspruchnahme der Priorität dieser Anmeldung geschehen.

Auch das folgende Konzept für nichtinvasive Blutzuckermessung durch eine Bestimmung der Glucose in der Haut mittels Anregung durch Quantenkaskadenlaser und Messung der Wärmewelle durch Strahlungswärme soll von der Erfindung mit umfasst sein und kann mit den Gegenständen der Ansprüche kombiniert oder eigenständig in einer Teilanmeldung weiterverfolgt werden:
Es wird ein Verfahren beschrieben, mit dem die Konzentration der Glucose oder auch einem anderen Stoff in der interstitiellen Flüssigkeit (ISF) in der Haut bestimmt werden kann. Glucose in der ISF ist repräsentativ für Blutglucose und folgt ihr schnell bei Änderungen. Das Verfahren besteht aus zumindest einzelnen oder Gruppen der folgenden Schritte oder aus der Gesamtabfolge:
1. Die Hautstelle (in diesem Fall der erste Bereich der Stoffoberfläche) wird mit einem fokussierten und gegebenenfalls an einem Spiegel oder Hohlspiegel reflektierten Strahl eines Quantenkaskadenlasers, der stufenweise oder kontinuierlich über einen bestimmten Infrarotbereich in dem Glucose spezifisch Strahlung absorbiert durchgestimmt wird, bestrahlt. Anstelle des Quantenkaskadenlasers kann auch ein Laserarray mit mehreren mit einzelnen Wellenlängen strahlenden Lasern verwendet werden. Der Spektralbereich kann (oder die einzelnen Wellenlängen, typisch 5 oder mehr Wellenlängen können) insbesondere zwischen ca. 900 und ca. 1300 cm⁻¹ liegen, in dem Glucose einen Absorptionsfingerprint, das heißt typische und repräsentative Absorptionslinien aufweist.
2. Der Anregungsstrahl wird kontinuierlich (CW-Laser) oder gepulst mit hoher Pulswiederholrate oder moduliert verwendet. Zusätzlich wird der Anregungsstrahl niederfrequent moduliert, insbesondere im Frequenzbereich zwischen 10 und 1000 Hz. Die niederfrequente Modulation kann mit verschiedenen periodischen Funktionen, in verschiedenen Ausführungsformen Sinus, Rechteck oder Sägezahn oder ähnlich erfolgen.
3. Durch die Bestrahlung der Haut dringt die IR-Strahlung bis etwa 50-100 µm tief in die Haut ein und regt - je nach Wellenlänge - bestimmte Schwingungen im Molekül Glucose an. Diese Anregungen vom Schwingungsniveau v0 nach v1 gehen innerhalb von sehr kurzer Zeit in den Grundzustand zurück; bei diesem Schritt wird Wärme frei.
4. Als Folge der Wärmeentwicklung nach (3) bildet sich eine Wärmewelle aus, die isotrop vom Ort der Absorption ausgeht. Abhängig von der thermischen Diffusionslänge, die durch die unter (2) beschriebene niederfrequenten Modulation bestimmt wird, erreicht die Wärmewelle periodisch mit der Modulationsfrequenz die Oberfläche der Haut.
5. Das periodische Auftauchen der Wärmewelle an der Oberfläche entspricht einer periodischen Modulation der Wärmestrahlungseigenschaft der Haut (Stoffoberfläche der Probe). Die Haut kann hier näherungsweise als Schwarzer Strahler beschrieben werden, dessen Gesamtemission durch das Stefan-Boltzmann Gesetz proportional zur vierten Potenz der Oberflächentemperatur ist.
6. Mit einem Detektor für Wärmestrahlung, d.h. einem Infrarotdetektor, d.h. einem Thermoelement, Bolometer, Halbleiterdetektor, Piezodetektor oder ähnlichem, der auf die Hautstelle der Einstrahlung gerichtet ist, wird die unter (5) beschriebene periodische Temperaturerhöhung registriert. Sie ist abhängig von der unter (1) und (2) beschriebenen Einstrahlung von Infrarotlicht und von der unter (3) beschriebenen Absorption und somit abhängig von der Konzentration der Glucose.

Die Wärmestrahlung (in diesem Fall das Reaktionssignal) wird beispielsweise mittels eines optischen Elementes, in einer Ausführungsform einer Infrarotlinse oder eines Spiegels, insbesondere eines konkaven Parabolspiegels, gesammelt und, in einer Ausführungsform über einen konvexen Spiegel auf den Detektor geleitet. Hierzu kann ein verwendeter Sammelspiegel in einer Ausführungsform eine Öffnung aufweisen, durch die der gesammelte Strahl geleitet wird. Zudem kann ein Filter im Strahlengang vorgesehen sein, das nur Infrarotstrahlung eines bestimmten Wellenlängenbereichs durchlässt.

In einem anderen Ausführungsbeispiel wird die Wärmestrahlung mittels eines Messkörpers, wie er in den Patentansprüchen beansprucht ist, durch einen Piezo-Effekt nachgewiesen.

7. Es kann bei der Bearbeitung der Reaktionssignale speziell die Modulationsfrequenz berücksichtigt werden, wozu das Reaktionssignal in einem Lock-in- Verstärker verarbeitet werden kann. Durch Analyse der Phasenlage zwischen Anregungssignal und Wärmestrahlungssignal (Reaktionssignal) mittels einer Steuerungs- und Verarbeitungseinrichtung kann die Tiefeninformation über die Tiefe unter der Stoffoberfläche gewonnen werden, aus der die Reaktionssignale vorwiegend erhalten werden.

8. Durch die Auswahl und Analyse verschiedener niederfrequenter Modulationsfrequenzen wie unter (2) beschrieben für den Anregungsstrahl und die Verknüpfung der Ergebnisse für verschiedene Modulationsfrequenzen (wobei die Ergebnisse für verschiedene Modulationsfrequenzen auch unterschiedlich gewichtet werden können) kann ebenfalls die Tiefeninformation erhalten werden. Dabei können ggf. Differenzverfahren, eine Quotientenbildung von jeweils mindestens zwei Reaktionssignalen (zum Beispiel jeweils für eine einzelne Wellenlänge und dies wellenlängenweise durch das vermessene Spektrum hindurch) oder andere Bestimmungsverfahren verwendet werden, um die Absorption der obersten Hautschichten zu kompensieren.

9. Um die Detektion der Wärmestrahlung nach (6) möglichst empfindlich zu machen, wird sie spektral breitbandig für den gesamten in Frage kommenden Infrarotbereich genutzt.

Dabei sollen möglichst viele Bereiche der Planckschen Strahlungskurve genutzt werden. Um die Detektion für die intensive Anregungsstrahlung unempfindlich zu machen, wird die Detektion der Wärmestrahlung mit Sperrfilter (Notch-Filter) für diese Anregungswellenlängen versehen.

10. Aus dem nach (6-9) gemessenen, von der Anregungswellenlänge abhängigen Wärmesignal wird somit in einer Ausführungsform, wenn Glucose nachgewiesen werden soll, zunächst bei nicht (oder unter Ausnahme von) glucoserelevanten Wellenlängen des Anregungsstrahls der Hintergrund bestimmt, anschließend bei (oder unter Einschluss von) glucoserelevanten Wellenlängen die Differenz zum Hintergrundsignal. Daraus ergibt sich die Glucosekonzentration in der Hautschicht oder den Hautschichten, die durch die ausgewählte Phasenlage nach (7) oder die verschiedenen Modulationsfrequenzen nach (8) oder deren Verknüpfung festgelegt werden.

Obwohl die Erfindung im Detail durch bevorzugte Ausführungsbeispiele näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

### Bezugszeichenliste

- 1: Messkörper
- 1': Schicht
- 2: Messfläche
- 3: Lasereinrichtung
- 4: Detektionsbereich
- 4': Detektionsbereich
- 5: Bereich, Stoff
- 5': Position
- 6: Kontakteinrichtung
- 6e - 6y: Elektroden
- 7: Flächennormale
- 8: Anregungsstrahl
- 9: Modulationseinrichtung
- 10: Bereich
- 11: Flachkörper
- 12: Spiegeleinrichtung
- 13: Ausnehmung
- 14, 14a: Wärmesenke
- 15: Wärmebariere
- 16: Auswerteeinrichtung
- 17-21: Ausnehmungen
- 22 - 29: Elektroden
- 32: Leiterbahnen
- A: Richtung, Flächennormale
- B: Richtung

## Patentansprüche

1. Einrichtung zur Analyse eines Stoffes (5) mit:
- einem Messkörper (1, 1', 11), der eine Messfläche (2) aufweist, die zur Messung wenigstens teilweise mit dem Stoff (5) in Kontakt zu bringen ist,
- einer Anregungsstrahlquelle, insbesondere einer Lasereinrichtung (3),weiter insbesondere mit einem Quantenkaskadenlaser (QCL), einem durchstimmbaren QCL, und/oder mit einem Laserarray, vorzugsweise einem Array von QCLs, zur Erzeugung eines oder mehrerer Anregungsstrahlen (8) mit verschiedenen Wellenlängen, im infraroten Spektralbereich zwischen 3 µm und 20 µm, der die Messfläche durchsetzend auf den Stoff gerichtet ist, und
- einer Detektionsvorrichtung, (4,6), die Folgendes umfasst:
• einen Detektionsbereich(4, 4'), der Teil des Messkörpers (1, 1', 11) und insbesondere der Messfläche (2) benachbart oder unmittelbar benachbart ist, und der elektrische Eigenschaften hat, die sich in Abhängigkeit von einer Änderung in dem Druck oder der Temperatur ändern, und
• Elektroden (6a bis 6y, 22, 23, 24, 25, 26, 27, 28, 29, 123, 124, 130, 131), mit denen elektrische Signale erfasst werden können, die die genannten elektrischen Eigenschaften repräsentieren,
**dadurch gekennzeichnet, dass** wenigstens zwei Elektroden 6a, 6b in einer Richtung senkrecht zu einer Flächennormalen (7) der Messfläche (2) voneinander beabstandet auf verschiedenen Seiten des Detektionsbereiches (4) angeordnet sind.

2. Einrichtung zur Analyse eines Stoffes nach Anspruch 1, bei der die elektrische Eigenschaft, die sich in Abhängigkeit von dem Druck oder der Temperatur ändert,
• zu piezoelektrischen Signalen an den Elektroden (6a bis 6y, 22, 23, 24, 25, 26, 27, 28, 29, 123, 124, 130, 131)als Funktion der Druckänderung und/oder der Temperaturänderung führt, oder
• durch einen spezifischen elektrischen Widerstand gebildet wird, der sich in Abhängigkeit von der Temperatur ändert,
wobei die Einrichtung ferner eine elektrische Kontakteinrichtung (6) umfasst, die die genannten Elektroden (6a bis 6y, 22, 23, 24, 25, 26, 27, 28, 29, 123, 124, 130, 131) aufweist, welche mit dem Detektionsbereich des Messkörpers elektrisch leitend zur Erfassung des elektrischen Widerstandes und/oder der piezoelektrischen Signale verbunden sind.

3. Einrichtung zur Analyse eines Stoffes nach einem der vorhergehenden Ansprüche, **dadurch gekennzeichnet, dass** wenigstens zwei Elektroden (6c, 6d entlang der Flächennormalen (7) der Messfläche (2), hintereinander in verschiedenen Entfernungen von der Messfläche (2) angeordnet sind.

4. Einrichtung zur Analyse eines Stoffes nach einem der vorhergehenden Ansprüche, **dadurch gekennzeichnet, dass** der Anregungsstrahl (8) den Messkörper (1), insbesondere den Detektionsbereich (4, 4') des Messkörpers durchsetzt, wobei insbesondere zur Führung des Anregungsstrahls in oder an dem Messkörper (1) ein Lichtwellenleiter (126, 133, 134, 135, 136, 137) angeordnet und weiter insbesondere der Lichtwellenleiter in den Messkörper integriert ist.

5. Einrichtung zur Analyse eines Stoffes nach einem der vorhergehenden Ansprüche, die ferner ein optisches Element zur Fokussierung eines Anregungsstrahls (8) umfasst,
wobei das optische Element
- zwischen der Anregungsstrahlquelle und dem Messkörper (1), oder
- am Messkörper (1) dort, wo der Anregungsstrahl (8) in den Messkörper (1) eintritt, oder
- am Messkörper in dem Bereich vorgesehen ist, wo der Anregungsstrahl den Messkörper (1) verlässt.

6. Einrichtung zur Analyse eines Stoffes nach einem der Ansprüche 1 bis 5, **dadurch gekennzeichnet, dass** eine Modulationseinrichtung (9) zur Modulation der Intensität des Anregungsstrahls (8) vorgesehen ist, und/oder
dass wenigstens zwei, insbesondere wenigstens drei oder vier, weiter insbesondere wenigstens 6, weiter insbesondere wenigstens 8 Elektroden hintereinander in verschiedenen Abständen von der Messfläche (3) oder in einer Richtung senkrecht zu einer Flächennormalen (7) der Messfläche voneinander beabstandet angeordnet sind.

7. Einrichtung zur Analyse eines Stoffes nach einem der Ansprüche 1 bis 6, **dadurch gekennzeichnet, dass** wenigstens zwei, insbesondere wenigstens drei oder vier, weiter insbesondere wenigstens 6, weiter insbesondere wenigstens 8 Elektroden (6a bis 6y, 22, 23, 24, 25, 26, 27, 28, 29, 123, 124, 130, 131) in Richtung einer Flächennormalen (7) der Messfläche (2) oder senkrecht dazu oder in einer Richtung zwischen 0 und 90 Grad zur Flächennormalen (7) hintereinander in verschiedenen Abständen vom Detektionsbereich (4,4'), insbesondere in verschiedenen Abständen von der Mitte des Detektionsbereichs, angeordnet sind, und/oder
dass wenigstens zwei, insbesondere wenigstens drei oder vier, weiter insbesondere wenigstens 6, weiter insbesondere wenigstens 8 Elektroden (6a bis 6y, 22, 23, 24, 25, 26, 27, 28, 29, 123, 124, 130, 131) in einem kreisringförmigen Bereich(10) oder einem kugelschalenförmigen Bereich um den Detektionsbereich (4, 4') herum und wenigstens teilweise einander auf verschiedenen Seiten des Detektionsbereiches gegenüberliegend angeordnet sind, wobei verschiedene Elektroden jeweils im wesentlichen denselben Abstand von der Mitte des Detektionsbereichs oder unterschiedliche Abstände von der Mitte des Detektionsbereichs aufweisen.

8. Einrichtung zur Analyse eines Stoffes nach einem der vorhergehenden Ansprüche, **dadurch gekennzeichnet, dass** eine oder mehrere oder alle der Elektroden (6a bis 6y, 22, 23, 24, 25, 26, 27, 28, 29, 123, 124, 130, 131) der Kontakteinrichtung (6) scheiben- oder plattenförmig, ringförmig, ringscheibenförmig, in Form eines viereckigen oder polygonalen Rahmens mit einer Öffnung, kalottenförmig oder strangförmig ausgebildet sind, und/oder
dass eine oder mehrere oder alle der Elektroden (6a bis 6y, 22, 23, 24, 25, 26, 27, 28, 29, 123, 124, 130, 131) der Kontakteinrichtung (6) auf einer Oberfläche des Messkörpers (1, 1', 11) oder der Detektionsvorrichtung (4,4', 6) angeordnet und insbesondere mittels eines Fügeverfahrens, weiter insbesondere durch Kleben oder Schweißen aufgebracht sind.

9. Einrichtung zur Analyse eines Stoffes nach einem der vorhergehenden Ansprüche, **dadurch gekennzeichnet, dass** eine oder mehrere oder alle der Elektroden (6a bis 6y, 22, 23, 24, 25, 26, 27, 28, 29, 123, 124, 130, 131) der Kontakteinrichtung (6) im Inneren oder an der Außenseite des Messkörpers (1, 1', 11) in einer oder mehreren Ausnehmungen (17, 18, 19, 20, 21) des Messkörpers angeordnet sind, wobei sie insbesondere eingelegt, eingegossen, durch Spritzgießen oder durch ein additives Fertigungsvervahren (3D-Druck) eingebracht sind, und/oder
dass der Messkörper (1, 1' 11) als Flachkörper (11), insbesondere als planparalleler Körper in Form einer Platte ausgebildet ist, wobei insbesondere die Dicke des Messkörpers (1) in Richtung senkrecht zur Messfläche (2) weniger als 50% der geringsten Ausdehnung des Messkörpers in einer in der Messfläche verlaufenden Richtung beträgt, insbesondere weniger als 25%, weiter insbesondere weniger als 10%,
wobei der Messkörper (1, 1', 11) vorzugsweise eine Spiegeleinrichtung (12) zur Reflexion des von der Anregungsstrahlquelle, insbesondere Lasereinrichtung (3) eingestrahlten Anregungsstrahls (8) auf die Messfläche (2) aufweist oder trägt.

10. Einrichtung zur Analyse eines Stoffes nach einem der vorhergehenden Ansprüche, **dadurch gekennzeichnet, dass** der Anregungsstrahl (8) parallel zur Messfläche (2) oder in einem Winkel von weniger als 30 Grad, insbesondere weniger als 20 Grad, weiter insbesondere weniger als 10 Grad oder weniger als 5 Grad zur Messfläche in den Messkörper (1, 1', 11) eingestrahlt wird und dass der Anregungsstrahl in Richtung der Messfläche (2) umgelenkt oder abgelenkt wird und diese durchsetzt, und/oder
dass der Anregungsstrahl (8) das Material des Messkörpers (1) durchsetzt.

11. Einrichtung nach einem der vorhergehenden Ansprüche, **dadurch gekennzeichnet, dass** der Messkörper (1, 1', 11) wenigstens eine Ausnehmung oder Aussparung (13), insbesondere eine Bohrung aufweist, die durch den Anregungsstrahl (8) durchsetzt ist, wobei sich die Ausnehmung oder Aussparung und/oder Bohrung insbesondere von der Messfläche (2) oder von einer von der Messfläche begrenzten Sensor- Schicht des Messkörpers ausgehend in den Messkörper erstreckt, oder wobei die Ausnehmung oder Aussparung (13) und/oder Bohrung den gesamten Messkörper von einer der Messfläche (2) gegenüberliegenden Begrenzungsfläche des Messkörpers bis zu der Messfläche (2) durchsetzt, und/oder
dass in dem Messkörper (1, 1', 11) insbesondere in der Detektionsvorrichtung (4, 4', 6), oder unmittelbar an diesen/diese angrenzend und mit diesem im thermischen Kontakt stehend wenigstens eine Wärmesenke (14) in Form eines Körpers angeordnet ist, dessen spezifische Wärmekapazität und/oder spezifische Wärmeleitfähigkeit größer ist als die spezifische Wärmekapazität und/oder spezifische Wärmeleitfähigkeit des Materials oder der Materialien, aus dem der Messkörper besteht oder der als Peltier- Element ausgebildet ist.

12. Einrichtung nach einem der vorhergehenden Ansprüche, **dadurch gekennzeichnet, dass** in dem Messkörper (1, 1', 11) insbesondere in der Detektionsvorrichtung (4,4', 6), oder unmittelbar an diesen/diese angrenzend und mit diesem im thermischen Kontakt stehend wenigstens eine Wärmebarriere (15) in Form eines Körpers angeordnet ist, dessen spezifische Wärmekapazität und/oder spezifische Wärmeleitfähigkeit geringer ist als die spezifische Wärmekapazität und/oder spezifische Wärmeleitfähigkeit des Materials, aus dem der Messkörper (1) besteht, und/oder
dass die Detektionsvorrichtung(4,4', 6) und/oder der Messkörper (1, 1', 11) und/oder eine Sensor- Schicht (1') des Messkörpers wenigstens teilweise aus einem piezoelektrischen Material, insbesondere einer piezoelektrischen Keramik, insbesondere einer PZT- Keramik, weiter insbesondere einer gesinterten Keramik, oder einem einkristallinen piezoelektrischen Material, insbesondere Quarz, Turmalin, Lithiumniobat, Galliumorthophosphat, Berlinit, Seignettesalz, Ferroelektrika wie Bariumtitanat (BTO) oder Blei-Zirkonat-Titanat, Galliumphosphat oder einem Blei-Magnesium-Niobat, oder Zinkoxid (ZnO) oder Aluminiumnitrid als Dünnschichtabscheidung oder polarisiertem Polyvinylidenfluorid besteht, und/oder
dass ein piezoelektrisches Element oder ein piezoelektrischer Bereich des Messkörpers (1, 1', 11) als Aktor mit einer Spannungsquelle verbindbar ist und in Abhängigkeit von der Eingangsspannung für einen Anregungsstrahl eine Blockade darstellt.

13. Verfahren zum Betrieb einer Einrichtung nach einem der vorhergehenden Ansprüche, **dadurch gekennzeichnet, dass** ein modulierter Anregungsstrahl (8), insbesondere durch den Messkörper (1, 1', 11) hindurch, auf den zu analysierenden Stoff (5) gerichtet wird und dass gleichzeitig oder nacheinander Signale von verschiedenen Paaren von Elektroden der Kontakteinrichtung (6) erfasst und ausgewertet werden, dass zunächst anhand von Kriterien ermittelt wird, welche(s) der Elektrodenpaare zur Weiterverarbeitung geeignete Signale liefert/liefern und dass darauf die Signale von einem oder mehreren ausgewählten Elektrodenpaaren zur Messung verwendet und ausgewertet werden und dass insbesondere eine nachfolgende Messung durchgeführt wird, bei der die Signale des oder der ausgewählten Elektrodenpaare erfaßt und ausgewertet werden.

14. Verfahren nach Anspruch 13, **dadurch gekennzeichnet, dass** nach einem ersten Messversuch in Abhängigkeit von den erfassten Signalen eine Fehlausrichtung der Einrichtung relativ zu dem zu analysierenden Stoff (5) ermittelt und signalisiert wird und insbesondere der Nutzer zu einer Neuausrichtung aufgefordert wird.

15. Verfahren zum Analysieren eines Stoffes (5) unter Verwendung einer Einrichtung nach einem der Ansprüche 1 bis 12, wobei bei dem Verfahren
- mit einer Anregungssendeeinrichtung (3) mindestens ein intensitätsmodulierter elektromagnetischer Anregungsstrahl (8) mit zumindest einer Anregungswellenlänge erzeugt wird, die Anregungssendeeinrichtung (3) den mindestens einen elektromagnetischen Anregungsstrahl (8) in ein Stoffvolumen (5) einstrahlt, das unterhalb der Oberfläche des Stoffs (5) liegt,
- mit einer Detektionseinrichtung (16, 106, 107, 109) ein Reaktionssignal detektiert wird, und
- anhand des detektierten Reaktionssignals der Stoff analysiert wird, wobei insbesondere
- nacheinander unter Verwendung verschiedener Modulationsfrequenzen der Anregungssendeeinrichtung Reaktionssignale, insbesondere zeitliche Reaktionssignalverläufe für verschiedene Wellenlängen des Anregungsstrahls ermittelt und
- mehrere Reaktionssignalverläufe zu verschiedenen Modulationsfrequenzen miteinander verknüpft werden und wobei
- daraus insbesondere eine für einen Tiefenbereich unter der Stoffoberfläche spezifische Information gewonnen wird.

## Claims

1. A device for analysing a substance (5) having:
- a measuring body (1, 1', 11) which has a measuring surface (2) that is to be brought at least partially into contact with the substance (5) for the measurement,
- an excitation beam source, in particular a laser device (3), more particularly with a quantum cascade laser (QCL), a tuneable QCL, and/or with a laser array, preferably an array of QCLs, for generating one or more excitation beams (8) with different wavelengths in the infrared spectral range between 3 µm and 20 µm, which is directed at the substance while passing through the measuring surface, and
- a detection device (4, 6), which comprises the following:
• a detection region (4, 4') which is part of the measuring body (1, 1', 11) and arranged in particular adjacent or directly adjacent to the measuring surface (2), and has electrical properties that vary as a function of a change in pressure or temperature, and
• electrodes (6a to 6y, 22, 23, 24, 25, 26, 27, 28, 29, 123, 124, 130, 131) that can be used to detect electrical signals representing the above-mentioned electrical properties, **characterized in that** at least two electrodes (6a, 6b) are spaced apart from one another in a direction perpendicular to the surface normal (7) on different sides of the detection region (4).

2. The device for analysing a substance according to Claim 1, in which the electrical property which varies according to the pressure or temperature
• gives rise to piezoelectric signals on the electrodes (6a to 6y, 22, 23, 24, 25, 26, 27, 28, 29, 123, 124, 130, 13i) as a function of the pressure change and/or temperature change, or
• is formed by a specific electrical resistance, which varies according to the temperature,
wherein the device also comprises an electrical contact device (6) which comprises the said electrodes (6a to 6y, 22, 23, 24, 25, 26, 27, 28, 29, 123, 124, 130, 131), which are electrically conductively connected to the detection region of the measuring body for detecting the electrical resistance and/or piezoelectric signals.

3. The device for analysing a substance according to any one of the preceding claims, **characterized in that** at least two electrodes (6c, 6d) are arranged along the surface normal (7) of the measuring surface (2), one behind the other at different distances from the measuring surface (2) .

4. The device for analysing a substance according to any one of the preceding claims, **characterized in that** the excitation beam (8) passes through the measuring body (1), in particular the detection region (4, 4') of the measuring body, wherein an optical waveguide (126, 133, 134, 135, 136, 137) is arranged in or on the measuring body (1) in particular to guide the excitation beam and, more particularly, the optical waveguide is integrated into the measuring body.

5. The device for analysing a substance according to one of the preceding claims, further comprising an optical element for focusing and excitation beam (8), wherein the optical element is provided
• between the excitation radiation source and the measuring body (1),
• at the measuring body (1) at a position, where the excitation beam (8) enters the measuring body (1), or
• at the measuring body (1) is at a position, where the excitation beam leaves the measuring body (1).

6. The device for analysing a substance according to any one of Claims 1 to 5, **characterized in that** a modulation device (9) is provided for modulating the intensity of the excitation beam (8), and/or **in that** at least two, in particular at least three or four, more particularly at least 6, more particularly at least 8 electrodes are arranged one behind another at different distances from the measuring surface (3), or spaced apart from each other in a direction perpendicular to a surface normal (7) of the measuring surface.

7. The device for analysing a substance according to any one of Claims 1 to 6, **characterized in that** at least two, in particular at least three or four, more particularly at least 6, more particularly at least 8 electrodes (6a to 6y, 22, 23, 24, 25, 26, 27, 28, 29, 123, 124, 130, 131) are arranged one behind another in the direction of a surface normal (7) of the measuring surface (2) or perpendicular thereto or in a direction between 0 and 90 degrees to the surface normal (7) at different distances from the detection region (4, 4'), in particular at different distances from the centre of the detection region, and/orin that at least two, in particular at least three or four, more particularly at least 6, more particularly at least 8 electrodes (6a to 6y, 22, 23, 24, 25, 26, 27, 28, 29 123, 124, 130, 131) are arranged in an annular region (10) or a spherical shell-shaped region around the detection region (4, 4') and at least partially opposite one another on different sides of the detection region, different electrodes each being substantially the same distance from the centre of the detection region or different distances from the centre of the detection region.

8. The device for analysing a substance according to any one of the preceding claims, **characterized in that** one or more or all of the electrodes (6a to 6y, 22, 23, 24, 25, 26, 27, 28, 29, 123, 124, 130, 131) of the contact device (6) are disc- or plate-shaped, annular, annular disc-shaped, in the form of a rectangular or polygonal frame with an opening, cap-shaped or rod-shaped, and/orin that one or more or all of the electrodes (6a to 6y, 22, 23, 24, 25, 26, 27, 28, 29, 123, 124, 130, 131) of the contact device (6) are arranged on a surface of the measuring body (1, 1', 11) or the detection device (4, 4', 6) and in particular are attached by means of a joining method, more particularly by adhesive bonding or welding.

9. The device for analysing a substance according to any one of the preceding claims, **characterized in that** one or more or all of the electrodes (6a to 6y, 22, 23, 24, 25, 26, 27, 28, 29, 123, 124, 130, 131) of the contact device (6) are arranged on the inside of the measuring body (1, 1', 11) or on an outer side thereof in one or more recesses (17, 18, 19, 20, 21) of the measuring body, wherein they are, in particular, inserted, introduced by casting, by injection moulding or by an additive manufacturing method (3D printing), and/or **in that** the measuring body (1, 1', 11) is formed as a flat body (11), in particular as a plane-parallel body in the form of a plate, wherein in particular the thickness of the measuring body (1) in the direction perpendicular to the measuring surface (2) is less than 50 % of the smallest extension of the measuring body in a direction extending in the measuring surface, in particular, less than 25 %, more particularly less than 10 %, wherein the measuring body (1, 1', 11) preferably has or carries a mirror device (12) for reflecting the excitation beam (8) irradiated by the excitation beam source, in particular laser device (3), onto the measuring surface (2).

10. The device for analysing a substance according to any one of the preceding claims, **characterized in that** the excitation beam (8) is irradiated into the measuring body (1, 1', 11) parallel to the measuring surface (2) or at an angle of less than 30 degrees, in particular less than 20 degrees, more particularly less than 10 degrees or less than 5 degrees to the measuring surface, and that the excitation beam is diverted or deflected in the direction of the measuring surface (2) and passes through it, and/or the excitation beam (8) passes through the material of the measuring body (1).

11. The device according to any one of the preceding claims, **characterized in that** the measuring body (1, 1', 11) has at least one recess or slot (13), in particular a bored hole, through which the excitation beam (8) passes, wherein the recess or slot and/or bored hole extends into the measuring body, in particular from the measuring surface (2) or from a sensor layer of the measuring body bounded by the measuring surface, or wherein the recess or slot (13) and/or bored hole penetrates the entire measuring body from a boundary surface of the measuring body opposite the measuring surface (2) as far as the measuring surface (2), and/or
**in that** in the measuring body (1, 1', 11), in particular in the detection device (4, 4', 6) or directly adjacent thereto and in thermal contact therewith, at least one heat sink (14) is arranged in the form of a body, the specific thermal capacity and/or specific thermal conductivity of which is greater than the specific thermal capacity and/or specific thermal conductivity of the material or the materials from which the measuring body is made, or which is designed as a Peltier element.

12. The device according to any one of the preceding claims, **characterized in that** in the measuring body (1, 1', 11), in particular in the detection device (4, 4', 6) or directly adjacent thereto and in thermal contact therewith, at least one thermal barrier (15) is arranged in the form of a body, the specific thermal capacity and/or specific thermal conductivity of which is greater than the specific thermal capacity and/or specific thermal conductivity of the material from which the measuring body (1) is made, and/or in that the detection device (4, 4', 6) and/or the measuring body (1, 1', 11) and/or a sensor layer (1') of the measuring body, is at least partially made of a piezoelectric material, in particular a piezoelectric ceramic, in particular a PZT ceramic, more particularly a sintered ceramic, or a mono-crystalline piezoelectric material, in particular quartz, tourmaline, lithium niobate, gallium orthophosphate, berlinite, Seignette salt, ferroelectrics such as barium titanate (BTO) or lead zirconate-titanate, gallium phosphate or a lead-magnesium niobate, or zinc oxide (ZnO) or aluminium nitride as a thin-layer deposit or polarized polyvinyl fluoride, and/or **in that** a piezoelectric element or piezoelectric region of the measuring body (1, 1', 11) can be connected as an actuator to a voltage source and depending on the input voltage, represents a blockage for an excitation beam.

13. A method for operating a device according to any one of the preceding claims, **characterized in that** a modulated excitation beam (8) is directed, in particular through the measuring body (1, 1', 11), at the substance to be analysed (5) and that signals from different electrode pairs of the contact device (6) are acquired and evaluated simultaneously or sequentially, that it is firstly determined based on criteria which one or more of the pairs of electrodes delivers/deliver signals suitable for further processing, and that the signals from one or more selected electrode pairs are then used for measurement and evaluated, and that in particular a subsequent measurement is performed in which the signals of the selected electrode pair or pairs are acquired and evaluated.

14. The method according to Claim 13, **characterized in that** after an initial measurement test, depending on the signals detected a misalignment of the device relative to the substance to be analysed (5) is determined and indicated and, in particular, the user is prompted to perform a realignment.

15. A method for analysing a substance (5) using a device according to any one of Claims 1 to 12, wherein in the method
- with an excitation transmission device (3), at least one intensity-modulated electromagnetic excitation beam (8) with at least one excitation wavelength is generated, the excitation transmission device (3) irradiates the at least one electromagnetic excitation beam (8) into a volume of substance (5) which is located below the surface of the substance (5),
- a response signal is detected using a detection device (16, 106, 107, 109), and
- the substance is analysed on the basis of the detected response signal, wherein in particular
- using different modulation frequencies of the excitation transmission device, response signals, in particular temporal response signal waveforms for different wavelengths of the excitation beam, are successively determined and
- a plurality of response signal waveforms at different modulation frequencies are correlated with one another and wherein
- information specific to a depth range under the surface of the substance is obtained from these.

## Revendications

1. Dispositif d'analyse d'une substance (5) avec :
- un corps de mesure (1, 1', 11), qui présente une surface de mesure (2) à mettre en contact au moins partiellement avec la substance (5) pour la mesure ;
- une source de rayonnement d'excitation, plus particulièrement un dispositif laser (3), plus particulièrement un laser à cascade quantique (QCL), un QCL accordable, et/ou un réseau laser, de préférence un réseau de QCLs, pour générer un ou plusieurs faisceaux d'excitation (8) de différentes longueurs d'onde, dans le domaine spectral infrarouge entre 3 µm et 20 µm, qui est dirigé sur la substance en traversant la surface de mesure ; et
- un dispositif de détection (4, 6), qui comprend :
• une zone de détection (4, 4'), qui fait partie du corps de mesure (1, 1', 11) et est plus particulièrement adjacente ou immédiatement adjacente à la surface de mesure (2), et qui possède des propriétés électriques qui changent en fonction d'une modification de la pression ou de la température ; et
• des électrodes (6a à 6y, 22, 23, 24, 25, 26, 27, 28, 29, 123, 124, 130, 131), avec lesquelles des signaux électriques représentant lesdites propriétés électriques peuvent être détectés ;
**caractérisé en ce que** :
au moins deux électrodes (6a, 6b) sont disposées à distance l'une de l'autre, sur différentes côtés de la zone de détection (4), dans une direction perpendiculaire à une normale de surface (7) de la surface de mesure (2).

2. Dispositif d'analyse d'une substance selon la revendication 1, dans lequel la propriété électrique, qui varie en fonction de la pression ou de la température,
• conduit à des signaux piézoélectriques au niveau des électrodes (6a à 6y, 22, 23, 24, 25, 26, 27, 28, 29, 123, 124, 130, 131) en fonction du changement de pression et/ou du changement de température ; ou
• est constituée d'une résistance électrique spécifique qui change en fonction de la température ;
dans lequel le dispositif comprend en outre un dispositif de contact électrique (6), qui comporte lesdites électrodes (6a à 6y, 22, 23, 24, 25, 26, 27, 28, 29, 123, 124, 130, 131) qui sont connectées électriquement conductrices à la zone de détection du corps de mesure pour la détection de la résistance électrique et/ou des signaux piézoélectriques.

3. Dispositif d'analyse d'une substance selon l'une des revendications précédentes, **caractérisé en ce qu'**au moins deux électrodes (6c, 6d) sont disposées le long de la normale de surface (7) de la surface de mesure (2), l'une derrière l'autre à différentes distances de la surface de mesure (2).

4. Dispositif d'analyse d'une substance selon l'une des revendications précédentes, **caractérisé en ce que** le faisceau d'excitation (8) traverse le corps de mesure (1), plus particulièrement la zone de détection (4, 4') du corps de mesure, dans lequel, plus particulièrement, un guide d'ondes optique (126, 133, 134, 135, 136, 137) est disposé dans ou sur le corps de mesure (1) pour guider le faisceau d'excitation, et, encore plus particulièrement, le guide d'ondes optique est intégré dans le corps de mesure.

5. Dispositif d'analyse d'une substance selon l'une des revendications précédentes, qui comprend en outre un élément optique pour la focalisation d'un faisceau d'excitation (8), dans lequel l'élément optique est prévu :
- entre la source de rayonnement d'excitation et le corps de mesure (1), ou
- sur le corps de mesure (1) là où le faisceau d'excitation (8) entre dans le corps de mesure (1), ou
- sur le corps de mesure dans la zone où le faisceau d'excitation quitte le corps de mesure (1).

6. Dispositif d'analyse d'une substance selon l'une des revendications 1 à 5, **caractérisé en ce qu'**un dispositif de modulation (9) est prévu pour la modulation de l'intensité du faisceau d'excitation (8), et/ou
**en ce qu'**au moins deux, plus particulièrement au moins trois ou quatre, plus particulièrement au moins 6, plus particulièrement au moins 8 électrodes sont disposées l'une derrière l'autre à différentes distances de la surface de mesure (3) ou sont disposées à distance les unes des autres dans une direction perpendiculaire à une normale de surface (7) de la surface de mesure.

7. Dispositif d'analyse d'une substance selon l'une des revendications 1 à 6, **caractérisé en ce qu'**au moins deux, plus particulièrement au moins trois ou quatre, plus particulièrement au moins 6, plus particulièrement au moins 8 électrodes (6a à 6y, 22, 23, 24, 25, 26, 27, 28, 29, 123, 124, 130, 131) sont disposées dans la direction d'une normale de surface (7) de la surface de mesure (2) ou perpendiculairement à celle-ci ou dans une direction entre 0 et 90 degrés par rapport à la normale de surface (7), l'une derrière l'autre à différentes distances de la zone de détection (4, 4'), plus particulièrement à différentes distances du centre de la zone de détection, et/ou
**en ce qu'**au moins deux, plus particulièrement au moins trois ou quatre, plus particulièrement au moins 6, plus particulièrement au moins 8 électrodes (6a à 6y, 22, 23, 24, 25, 26, 27, 28, 29, 123, 124, 130, 131) sont disposées dans une zone annulaire (10) ou une zone en forme de calotte sphérique autour de la zone de détection (4, 4') et au moins partiellement opposées les unes aux autres sur différentes côtés de la zone de détection, dans lequel différentes électrodes présentent chacune sensiblement la même distance du centre de la zone de détection ou des distances différentes du centre de la zone de détection.

8. Dispositif d'analyse d'une substance selon l'une des revendications précédentes, **caractérisé en ce qu'**une ou plusieurs ou toutes les électrodes (6a à 6y, 22, 23, 24, 25, 26, 27, 28, 29, 123, 124, 130, 131) du dispositif de contact (6) sont formées en forme de disque ou de plaque, en forme d'anneau, en forme d'anneau-disque, en forme de cadre quadrangulaire ou polygonal avec une ouverture, en forme de calotte ou en forme de brin, et/ou
**en ce qu'**une ou plusieurs ou toutes les électrodes (6a à 6y, 22, 23, 24, 25, 26, 27, 28, 29, 123, 124, 130, 131) du dispositif de contact (6) sont disposées sur une surface du corps de mesure (1, 1', 11) ou du dispositif de détection (4, 4', 6) et sont plus particulièrement appliquées au moyen d'un procédé d'assemblage, plus particulièrement par collage ou soudage.

9. Dispositif d'analyse d'une substance selon l'une des revendications précédentes, **caractérisé en ce qu'**une ou plusieurs ou toutes les électrodes (6a à 6y, 22, 23, 24, 25, 26, 27, 28, 29, 123, 124, 130, 131) du dispositif de contact (6) sont disposées à l'intérieur ou à l'extérieur du corps de mesure (1, 1', 11) dans une ou plusieurs cavités (17, 18, 19, 20, 21) du corps de mesure, dans lequel elles sont plus particulièrement insérées, coulées, introduites par moulage par injection ou par un procédé de fabrication additive (impression 3D), et/ou
**en ce que** le corps de mesure (1, 1', 11) est formé comme un corps plat (11), plus particulièrement comme un corps plan-parallèle en forme de plaque, dans lequel, plus particulièrement, l'épaisseur du corps de mesure (1) dans la direction perpendiculaire à la surface de mesure (2) est inférieure à 50 % de la plus petite extension du corps de mesure dans une direction s'étendant dans la surface de mesure, plus particulièrement moins de 25 %, plus particulièrement moins de 10 %,
dans lequel le corps de mesure (1, 1', 11) présente ou supporte de préférence un dispositif miroir (12) pour la réflexion du faisceau d'excitation (8) émis par la source de rayonnement d'excitation, plus particulièrement le dispositif laser (3), sur la surface de mesure (2).

10. Dispositif d'analyse d'une substance selon l'une des revendications précédentes, **caractérisé en ce que** le faisceau d'excitation (8) est émis parallèlement à la surface de mesure (2) ou sous un angle inférieur à 30 degrés, plus particulièrement moins de 20 degrés, plus particulièrement moins de 10 degrés ou moins de 5 degrés par rapport à la surface de mesure, dans le corps de mesure (1, 1', 11) et **en ce que** le faisceau d'excitation est dévié ou réfléchi dans la direction de la surface de mesure (2) et la traverse, et/ou
**en ce que** le faisceau d'excitation (8) traverse le matériau du corps de mesure (1).

11. Dispositif selon l'une des revendications précédentes, **caractérisé en ce que** le corps de mesure (1, 1', 11) présente au moins un évidement ou une cavité (13), plus particulièrement un alésage, qui est traversé par le faisceau d'excitation (8), dans lequel l'évidement ou la cavité et/ou l'alésage s'étend plus particulièrement à partir de la surface de mesure (2) ou d'une couche de capteur du corps de mesure délimitée par la surface de mesure dans le corps de mesure, ou l'évidement ou la cavité (13) et/ou l'alésage traverse l'ensemble du corps de mesure d'une surface de délimitation du corps de mesure opposée à la surface de mesure (2) jusqu'à la surface de mesure (2), et/ou
**en ce qu'**au moins un puits de chaleur (14) sous la forme d'un corps est disposé dans le corps de mesure (1, 1', 11), plus particulièrement dans le dispositif de détection (4, 4', 6), ou immédiatement adjacent à celui-ci et en contact thermique avec celui-ci, dont la capacité thermique spécifique et/ou la conductivité thermique spécifique est supérieure à la capacité thermique spécifique et/ou à la conductivité thermique spécifique du matériau ou des matériaux dont est constitué le corps de mesure ou qui est formé comme un élément Peltier.

12. Dispositif selon l'une des revendications précédentes, **caractérisé en ce qu'**au moins une barrière thermique (15) sous la forme d'un corps est disposée dans le corps de mesure (1, 1', 11), plus particulièrement dans le dispositif de détection (4, 4', 6), ou immédiatement adjacente à celui-ci et en contact thermique avec celui-ci, dont la capacité thermique spécifique et/ou la conductivité thermique spécifique est inférieure à la capacité thermique spécifique et/ou à la conductivité thermique spécifique du matériau dont est constitué le corps de mesure (1), et/ou
**en ce que** le dispositif de détection (4, 4', 6) et/ou le corps de mesure (1, 1', 11) et/ou une couche de capteur (1') du corps de mesure est au moins partiellement constitué d'un matériau piézoélectrique, plus particulièrement une céramique piézoélectrique, plus particulièrement une céramique PZT, plus particulièrement une céramique frittée, ou un matériau piézoélectrique monocristallin, plus particulièrement le quartz, la tourmaline, le niobate de lithium, l'orthophosphate de gallium, la berlinit, le sel de Seignette, des ferroélectriques tels que le titanate de baryum (BTO) ou le titanate de zirconate de plomb, le phosphate de gallium ou un niobate de plomb-magnésium, ou l'oxyde de zinc (ZnO) ou le nitrure d'aluminium en tant que dépôt en couche mince ou le polyfluorure de vinylidène polarisé, et/ou
**en ce qu'**un élément piézoélectrique ou une zone piézoélectrique du corps de mesure (1, 1', 11) peut être connecté comme un actionneur à une source de tension et représente un blocage en fonction de la tension d'entrée pour un faisceau d'excitation.

13. Procédé de fonctionnement d'un dispositif selon l'une des revendications précédentes, **caractérisé en ce qu'**un faisceau d'excitation modulé (8) est dirigé, plus particulièrement à travers le corps de mesure (1, 1', 11), sur la substance (5) à analyser et qu'en même temps ou successivement des signaux de différentes paires d'électrodes du dispositif de contact (6) sont détectés et évalués, qu'il est d'abord déterminé sur la base de critères quelle(s) paire(s) d'électrodes fournit/fournissent des signaux appropriés pour un traitement ultérieur et qu'ensuite les signaux d'une ou plusieurs paires d'électrodes sélectionnées sont utilisés et évalués pour la mesure et qu'plus particulièrement une mesure ultérieure est effectuée, au cours de laquelle les signaux de la ou des paires d'électrodes sélectionnées sont détectés et évalués.

14. Procédé selon la revendication 13, **caractérisé en ce que**, après une première tentative de mesure, un défaut d'alignement du dispositif par rapport à la substance (5) à analyser est détecté et signalé en fonction des signaux détectés et plus particulièrement l'utilisateur est invité à un réalignement.

15. Procédé d'analyse d'une substance en utilisant un dispositif selon l'une des revendications 1 à 12, dans lequel, dans le procédé,
- au moyen d'un dispositif d'émission d'excitation (3), au moins un faisceau d'excitation électromagnétique (8) à intensité modulée avec au moins une longueur d'onde d'excitation est généré, le dispositif d'émission d'excitation (3) émettant le ou les faisceaux d'excitation électromagnétiques (8) dans un volume de substance (5) situé sous la surface de la substance (5) ;
- au moyen d'un dispositif de détection (16, 106, 107, 109), un signal de réaction est détecté ; et
- la substance est analysée sur la base du signal de réaction détecté, plus particulièrement :
- des signaux de réaction, plus particulièrement des profils temporels de signaux de réaction, sont déterminés successivement en utilisant différentes fréquences de modulation du dispositif d'émission d'excitation pour différentes longueurs d'onde du faisceau d'excitation ; et
- plusieurs profils de signaux de réaction pour différentes fréquences de modulation sont combinés les uns aux autres ; et
- plus particulièrement, une information spécifique à une zone de profondeur sous la surface de la substance est tirée de celle-ci.
